(19) Europäisches Patentamt / European Patent Office / Office européen des brevets

(11) **EP 4 092 068 B1**

(12) **EUROPEAN PATENT SPECIFICATION**


(45) Date of publication and mention of the grant of the patent: **22.10.2025 Bulletin 2025/43**

(21) Application number: **21741616.3**

(22) Date of filing: **15.01.2021**

(51) International Patent Classification (IPC):
***C08G 64/02*** *(2006.01)* ***C08G 64/20*** *(2006.01)*
***C08L 69/00*** *(2006.01)* ***C07D 493/04*** *(2006.01)*

(52) Cooperative Patent Classification (CPC):
(C-Sets available)
**C08G 64/30; C07D 493/04; C08G 64/0208; G02B 1/041** (Cont.)

(86) International application number:
**PCT/JP2021/001325**

(87) International publication number:
**WO 2021/145443 (22.07.2021 Gazette 2021/29)**


(54) **POLYCARBONATE RESIN, POLYCARBONATE RESIN COMPOSITION, OPTICAL MOLDED BODY CONTAINING THESE, AND CYCLIC CARBONATE**

POLYCARBONATHARZ, POLYCARBONATHARZZUSAMMENSETZUNG, DIESE ENTHALTENDER OPTISCHER FORMKÖRPER UND ZYKLISCHES CARBONAT

RÉSINE DE POLYCARBONATE, COMPOSITION DE RÉSINE DE POLYCARBONATE, CORPS MOULÉ OPTIQUE CONTENANT CELLES-CI ET CARBONATE CYCLIQUE


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.01.2020 JP 2020005443**
**31.01.2020 JP 2020015754**

(43) Date of publication of application:
**23.11.2022 Bulletin 2022/47**



(73) Proprietor: **Asahi Kasei Kabushiki Kaisha**
**Tokyo 1000006 (JP)**

(72) Inventors:
- **TODA, Tatsuro**
**Tokyo 100-0006 (JP)**
- **YONEDA, Hisanari**
**Tokyo 100-0006 (JP)**
- **NAKATA, Takuto**
**Tokyo 100-0006 (JP)**
- **IDE, Shota**
**Tokyo 100-0006 (JP)**
- **FUKUOKA, Hiroshi**
**Tokyo 100-0006 (JP)**
- **IWASE, Katsuhiro**
**Tokyo 100-0006 (JP)**
- **ARAMAKI, Masaaki**
**Tokyo 100-0006 (JP)**
- **HABA, Osamu**
**Yonezawa-shi, Yamagata 992-8510 (JP)**
- **TAZAWA, Yusuke**
**Yonezawa-shi, Yamagata 992-8510 (JP)**

(74) Representative: **D Young & Co LLP**
**3 Noble Street**
**London EC2V 7BQ (GB)**



(56) References cited:
**CN-A- 107 991 842** **CN-A- 107 991 842**
**US-A1- 2016 068 630**

- **LYUTIK ET AL: "Derivatives of asymmetrically substituted myoinositol. IX. Methods for synthesizing phophoinositides. Synthesis of 1-0-(1',2'-di-0- stearoylglycerylphosphoryl) myoinosito l", ZHURNAL OBSHCHEI KHIMII [RUSSIAN JOURNAL OF ORGANIC CHEMISTRY], NAUKA, RU, vol. 41, no. 12, 30 November 1970 (1970-11-30), pages 2747 - 2753, XP009537841, ISSN: 0044-460X**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WILLIAM GUERIN ET AL: "Enantiopure Isotactic PCHC Synthesized by Ring-Opening Polymerization of Cyclohexene Carbonate", MACROMOLECULES, 24 June 2014 (2014-06-24), XP055126854, ISSN: 0024-9297, DOI: 10.1021/ma5009397**
- **LYUTIK, A. I. ET AL.: "Derivatives of asymmetrically substituted myoinositol. IX. Methods for synthesizing phophoinositides. Synthesis of 1-0-(1',2'-di-0-stearoylglycerylphosphoryl)myoinosito l", ZHURNAL OBSHCHEI KHIMII, vol. 41, no. 12, 1971, RU , pages 2747 - 2753, XP009537841, ISSN: 0044-460X**


(52) Cooperative Patent Classification (CPC): (Cont.)

C-Sets
**G02B 1/041, C08L 69/00**

## Description

Technical Field

The present invention relates to a polycarbonate resin, a polycarbonate resin composition, and an optical molded article comprising the same.

Background Art

Optical or information communications equipment including smartphones has spread rapidly in recent years. For example, the camera functions of smartphones have marked technical advantages, and lenses for cameras are required to possess excellent heat resistance, and excellent optical characteristics such as low birefringence, in addition to their weight saving and lower costs. The development of specialty polycarbonate resins for optical lenses is actively underway for the purpose of satisfying such requirements.
For example, Patent Documents 1 to 5 each disclose a low birefringent specialty polycarbonate resin having a bulky aromatic ring structure such as a binaphthyl skeleton or a fluorene skeleton. In the literatures, low birefringence is achieved by controlling the content ratios of a bisphenol A skeleton having a positive double refraction and the aromatic ring structure having a negative double refraction, in order to reduce the high double refraction of polycarbonate formed from existing bisphenol A.
On the other hand, a polycarbonate resin having an aliphatic, particularly, alicyclic structure, is also under development as a resin that is an alternative to aromatic polycarbonate, albeit not for optical lens purposes. Alicyclic polycarbonate tends to be superior in light resistance to polycarbonate resins having an aromatic ring such as bisphenol A. For example, Patent Document 6 discloses a polycyclic alicyclic polycarbonate resin excellent in transparency, heat resistance, and color. Furthermore, polycarbonate is also under development using not only petroleum feedstocks but starting materials derived from biomasses such as plants. For example, Patent Document 7 discloses a polycarbonate resin obtained using isosorbide inducible from starch as a starting material.
Among such alicyclic polycarbonate resins, poly(cyclohexene carbonate) having a cyclohexene carbonate structure is the simplest polycarbonate having a saturated six-membered carbon ring corresponding to a benzene ring. It is widely known that, as shown in, for example, Patent Document 8, poly(cyclohexene carbonate) can be synthesized through the reaction of cyclohexene oxide with carbon dioxide. It is also known that, as described in Patent Document 9, Non Patent Document 1 and Non Patent Document 2, poly(cyclohexene carbonate) is obtained by the ring-opening polymerization of 1,2-cyclohexene carbonate.
Meanwhile, a cyclic carbonate (cyclic carbonic acid ester) is known to be used for various purposes such as aprotic solvents, electrolytes, additives for electrolytes, polymer additives, synthetic intermediates, and polymer precursors (monomers).
Typically, for example, a mixed solvent of a cyclic carbonate, such as ethylene carbonate (EC) or propylene carbonate (PC), having a favorable permittivity, and a chain carbonate such as dimethyl carbonate (DMC), ethyl methyl carbonate (EMC), or diethyl carbonate (DEC) is used as an electrolyte in a lithium ion battery.
A cyclic carbonate has also been found to be useful as an additive for epoxy resins. A cyclic carbonate is listed as, for example, an additive that reduces the viscosity of epoxy resins without deteriorating their mechanical characteristics or solvent resistance (see, for example, Patent Document 10). It has also been found that use of cyclic carbonate in combination with another alicyclic monomer or oxetane is effective as an additive that reduces the cure shrinkage of light-curable resins (see, for example, Patent Document 11).
A cyclic carbonate plays an important role as an intermediate for precise synthesis, for example, in order to obtain carbamate with controlled optical activity (see, for example, Non Patent Document 3), though used as a synthetic intermediate for various purposes.
As for monomeric starting materials, the exploitation of polycarbonate as a starting material monomer by ring-opening polymerization has been shown (see, for example, Patent Document 9). Also, cases have been reported in which a cyclic carbonate is exploited as a comonomer as a biocompatible (biodegradable, low toxic, etc.) material (see, for example, Non Patent Documents 4 and 5).
As described above, a cyclic carbonate is an industrially very useful material. Its feature is that a renewable starting material $CO_2$ can be used as a starting material source of carbonyl groups. For example, cases have been shown in which a cyclic carbonate can be obtained through the reaction of various compounds such as epoxide (oxiranes) or oxetane with $CO_2$ (see, for example, Non Patent Document 6). Synthesis methods using diphenyl carbonate indirectly inducible from $CO_2$ as a carbonyl source have also been proposed (see, for example, Patent Documents 12 and 13).
A feature of a material of a cyclic carbonate is the presence of a carbonyl group derived from $CO_2$, i.e., a renewable starting material, in its structure. In recent years, a case has been reported in which a material having a higher ratio of a starting material derived from a renewable resource is synthesized by introducing a renewable starting material to

a principal skeleton which accounts for the great majority of a molecular weight (see, for example, Non Patent Document 7).

Particularly, inositol having a structurally rigid alicyclic skeleton is expected to have favorable physical characteristics such as heat resistance. In actuality, a synthesis case of carbonate has also been reported (see, for example, Non Patent Document 8). Patent Document 14 relates to a polycarbonate as an electron beam photoresist material. Example 1 describes the preparation of poly(cyclohexene carbonate) with a molecular weight of 41,9 kDa and a molecular weight distribution of 1,12. Therefore, the weight-average molecular weight of the polymer disclosed in Example 1 can be calculated as 46,9 KDa (46 928 gmol-1).

List of Prior Art Documents

Patent Document

Patent Document 1: Japanese Patent No. 6327151
Patent Document 2: Japanese Patent No. 6512219
Patent Document 3: Japanese Patent Laid-Open No. 2019-143152
Patent Document 4: Japanese Patent Laid-Open No. 2019-131824
Patent Document 5: Japanese Patent Laid-Open No. 2018-059107
Patent Document 6: Japanese Patent No. 4774610
Patent Document 7: Japanese Patent No. 6507495
Patent Document 8: Japanese Patent No. 5403537
Patent Document 9: Japanese Patent Laid-Open No. 2019-108547
Patent Document 10: Japanese Translation of PCT International Application Publication No. 2013-528685
Patent Document 11: Japanese Patent Laid-Open No. 2008-238417
Patent Document 12: Japanese Patent Laid-Open No. 2019-127441
Patent Document 13: Japanese Patent Laid-Open No. 2019-127442 Patent Document 14: CN 107 991 842 A

Non Patent Document

Non Patent Document 1: Macromolecules 2014, 47, 4230-4235
Non Patent Document 2: Polymer Journal 2013, 45, 1183-1187
Non Patent Document 3: Chem. Eur. J. 2016, 22, 1722-1727
Non Patent Document 4: Biomacromolecules 2006, 7, 2269-2273
Non Patent Document 5: ACS Macro Lett. 2018, 7, 336-340
Non Patent Document 6: ACS Catal. 2015, 5, 1353-1370
Non Patent Document 7: Polymer Journal (2013) 45, 1183-1187
Non Patent Document 8: Bull. Korean Chem. Soc. (2006) 27, 3, 359-360

Summary of Invention

Problems to be Solved by Invention

The present invention relates to a novel polycarbonate resin obtainable using a renewable resource with low environmental load, a polycarbonate resin composition, and an optical molded article comprising the same.

Means for Solving Problems

Specifically, the present invention is as follows.

[1] A polycarbonate resin comprising a structural unit represented by the following formula (1):

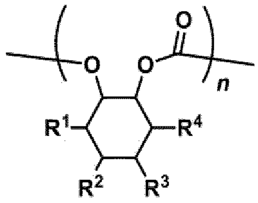

(1)

wherein $R^1$, $R^2$, $R^3$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain, wherein

weight-average molecular weight Mw measured by size-exclusion chromatography using polymethyl methacrylate as a standard sample is 50,000 or larger and 500,000 or smaller.

[2] The polycarbonate resin according to [1], wherein glass transition temperature Tg measured with a differential scanning calorimeter is 80°C or higher and 180°C or lower.

[3] The polycarbonate resin according to [1] or [2], wherein the polycarbonate resin comprises the structural unit represented by the formula (1) in the main chain.

[4] The polycarbonate resin according to any of [1] to [3], wherein a ratio of mr / (mm + mr + rr) measured by $^{13}$C-NMR is 40% or more, wherein mm represents an isotactic moiety in a periodic meso structure of the monomer, rr represents a syndiotactic moiety in a periodic structure based on a racemo bond, and mr represents a moiety in which the meso moiety and the racemo moiety are bonded.

[5] The polycarbonate resin according to any of [1] to [4], wherein the polycarbonate resin comprises a terminal alkoxy group.

[6] The polycarbonate resin according to [5], wherein the number of carbon atoms in the alkoxy group is 1 to 10.

[7] The polycarbonate resin according to any of [1] to [6], wherein a polymer component having a polystyrene-based molecular weight of 1,000 or larger measured by size-exclusion chromatography has a weight-average molecular weight (Mw) of 100,000 or larger and 500,000 or smaller and a ratio (Mw/Mn) of the weight-average molecular weight (Mw) to a number-average molecular weight (Mn) of 2.5 or less.

[8] The polycarbonate resin according to [7], wherein a rate of decrease in weight at 150 to 260°C is 30% by mass or less when alcohol-insoluble matter is heated in a nitrogen atmosphere by thermogravimetry.

[9] The polycarbonate resin according to any of [1] to [8], wherein an absolute value of a photoelastic coefficient is $10 \times 10^{-12}$ $Pa^{-1}$ or smaller.

[10] The polycarbonate resin according to any of [1] to [9], wherein an absolute value of an in-plane phase difference in a 100% uniaxially drawn film of the polycarbonate resin is 100 nm or smaller in terms of a thickness of 100 $\mu$m.

[11] The polycarbonate resin according to any of [1] to [10], wherein the polycarbonate resin comprises a structural unit represented by the formula (1) wherein each of $R^1$, $R^2$, $R^3$ and $R^4$ is a hydrogen atom.

[12] The polycarbonate resin according to any of [1] to [11], wherein the polycarbonate resin comprises a structural unit represented by the following formula (1') :

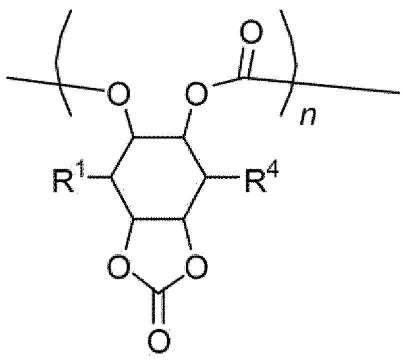

(1')

wherein $R^1$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon

atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain.

[13] A polycarbonate resin composition comprising
the polycarbonate resin according to any of [1] to [12] and an antioxidant.

[14] An optical component comprising the polycarbonate resin according to any of [1] to [12] or the polycarbonate resin composition according to [13].

[15] Use of the polycarbonate resin according to any of [1] to [12] or the polycarbonate resin composition according to [13] as a material for an optical component.

[16] A method for producing the polycarbonate resin according to any of [1] to [12], comprising
a polymerization step of ring-opening polymerizing a cyclic carbonate represented by the following formula (3) to obtain the polycarbonate resin:

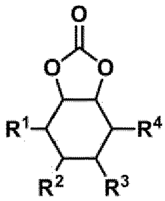
(3)

wherein $R^1$, $R^2$, $R^3$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain.

[17] The method for producing the polycarbonate resin according to [16], wherein in the polymerization step, organolithium, a metal alkoxide, or a combination of an organic compound comprising a HO group, a HS group or a HN group and a cyclic amine is used as a polymerization initiator.

[18] The method for producing the polycarbonate resin according to [16] or [17], wherein a reaction temperature (polymerization temperature) in the polymerization step is higher than -5°C and 40°C or lower.

[19] The method for producing the polycarbonate resin according to [18], wherein an initial concentration of the polymerizable monomer (initial monomer concentration) in the polymerization step is 10% by mass or more.

[20] The method for producing the polycarbonate resin according to [19], wherein a ratio of the polymerization initiator to be added to the monomer is 0.0001 mol% or more and 1 mol% or less.

Advantages of Invention

The present invention can provide a novel polycarbonate resin obtainable using a renewable resource with low environmental load, a polycarbonate resin composition, and an optical molded article comprising the same.

Brief Description of Drawings

[Figure 1] Figure 1 shows results of measuring a polycarbonate resin with a differential scanning calorimetric apparatus in Example A1.

[Figure 2] Figure 2 shows a $^1$H-NMR spectrum of a polycarbonate resin in Example A3.

[Figure 3] Figure 3 shows a $^1$H-NMR spectrum of a polycarbonate resin in Example A4.

[Figure 4] Figure 4 shows a MALDI-TOF-MS spectrum of a polycarbonate resin in Comparative Example A3.

[Figure 5] Figure 5 shows a MALDI-TOF-MS spectrum of a polycarbonate resin in Example A2.

[Figure 6] The upper part of Figure 6 is a $^1$H-NMR chart of 2,3:5,6-di-O-cyclohexylidene-myo-inositol synthesized in step 1-1 of Example B1 of the present application. The lower part of Figure 6 is a $^1$H-NMR chart of a 1,4-di-O-benzyl-2,3:5,6-di-O-cyclohexylidene-myo-inositol derivative (BCyI) synthesized in step 1-2 of Example B1 of the present application.

[Figure 7] Figure 7 is a $^{1}$H-NMR chart of 1,4-di-O-benzyl-myo-inositol (BI) synthesized in step 1-3 of Example B1 of the present application.

[Figure 8] Figure 8 is a $^{1}$H-NMR chart of 1,4-di-O-benzyl-2,3:5,6-dicarbonate-myo-inositol (BCI) synthesized in step 1-4 of Example B1 of the present application.

[Figure 9] Figure 9 is a $^{1}$H-NMR chart of 1,4-di-O-octyl-2,3:5,6-dicarbonate-myo-inositol (OCI) synthesized in step 2-4 of Example B2 of the present application.

[Figure 10] The upper part of Figure 10 is a GC/MS chromatograph of a carbonate-myo-inositol composition obtained in Example B2 of the present application. The lower part of Figure 10 is a MS spectrum of each compound in the carbonate-myo-inositol composition obtained in Example B2 of the present application.

[Figure 11] Figure 11 is a $^{1}$H-NMR chart of trans-1,2-cyclohexene carbonate (T6C) synthesized in Synthesis Example 1 of the present application.

[Figure 12] Figure 12 is a $^{1}$H-NMR chart of a myo-inositol-containing copolymer (BCI/T6C copolymer) synthesized in Synthesis Example 2 of the present application.

[Figure 13] Figure 13 is a $^{1}$H-NMR chart of a myo-inositol-containing copolymer (OCI/T6C copolymer) synthesized in Synthesis Example 3 of the present application.

[Figure 14] Figure 14 is a $^{1}$H-NMR chart of a myo-inositol-containing copolymer (BCI/T6C copolymer) synthesized in Synthesis Example 4 of the present application.

[Figure 15] Figure 15 is a $^{1}$H-NMR chart of a myo-inositol-containing copolymer (BCI/T6C copolymer) synthesized in Synthesis Example 5 of the present application.

[Figure 16] Figure 16 is a $^{1}$H-NMR chart of a myo-inositol-containing copolymer (OCI/T6C copolymer) synthesized in Synthesis Example 6 of the present application.

Mode for Carrying Out Invention

Hereinafter, the mode for carrying out the present invention (hereinafter, also referred to as the "present embodiment") will be described in detail.

[Polycarbonate resin and method for producing same]

As described in Background Art mentioned above, the previous literatures that disclose poly(cyclohexene carbonate) merely evaluated the correlation between polymerization conditions and molecular weights and rarely mentioned the characteristics of poly(cyclohexene carbonate). The literatures made no mention about, particularly, its optical characteristics.

The present inventors have conducted detailed studies on conventional polycarbonate resins including those described in the literatures, and consequently found that at least any of heat resistance, and optical characteristics such as low birefringence are insufficient.

For example, a polycarbonate resin having an aromatic ring structure as described in Patent Documents 1 to 5 does not have sufficient optical characteristics such as low birefringence. Also, a polycarbonate resin having a cyclohexene carbonate structure as described in

Patent Document 9 and Non Patent Document 1 is difficult to mold into a film sample for optical measurement due to its low molecular weight and thus have unknown optical characteristics.

The present embodiment has been made in light of the problems described above. An object of the present embodiment is to provide a polycarbonate resin moldable into a film, having excellent heat resistance, and excellent optical characteristics such as low birefringence, a polycarbonate resin composition, and an optical molded article comprising the same.

The present inventors have pursued diligent studies to attain the object and consequently found that a polycarbonate resin comprising a specific cyclic structure in the main chain and having a specific weight-average molecular weight (Mw) has excellent heat resistance, and excellent optical characteristics such as low birefringence and is moldable into a film.

(Polycarbonate resin)

The polycarbonate resin of the present embodiment comprises a structural unit represented by the following formula (1), wherein weight-average molecular weight Mw measured by size-exclusion chromatography (SEC) using polymethyl methacrylate as a standard sample is 50,000 or larger and 500,000 or smaller:

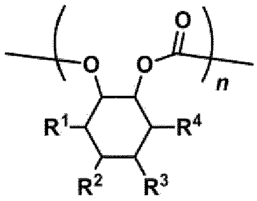

(1)

In the formula (1), $R^1$, $R^2$, $R^3$ and $R^4$ (hereinafter, also referred to as "$R^1$ to $R^4$") are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain. In the present specification, the "carbonate group" means a divalent substituent represented by -OC(=O)O-.

It is preferred to have the structural unit represented by the formula (1) in the main chain.

The polycarbonate resin composition of the present embodiment having the configuration described above has excellent heat resistance, and excellent optical characteristics such as low birefringence. The reason for this is probably, but is not limited to, as follows.

The double refraction of a conventional polycarbonate resin comprising an aromatic ring structure is difficult to reduce due to its structure. By contrast, the polycarbonate resin of the present embodiment has no aromatic ring structure and is therefore presumed to have excellent optical characteristics such as low birefringence. The presence of a specific alicyclic structure reduces a photoelastic coefficient and prevents the occurrence of a double refraction, presumably exerting excellent optical characteristics. Furthermore, the presence of the specific alicyclic structure in the main chain offers excellent heat resistance. Moreover, the polycarbonate resin of the present embodiment has a weight-average molecular weight (Mw) that falls within the range of 50,000 or larger and 500,000 or smaller, and is therefore excellent in heat resistance and optical characteristics. Particularly, Mw of 50,000 or larger enables the polycarbonate resin to be molded into a film.

In the present embodiment, in the formula (1), $R^1$ to $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms. In the formula (1), $R^1$ to $R^4$ are preferably each independently one or more substituents selected from the group consisting of a hydrogen atom, a hydroxy group, a vinyl group, an allyl group, an alkoxy group having 1 to 10 carbon atoms, an ester group having 1 to 11 carbon atoms, an acyl group having 1 to 11 carbon atoms, and an unsubstituted linear, branched, or cyclic alkyl group having 1 to 10 carbon atoms from the viewpoint of more effectively and reliably exerting the advantageous effects of the present invention. From a similar viewpoint, in the formula (1), $R^1$ to $R^4$ are more preferably each independently one or more substituents selected from the group consisting of a hydrogen atom, a hydroxy group, a vinyl group, an allyl group, an alkoxy group having 1 to 10 carbon atoms, and an unsubstituted linear, branched, or cyclic alkyl group having 1 to 10 carbon atoms. From a similar viewpoint, in the formula (1), $R^1$ to $R^4$ are further preferably each independently one or more substituents selected from the group consisting of a hydrogen atom, a hydroxy group, an alkoxy group having 1 to 10 carbon atoms, and an unsubstituted linear, branched, or cyclic alkyl group having 1 to 10 carbon atoms. From a similar viewpoint, in the formula (1), $R^1$ to $R^4$ are particularly preferably each independently one or more substituents selected from the group consisting of a hydrogen atom, a hydroxy group, and an alkoxy group having 1 to 10 carbon atoms.

In the present embodiment, in the formula (1), $R^1$ to $R^4$ are optionally bonded to each other via an alkylene group or a carbonate group (-OC(=O)O- group) to form a cyclic structure. The alkylene group is optionally substituted by a hydroxy group, a phosphoric acid group, an amino group, an alkoxy group, or an ester group, and optionally has a carbonyl group inserted in the main chain. When $R^1$ to $R^4$ are bonded to each other via an alkylene group to form a cyclic structure, the number of carbon atoms in the alkylene group is preferably 1 to 10, more preferably 1 to 8, further preferably 1 to 6, from the viewpoint of more reliably and effectively exerting the advantageous effects of the present invention. From a similar viewpoint, the substituent of the alkylene group is preferably a hydroxy group, an alkoxy group, or an ester group, more preferably a hydroxy group or an alkoxy group. From a similar viewpoint, when $R^1$ to $R^4$ form a cyclic structure, preferably, the cyclic structure is formed via an unsubstituted alkylene group or a carbonate group (-OC(=O)O- group). Examples of the unsubstituted alkylene group include a methylene group, an ethylene group, a n-propylene group, a n-butylene group, n-pentylene group, and a n-hexylene group.

In the formula (1) of the present embodiment, the phosphoric acid group and the amino group may be unsubstituted or substituted. Specifically, these groups may be a monosubstituted phosphoric acid group and amino

group or may be a disubstituted phosphoric acid group and amino group. When the phosphoric acid group and the amino group are substituted, each substituent is preferably an unsubstituted linear, branched, or cyclic alkyl group having 1 to 10 carbon atoms from the viewpoint of more effectively and reliably exerting the advantageous effects of the present invention. From a similar viewpoint, the phosphoric acid group and the amino group according to the present embodiment are preferably unsubstituted.

In the formula (1) of the present embodiment, examples of the alkoxy group having 1 to 10 carbon atoms include, but are not particularly limited to, a methoxy group, an ethoxy group, a propoxy group, a butoxy group, a pentyloxy group, a cyclopentyloxy group, a hexyloxy group, a cyclohexyloxy group, a heptyloxy group, an octyloxy group, a nonyloxy group, a decyloxy group, a phenoxy group, a benzyloxy group, a vinyloxy group, and an allyloxy group.

In the formula (1) of the present embodiment, examples of the ester group having 1 to 11 carbon atoms include, but are not particularly limited to, a methyl ester group, an ethyl ester group, a propyl ester group, a butyl ester group, a pentyl ester group, a cyclopentyl ester group, a hexyl ester group, a cyclohexyl ester group, a heptyl ester group, an octyl ester group, a nonyl ester group, a decyl ester group, a phenyl ester group, a benzyl ester group, a vinyl ester group, and an allyl ester group.

In the formula (1) of the present embodiment, examples of the acyl group having 1 to 11 carbon atoms include, but are not particularly limited to, a formyl group, an acetyl group, a propionyl group, a butyryl group, a valeryl group, and a benzoyl group.

In the formula (1) of the present embodiment, examples of the unsubstituted linear, branched, or cyclic alkyl group having 1 to 10 carbon atoms include, but are not particularly limited to, a methyl group, an ethyl group, a n-propyl group, an isopropyl group, a n-butyl group, an isobutyl group, a tert-butyl group, a n-pentyl group, a cyclopentyl group, a n-hexyl group, a cyclohexyl group, a n-heptyl group, a n-octyl group, a n-nonyl group, and a n-decyl group.

The amount of the structural unit represented by the formula (1) in the polycarbonate resin of the present embodiment is preferably 50 mol% or more, more preferably 70 mol% or more, further preferably 90 mol% or more, further preferably 99 mol% or more, per 100 mol% of monomer units constituting the polycarbonate resin. The upper limit of the amount is not particularly limited and is, for example, 100 mol%.

The polycarbonate resin of the present embodiment preferably comprises a terminal alkoxy group. The number of carbon atoms in the alkoxy group is preferably 1 to 10, more preferably 3 to 8. Examples of the alkoxy group include, but are not particularly limited to, a methoxy group, an ethoxy group, a n-propoxy group, an isopropoxy group, a n-butoxy group, an isobutoxy group, a tert-butoxy group, a n-pentoxy group, a cyclopentoxy group, a n-hexyloxy group, a cyclohexyloxy group, a hydroxycyclohexyloxy group, a n-heptyloxy group, a n-octyloxy group, a n-nonyloxy group, a n-decyloxy group, a phenoxy group, and a benzyloxy group. Among them, a methoxy group, a tert-butoxy group, a hydroxycyclohexyloxy group, a phenoxy group, or a benzyloxy group is preferred.

The content of the terminal alkoxy group is preferably 0.01 mol% or more and 1 mol% or less per 100 mol% of monomer units constituting the polycarbonate resin.

The polycarbonate resin of the present embodiment preferably comprises a structural unit represented by the formula (1) wherein each of $R^1$, $R^2$, $R^3$ and $R^4$ is a hydrogen atom. The polycarbonate resin of the present embodiment having such a structural unit tends to have much better heat resistance, and much better optical characteristics such as low birefringence.

The amount of the structural unit represented by the formula (1) wherein each of $R^1$, $R^2$, $R^3$ and $R^4$ is a hydrogen atom in the polycarbonate resin of the present embodiment is preferably 50 mol% or more, more preferably 70 mol% or more, further preferably 90 mol% or more, further preferably 95 mol% or more, further preferably 99 mol% or more, per 100 mol% of monomer units constituting the polycarbonate resin. The upper limit of the amount is not particularly limited and is, for example, 100 mol%.

The polycarbonate resin of the present embodiment preferably comprises a structural unit represented by the following formula (1'):

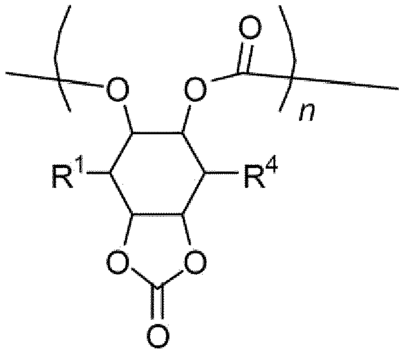

(1')

wherein $R^1$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain.

The polycarbonate resin of the present embodiment having such a structural unit tends to have much better heat resistance, and much better optical characteristics such as low birefringence.

The amount of the structural unit represented by the formula (1') in the polycarbonate resin of the present embodiment is preferably 0.1 mol% or more and 50 mol% or less, more preferably 1 mol% or more and 30 mol% or less, further preferably 2 mol% or more and 10 mol% or less, further preferably 2 mol% or more and 5 mol% or less, per 100 mol% of monomer units constituting the polycarbonate resin.

The polycarbonate resin of the present embodiment preferably comprises terminal structures represented by $R^5$ and $R^6$ in the following formula (2):

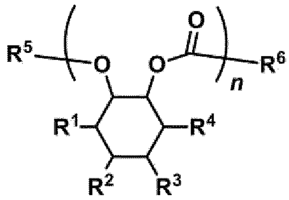

(2)

In the formula (2), $R^5$ and $R^6$ (hereinafter, also referred to as "$R^5$ to $R^6$") are each independently a hydrogen atom, a hydroxy group, a phosphoric acid group, an amino group, a vinyl group, an allyl group, an alkoxy group having 1 to 10 carbon atoms, a silyl group having 1 to 30 carbon atoms, a silylalkoxy group having 1 to 30 carbon atoms, an ester group having 1 to 11 carbon atoms, an acyl group having 1 to 11 carbon atoms, or an unsubstituted linear, branched, or cyclic alkyl group having 1 to 10 carbon atoms. In the formula (2), $R^1$ to $R^4$ are as defined in the formula (1).

In the polycarbonate resin of the present embodiment, in the formula (2), $R^5$ and $R^6$ are optionally bonded to each other to form a cyclic structure. Specifically, the terminal structures represented by $R^5$ and $R^6$ may be absent.

In the present embodiment, in the formula (2), preferred $R^1$ to $R^4$ are as defined in the formula (1). In the formula (2), examples of the phosphoric acid group, the amino group, the alkoxy group having 1 to 10 carbon atoms, the ester group having 1 to 11 carbon atoms, the acyl group having 1 to 11 carbon atoms, and the unsubstituted linear, branched, or cyclic alkyl group having 1 to 10 carbon atoms are also as defined in the formula (1). In the formula (2), $R^5$ and $R^6$ are preferably each independently one or more substituents selected from the group consisting of a hydrogen atom, a hydroxy group, a vinyl group, an allyl group, an alkoxy group having 1 to 10 carbon atoms, a silyl group having 1 to 30 carbon atoms, a silylalkoxy group having 1 to 30 carbon atoms, an ester group having 1 to 11 carbon atoms, an acyl group having 1 to 11 carbon atoms, and an unsubstituted linear, branched, or cyclic alkyl group having 1 to 10 carbon atoms from the viewpoint of more effectively and reliably exerting the advantageous effects of the present invention. From a similar viewpoint, in the formula (2), $R^5$ and $R^6$ are more preferably each independently one or more substituents selected from the group consisting of a hydrogen atom, a hydroxy group, a vinyl group, an allyl group, an alkoxy group having 1 to 10 carbon atoms, a silyl group having 1 to 30 carbon atoms, a silylalkoxy group having 1 to 30 carbon atoms, and an unsubstituted linear, branched, or cyclic alkyl group having 1 to 10 carbon atoms. From a similar viewpoint, in the formula (2), $R^5$ and $R^6$ are further preferably each independently one or more substituents selected from the group consisting of a hydrogen atom, a hydroxy group, an alkoxy group having 1 to 10 carbon atoms, a silyl group having 1 to 30 carbon atoms, a silylalkoxy group

having 1 to 30 carbon atoms, and an unsubstituted linear, branched, or cyclic alkyl group having 1 to 10 carbon atoms.

In the formula (2), examples of the silyl group having 1 to 30 carbon atoms include, but are not particularly limited to, a trimethylsilyl group, a triethylsilyl group, a triisopropylsilyl group, a triphenylsilyl group, a tert-butyldimethylsilyl group, a di-tert-butylisobutylsilyl group, and a tertbutyldiphenylsilyl group.

In the formula (2), examples of the silylalkoxy group having 1 to 30 carbon atoms include, but are not particularly limited to, a trimethylsilylmethoxy group, a trimethylsilylethoxy group, a trimethylsilylphenoxy group, a trimethylsilylbenzyloxy group, a triethylsilylmethoxy group, a triethylsilylethoxy group, a triethylsilylphenoxy group, a triethylsilylbenzyloxy group, a triisopropylsilylmethoxy group, a triisopropylsilylethoxy group, a triisopropylsilylphenoxy group, a triisopropylsilylbenzyloxy group, a triphenylsilylmethoxy group, a triphenylsilylethoxy group, a triphenylsilylphenoxy group, a triphenylsilylbenzyloxy group, a tertbutyldimethylsilylmethoxy group, a tertbutyldimethylsilylethoxy group, a tertbutyldimethylsilylphenoxy group, a tertbutyldimethylsilylbenzyloxy group, a di-tert-butylisobutylsilylmethoxy group, a di-tert-butylisobutylsilylethoxy group, a di-tert-butylisobutylsilylphenoxy group, a di-tert-butylisobutylsilylbenzyloxy group, a tertbutyldiphenylsilylmethoxy group, a tertbutyldiphenylsilylethoxy group, a tertbutyldiphenylsilylphenoxy group, and a tertbutyldiphenylsilylbenzyloxy group.

In the polycarbonate resin of the present embodiment, the weight-average molecular weight (Mw) measured by size-exclusion chromatography (SEC) using polymethyl methacrylate as a standard sample is 50,000 or larger and 500,000 or smaller. The polycarbonate resin of the present embodiment having the Mw that falls within the range described above has improved toughness because of a further entangled molecular chain of the polymer and becomes moldable into a film. Such a polycarbonate resin is excellent in heat resistance and optical characteristics. From a similar viewpoint, the Mw is preferably 80,000 or larger and 500,000 or smaller, more preferably 100,000 or larger and 500,000 or smaller. The upper limit of the Mw may be 300,000. The weight-average molecular weight of the polycarbonate resin by size-exclusion chromatography can be measured, specifically, by a method described in Examples.

In order to control the weight-average molecular weight (Mw) of the polycarbonate resin of the present embodiment within the range described above, the ratio between a polymerizable monomer and a polymerization initiator can be appropriately adjusted, and the polycarbonate resin can be produced by a production method mentioned later. The Mw tends to be able to be increased by decreasing the ratio of the polymerization initiator to the polymerizable monomer. The Mw tends to be able to be increased by lengthening a polymerization time. The Mw tends to be able to be increased by performing stirring using a stirring blade.

In the polycarbonate resin of the present embodiment, a polymer component having a polystyrene-based molecular weight of 1,000 or larger measured by size-exclusion chromatography preferably has a weight-average molecular weight (Mw) of 100,000 or larger and 500,000 or smaller and a ratio (Mw/Mn) of the weight-average molecular weight (Mw) to a number-average molecular weight (Mn) of 2.5 or less.

The polycarbonate resin composition of the present embodiment having the configuration described above can more markedly suppress thermal weight loss. The reason for this is probably, but is not limited to, as follows.

The thermal weight loss is probably suppressed more markedly for reasons such as an elevated molecular weight, a sufficient degree of polymerization obtained, and a decreased content of low-molecular-weight components (mainly, mono-, di- and trimer) having a low thermal decomposition temperature, which can prevent the decomposition of the polymer chain from being promoted by chemical species resulting from the decomposition of the low-molecular-weight components.

In the polycarbonate resin of the present embodiment, the polymer component having a molecular weight (polystyrene-based molecular weight) of 1,000 or larger measured by size-exclusion chromatography (SEC) using polystyrene as a standard sample preferably has a weight-average molecular weight (Mw) of 100,000 or larger and 500,000 or smaller. The polycarbonate resin of the present embodiment having the Mw that falls within the range described above has improved toughness because of a further entangled molecular chain of the polymer and becomes moldable into a film. Such a polycarbonate resin is excellent in heat resistance and optical characteristics. From a similar viewpoint, the Mw is more preferably 110,000 or larger and 500,000 or smaller, further preferably 120,000 or larger and 500,000 or smaller. The upper limit of the Mw may be 350,000. In the polycarbonate resin of the present embodiment, the weight-average molecular weight of the polymer component having a polystyrene-based molecular weight of 1,000 or larger measured by size-exclusion chromatography can be measured, specifically, by a method described in Examples.

In order to control the weight-average molecular weight (Mw) in the polycarbonate resin of the present embodiment within the range described above, the ratio between a polymerizable monomer and a polymerization initiator and an additive can be appropriately adjusted, and the polycarbonate resin can be produced by a production method mentioned later. The Mw tends to be able to be increased by decreasing the ratio of the polymerization initiator and the additive to the polymerizable monomer. The Mw tends to be able to be increased by lengthening a polymerization time. The Mw tends to be able to be increased by performing stirring using a stirring blade.

In the polycarbonate resin of the present embodiment, the ratio (Mw/Mn) of the weight-average molecular weight (Mw) to a number-average molecular weight (Mn) measured by size-exclusion chromatography is 2.5 or less. When the Mw/Mn is 2.5 or less, heat resistance is excellent. From a similar viewpoint, the Mw/Mn is more preferably 2.5 or less,

further preferably 2.4 or less, particularly preferably 2.3 or less. The lower limit of the Mw/Mn is not particularly limited and is, for example, 1.0.

In the polycarbonate resin of the present embodiment, the rate of decrease in weight at 150 to 260°C is preferably 30% by mass or less when alcohol-insoluble matter is heated in a nitrogen atmosphere by thermogravimetry. It is preferred to reduce the rate of decrease in weight from 150°C which is regarded as being higher than the glass transition temperature (Tg) of a general alicyclic polycarbonate resin to approximately 270°C which is considered as the upper limit of a cylinder temperature at the time of a molding process. The polycarbonate resin of the present embodiment having the rate of decrease in weight that falls within the range described above can suppress coloring or poor molding ascribable to main chain cleavage. From a similar viewpoint, the rate of decrease in weight is preferably 30% by mass or less, more preferably 25% by mass or less, further preferably 20% by mass or less, still further preferably 10% by mass or less, even further preferably 5% by mass or less. The lower limit of the rate of decrease in weight is not particularly limited and is, for example, 0.5% by mass.

In the polycarbonate resin of the present embodiment, the rate of decrease in weight can be measured, specifically, by a method described in Examples.

In order to control the rate of decrease in weight of the polycarbonate resin of the present embodiment within the preferred range described above, for example, a polymerization temperature for the polycarbonate resin, the addition of an additive that regulates a terminal structure, purification by a reprecipitation operation, or the molecular weight of the polycarbonate resin can be controlled. The rate of decrease in weight tends to be reduced, particularly, by performing polymerization for the polycarbonate resin at higher than -5°C and 40°C or lower, elevating the weight-average molecular weight Mw of the polycarbonate resin to 50,000 or larger based on polymethyl methacrylate, setting Mw/Mn to 2.5 or less, or performing purification by a reprecipitation operation.

In the polycarbonate resin of the present embodiment, the glass transition temperature Tg measured with a differential scanning calorimeter (DSC) is preferably 80°C or higher and 180°C or lower. When the glass transition temperature Tg is 80°C or higher, morphology tends to be able to be further maintained in a general environment of usage. When Tg is 180°C or lower, molding processability tends to be much better. From a similar viewpoint, the glass transition temperature Tg of the polycarbonate resin of the present embodiment is more preferably 90°C or higher and 180°C or lower, further preferably 100°C or higher and 180°C or lower. The glass transition temperature Tg of the polycarbonate resin can be measured, specifically, by a method described in Examples.

In order to control the glass transition temperature Tg of the polycarbonate resin of the present embodiment within the preferred range described above, the weight-average molecular weight and the number-average molecular weight of the polycarbonate resin can be controlled, and the polycarbonate resin can be produced by a production method mentioned later. The glass transition temperature Tg tends to be elevated, particularly, by increasing the weight-average molecular weight and the number-average molecular weight.

In the polycarbonate resin of the present embodiment, the ratio of mr / (mm + mr + rr) measured by $^{13}$C-NMR is preferably 40% or more, wherein mm represents an isotactic moiety in a periodic meso structure of the monomer, rr represents a syndiotactic moiety in a periodic structure based on a racemo bond, and mr represents a moiety in which the meso moiety and the racemo moiety are bonded. When the ratio of mr / (mm + mr + rr) falls within the range described above, the blockiness of the sequences of the isotactic moiety and the syndiotactic moiety in the polymer chain is decreased with randomness improved. This can reduce the crystallinity of the polycarbonate resin, which therefore tends to exhibit high transparency when molded into a film. From a similar viewpoint, the ratio of mr / (mm + mr + rr) is more preferably 42% or more, further preferably 44% or more, still further preferably 45% or more, even further preferably 46% or more. The upper limit of the ratio of mr / (mm + mr + rr) is not particularly limited and is, for example, 100%. The ratio of mr / (mm + mr + rr) of the polycarbonate resin can be measured, specifically, by a method described in Examples.

In order to control the ratio of mr / (mm + mr + rr) of the polycarbonate resin of the present embodiment within the preferred range described above, the steric structure of an initiator can be adjusted, the optical purity of a monomer can be adjusted, or a less polar solvent system can be used. The ratio of mr / (mm + mr + rr) tends to be decreased, particularly, by using an achiral initiator that induces neither simple alkoxide nor asymmetric reaction sites in order to enhance the spatial degree of freedom around a growing end, or using a low polar solvent system.

In the polycarbonate resin of the present embodiment, the absolute value of a photoelastic coefficient is preferably $10 \times 10^{-12}$ $Pa^{-1}$ or smaller. When the absolute value of the photoelastic coefficient falls within the range described above, change in phase difference can be suppressed when strain is applied to the polycarbonate resin. The polycarbonate resin therefore tends to exhibit lower birefringence. From a similar viewpoint, the absolute value of the photoelastic coefficient of the polycarbonate resin of the present embodiment is more preferably $8 \times 10^{-12}$ $Pa^{-1}$ or smaller, further preferably $5 \times 10^{-12}$ $Pa^{-1}$ or smaller, still further preferably $3 \times 10^{-12}$ $Pa^{-1}$ or smaller, even further preferably $2 \times 10^{-12}$ $Pa^{-1}$ or smaller. In the polycarbonate resin of the present embodiment, the lower limit of the absolute value of the photoelastic coefficient is not particularly limited and is, for example, $0.01 \times 10^{-12}$ $Pa^{-1}$. The absolute value of the photoelastic coefficient of $3 \times 10^{-12}$ $Pa^{-1}$ or smaller is lower than the value of general-purpose borosilicate glass as optical glass and is preferred because a double refraction is less likely to occur by the application of stress. The photoelastic

coefficient of the polycarbonate resin can be measured, specifically, by a method described in Examples. A test piece for use in measuring the photoelastic coefficient of the polycarbonate resin of the present embodiment is molded into a film at 150°C or higher and 300°C or lower. The film thickness of the prepared film is preferably 10 μm or larger and 1000 μm or smaller.

In order to control the absolute value of the photoelastic coefficient of the polycarbonate resin of the present embodiment within the preferred range described above, $R^1$ to $R^4$ in the formula (1) can be appropriately selected, and the polycarbonate resin can be produced by a production method mentioned later. The absolute value of the photoelastic coefficient tends to be decreased, particularly, by selecting the preferred $R^1$ to $R^4$ described above.

In the polycarbonate resin of the present embodiment, the absolute value of an in-plane phase difference in a 100% uniaxially drawn film is preferably 100 nm or smaller in terms of a thickness of 100 μm. When the absolute value of the in-plane phase difference falls within the range described above, the double refraction of the polycarbonate resin ascribable to the orientation of molecules contained in the polymer chain tends to be able to be further prevented. From a similar viewpoint, the absolute value of the in-plane phase difference in the 100% uniaxially drawn film of the polycarbonate resin of the present embodiment is more preferably 80 nm or smaller in terms of a thickness of 100 μm, further preferably 60 nm or smaller in terms of a thickness of 100 μm. The lower limit of the absolute value of the in-plane phase difference is not particularly limited and may be 0 nm in terms of a thickness of 100 μm. The in-plane phase difference of the polycarbonate resin can be measured, specifically, by a method described in Examples. The 100% uniaxially drawn film of the polycarbonate resin means a film obtained by molding the polycarbonate resin into a film by a method such as a casting method or vacuum heat press, and then drawing the film by 100% in the uniaxial direction under a temperature condition equal to or higher than the glass transition temperature. The film before drawing is formed at 150°C or higher and 300°C or lower. The film thickness of the prepared film is preferably 10 μm or larger and 1000 μm or smaller.

In order to control the absolute value of the in-plane phase difference in the polycarbonate resin of the present embodiment within the preferred range described above, $R^1$ to $R^4$ in the formula (1) can be appropriately selected, and the polycarbonate resin can be produced by a production method mentioned later. The absolute value of the in-plane phase difference tends to be decreased, particularly, by selecting the preferred $R^1$ to $R^4$ described above.

Examples of the method for synthesizing the polycarbonate resin having the characteristics described above include a method of ring-opening polymerizing a cyclic carbonate. A method for producing a polycarbonate resin mentioned later is preferably used as the method for synthesizing the polycarbonate resin of the present embodiment from the viewpoint of more effectively and reliably exerting the advantageous effects of the present invention.

(Polycarbonate resin composition)

The polycarbonate resin composition of the present embodiment contains the polycarbonate resin described above and an antioxidant.

The polycarbonate resin composition of the present embodiment containing the polycarbonate resin described above and an antioxidant can be further prevented from being degraded by heat or shear at the time of a molding process, and can therefore have further improved heat resistance. Furthermore, the polycarbonate resin composition of the present embodiment containing an antioxidant can prevent the polycarbonate resin from being oxidized in use, and can therefore have further improved light resistance.

Examples of the antioxidant for use in the polycarbonate resin composition of the present embodiment include, but are not particularly limited to, hindered phenol antioxidants and phosphorus antioxidants.

Examples of the hindered phenol antioxidant include, but are not particularly limited to, Irganox 1010 (pentaerythritol tetrakis[3-(3,5-di-t-butyl-4-hydroxyphenyl)propionate]), Irganox 1076 (octadecyl-3-(3,5-di-t-butyl-4-hydroxyphenyl)propionate), Irganox 1330 (3,3',3",5,5',5"-hexa-t-butyl-a,a',a"-(mesitylene-2,4,6-triyl)tri-p-cresol), Irganox 3114 (1,3,5-tris(3,5-di-t-butyl-4-hydroxybenzyl)-1,3,5-triazine-2,4,6(1H,3H,5H)-trione), Irganox 3125, ADKSTAB AO-60 (pentaerythritoltetrakis[3-(3,5-di-t-butyl-4-hydroxyphenyl)propionate]), ADKSTAB AO-80 (3,9-bis{2-[3-(3-t-butyl-4-hydroxy-5-methylphenyl)propionyloxy]-1,1-dimethylethyl}-2,4,8,10-tetraoxaspiro[5.5]undecane), Cyanox 1790, Sumilizer GA-80, Sumilizer GS (2-[1-(2-hydroxy-3,5-di-tert-pentylphenyl)ethyl]-4,6-di-tert-pentylphenyl acrylate), and Sumilizer GM (2-tert-butyl-4-methyl-6-(2-hydroxy-3-tert-butyl-5-methylbenzyl)phenyl acrylate). These may be used singly, or in combinations of two or more thereof.

Examples of the phosphorus antioxidant include, but are not particularly limited to, Irgafos 168 (tris(2,4-di-t-butylphenyl)phosphite), Irgafos 12 (tris[2-[[2,4,8,10-tetra-t-butyldibenzo[d,f][1,3,2]dioxaphosphepin-6-yl]oxy]ethyl] amine), ADKSTAB HP-10 (2,2'-methylenebis(4,6-di-tert-butylphenyl)octyl phosphite), ADKSTAB PEP36 (bis(2,6-di-t-butyl-4-methylphenyl)pentaerythritol diphosphite), ADKSTAB PEP36A (bis(2,6-di-t-butyl-4-methylphenyl)pentaerythritol diphosphite), Sumilizer GP ((6-[3-(3-t-butyl-4-hydroxy-5-methylphenyl)propoxy]-2,4,8,10-tetra-t-butyldibenzo[d,f][1,3,2] dioxaphosphepine), and GSY P101 (tetrakis(2,4-di-t-butyl-5-methylphenyl)4,4'-biphenylene diphosphonite). These may be used singly, or in combinations of two or more thereof.

The polycarbonate resin for use in the polycarbonate resin composition of the present embodiment is the same as

the polycarbonate resin described above, and its preferred form is also the same thereas.

The content of the polycarbonate resin in the polycarbonate resin composition of the present embodiment is preferably 50% by mass or more and 100% by mass or less, more preferably 60% by mass or more and less than 100% by mass, further preferably 65% by mass or more and less than 100% by mass, based on the whole resin composition from the viewpoint of more effectively and reliably exerting the advantageous effects of the present invention.

The content of the antioxidant in the polycarbonate resin composition of the present embodiment is preferably 0.001% by mass or more and 1% by mass or less, more preferably 0.003% by mass or more and 1% by mass or less, further preferably 0.005% by mass or more and 1% by mass or less, based on the whole resin composition from the viewpoint of further preventing degradation caused by heat or shear at the time of a molding process.

The polycarbonate resin composition of the present embodiment may contain an additional additive without inhibiting the attainment of the object of the present invention. Examples of the additive include, but are not particularly limited to, 1,2-cyclohexene carbonate, 1,2-cyclohexanediol, toluene, o-xylene, m-xylene, p-xylene, methanol, ethanol, acetone, methyl ethyl ketone, methyl propyl ketone, methyl isopropyl ketone, methyl isobutyl ketone, and acetic acid.

(Optical component)

The optical component of the present embodiment contains the polycarbonate resin described above or the polycarbonate resin composition described above.

The polycarbonate resin described above or the polycarbonate resin composition described above, contained in the optical component of the present embodiment has a small photoelastic coefficient, and a small in-plane phase difference associated with molecular orientation, as mentioned above. The optical component of the present embodiment is therefore less likely to cause a stress double refraction and an orientation double refraction. Thus, the optical component of the present embodiment tends to be able to suppress a double refraction in use. Furthermore, the polycarbonate resin described above or the polycarbonate resin composition described above, contained in the optical component of the present embodiment has excellent heat resistance, as mentioned above. The optical component of the present embodiment is therefore less likely to be degraded over time and can be used for a long period as compared with conventional optical components.

Examples of the optical component according to the present embodiment include, but are not particularly limited to, optical lenses such as camera, telescope, microscope, projector, and car-mounted lenses, and optical films such as diffuser panels, light guide plates, polarizers, and phase difference films. The optical component of the present embodiment is obtained by appropriately subjecting the polycarbonate resin described above or the polycarbonate resin composition described above to processing such as molding so as to have a shape suitable for the purpose thereof.

(Method for producing polycarbonate resin)

The method for producing the polycarbonate resin of the present embodiment comprises a polymerization step of ring-opening polymerizing a cyclic carbonate (A1) represented by the following formula (3) to obtain the polycarbonate resin:

(A1)

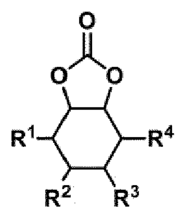

(3)

In the formula (3), $R^1$ to $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group (-OC(=O)O- group) to form a cyclic structure, wherein the alkylene group is optionally substituted by a hydroxy group, a phosphoric acid group, an amino group, an alkoxy group, or an ester group, and optionally has a carbonyl group inserted in the main chain.

In the present embodiment, preferred $R^1$ to $R^4$ in the formula (3) are as defined in the formula (1). In the formula (3), examples of the phosphoric acid group, the amino group, the alkoxy group having 1 to 10 carbon atoms, the ester group having 1 to 11 carbon atoms, the acyl group having 1 to 11 carbon atoms, and the unsubstituted linear, branched, or cyclic

alkyl group having 1 to 10 carbon atoms are also as defined in the formula (1).

In the method for producing the polycarbonate resin of the present embodiment, the cyclic carbonate (A1) for use in ring-opening polymerization may be one cyclic carbonate used alone, or any two or more cyclic carbonates differing in $R^1$ to $R^4$ may be used in combination.

In the method for producing the polycarbonate resin of the present embodiment, the cyclic carbonate (A1) encompasses cyclic carbonates (B1) to (B4) represented by the following formulas (4) to (7):

(B 1)

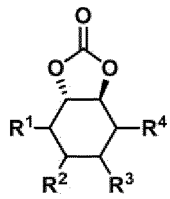

(4)

(B 2)

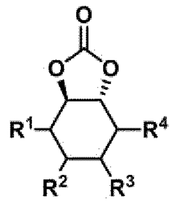

(5)

(B 3)

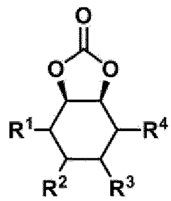

(6)

(B 4)

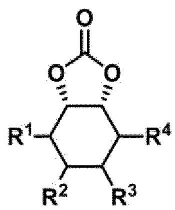

(7)

In the formulas (4) to (7), $R^1$ to $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group (-OC(=O)O- group) to form a cyclic structure, wherein the alkylene group is optionally substituted by a hydroxy group, a phosphoric acid group, an amino group, an alkoxy group, or an ester group, and optionally has a carbonyl group inserted in the main chain.

In the present embodiment, preferred $R^1$ to $R^4$ in the formulas (4) to (7) are as defined in the formula (1). In the formulas (4) to (7), examples of the phosphoric acid group, the amino group, the alkoxy group having 1 to 10 carbon atoms, the ester group having 1 to 11 carbon atoms, the acyl group having 1 to 11 carbon atoms, and the unsubstituted linear, branched, or cyclic alkyl group having 1 to 10 carbon atoms are also as defined in the formula (1).

The method for synthesizing the cyclic carbonate (A1) is not particularly limited, and the cyclic carbonate (A1) can

be obtained by reacting corresponding diol (C1) represented by the formula (8) given below with halogenated formic acid ester or carbonic acid ester. Examples of the halogenated formic acid ester include, but are not particularly limited to, methyl chloroformate, ethyl chloroformate, methyl bromoformate, ethyl bromoformate, methyl iodoformate, and ethyl iodoformate. Examples of the carbonic acid ester include, but are not particularly limited to, dimethyl carbonate, diethyl carbonate, propyl carbonate, isopropyl carbonate, butyl carbonate, isobutyl carbonate, and diphenyl carbonate.

(C1)

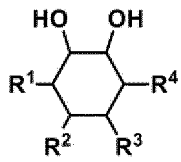

(8)

In the formula (8), $R^1$ to $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group (-OC(=O)O- group) to form a cyclic structure, wherein the alkylene group is optionally substituted by a hydroxy group, a phosphoric acid group, an amino group, an alkoxy group, or an ester group, and optionally has a carbonyl group inserted in the main chain.

In the present embodiment, preferred $R^1$ to $R^4$ in the formula (8) are as defined in the formula (1). In the formula (8), examples of the phosphoric acid group, the amino group, the alkoxy group having 1 to 10 carbon atoms, the ester group having 1 to 11 carbon atoms, the acyl group having 1 to 11 carbon atoms, and the unsubstituted linear, branched, or cyclic alkyl group having 1 to 10 carbon atoms are also as defined in the formula (1).

The cyclic carbonate (A1) can also be obtained by reacting a cyclohexene oxide derivative (C2) represented by the following formula (9) with carbon dioxide:

(C2)

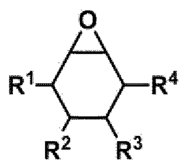

(9)

In the formula (9), $R^1$ to $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group (-OC(=O)O- group) to form a cyclic structure, wherein the alkylene group is optionally substituted by a hydroxy group, a phosphoric acid group, an amino group, an alkoxy group, or an ester group, and optionally has a carbonyl group inserted in the main chain.

In the present embodiment, preferred $R^1$ to $R^4$ in the formula (9) are as defined in the formula (1). In the formula (9), examples of the phosphoric acid group, the amino group, the alkoxy group having 1 to 10 carbon atoms, the ester group having 1 to 11 carbon atoms, the acyl group having 1 to 11 carbon atoms, and the unsubstituted linear, branched, or cyclic alkyl group having 1 to 10 carbon atoms are also as defined in the formula (1).

(Polymerization initiator)

Examples of the polymerization initiator for ring-opening polymerizing the cyclic carbonate (A1) include, but are not particularly limited to, acid catalysts, base catalysts, and enzyme catalysts. Examples of the base catalyst include, but are not particularly limited to, alkylmetals, metal alkoxides, organic acid salts of metals, and combinations of organic compounds having a HO group, a HS group or a HN group and basic substances. Examples of the alkylmetal include, but are not particularly limited to, organolithium such as methyllithium, n-butyllithium, sec-butyllithium, tertbutyllithium, and phenyllithium, methyl magnesium halide, ethyl magnesium halide, propyl magnesium halide, phenyl magnesium halide, trimethyl aluminum, and triethyl aluminum. Among them, methyllithium, n-butyllithium, or sec-butyllithium is preferably used. Examples of the metal ion in the metal alkoxide include, but are not particularly limited to, alkali metal and alkaline earth metal ions. An alkali metal is preferred. Examples of the alkoxide ion include, but are not particularly limited to, methoxide, ethoxide, propoxide, butoxide, phenoxide, and benzyl oxide. The phenoxide or the benzyl oxide may have a

substituent on the aromatic ring. Examples of the organic acid ion in the organic acid salt of a metal include, but are not particularly limited to, carboxylic acid ions having 1 to 10 carbon atoms. Examples of the metal in the organic acid salt of a metal include, but are not particularly limited to, lithium, sodium, potassium, rubidium, cesium, magnesium, calcium, and tin. Examples of the organic compound having a HO group, a HS group or a HN group include, but are not particularly limited to, alcohols such as methanol and ethanol, thiol such as methanethiol and ethanethiol, primary amine such as methylamine and ethylamine, and secondary amine such as dimethylamine and diethylamine. Examples of the basic substance include, but are not particularly limited to, organic bases. Examples of the organic base include, but are not particularly limited to, a cyclic amine such as cyclic monoamine and cyclic diamine (particularly, cyclic diamine compounds having an amidine skeleton), triamine compounds having a guanidine skeleton, and heterocyclic compounds containing a nitrogen atom. Examples of the organic base more specifically include, but are not particularly limited to, 1,4-diazabicyclo-[2.2.2]octane (DABCO), 1,8-diazabicyclo[5.4.0]undec-7-ene (DBU), 1,5-diazabicyclo[4.3.0]non-5-ene (DBN), 1,5,7-triazabicyclo[4.4.0]dec-5-ene (TBD), diphenylguanidine (DPG), N,N-dimethyl-4-aminopyridine (DMAP), imidazole, pyrimidine, and purine. Among these polymerization initiators, organolithium, a metal alkoxide, or a combination of an organic compound having a HO group, a HS group or a HN group and a cyclic amine is preferably used as the polymerization initiator in the polymerization step. The polymerization initiator of the present embodiment is preferably an alkylmetal or a metal alkoxide, more preferably an alkoxide of an alkali metal containing tert-butoxide, or n-butyllithium, from the viewpoint of more effectively and reliably exerting the advantageous effects of the present embodiment.

A polymer having a terminal alcohol residue, such as polycaprolactone diol or polytetramethylene glycol, may be used as the polymerization initiator for ring-opening polymerizing the cyclic carbonate (A1). This enables a diblock or triblock copolymer to be synthesized.

(Polymerization terminator)

In addition to the polymerization initiator, a polymerization terminator may be used from the viewpoint of controlling the average molecular weight of the resulting polycarbonate resin. Examples of the polymerization terminator include, but are not particularly limited to, inorganic acids and organic acids such as hydrochloric acid, sulfuric acid, nitric acid, methanesulfonic acid, benzenesulfonic acid, p-toluenesulfonic acid, trifluoromethanesulfonic acid, phosphoric acid, metaphosphoric acid, formic acid, acetic acid, propionic acid, butyric acid, lactic acid, citric acid, ascorbic acid, gluconic acid, oxalic acid, tartaric acid, Meldrum's acid, and benzoic acid.

(Additive)

In addition to the polymerization initiator, an additive may be used from the viewpoint of controlling the molecular weight of the resulting polymer, and from the viewpoint of exerting various characteristics by controlling a terminal structure. Examples of the additive include, but are not particularly limited to, monoalcohols such as methanol, ethanol, propanol, butanol, pentanol, hexanol, heptanol, nonanol, decanol, dodecanol, lauryl alcohol, myristyl alcohol, cetyl alcohol, stearyl alcohol, 5-norbornene-2-methanol, 1-adamantanol, 2-adamantanol, trimethylsilylmethanol, phenol, benzyl alcohol, and p-methylbenzyl alcohol, dialcohols such as ethylene glycol, 1,2-propanediol, 1,3-propanediol, 1,3-butanediol, 1,4-butanediol, hexanediol, nonanediol, tetramethylene glycol, and polyethylene glycol, polyhydric alcohols such as glycerol, sorbitol, xylitol, ribitol, erythritol, and triethanolamine as well as methyl lactate and ethyl lactate. These additives may be used singly, or in combinations of two or more thereof.

(Stirring)

In the method for producing the polycarbonate resin of the present embodiment, a reactant and/or a product is preferably stirred in the polymerization step. The stirring in the polymerization step improves homogeneity in the system and improves the frequency of contact between a growing chain and the monomer. The polycarbonate resin therefore tends to be able to be produced with a higher molecular weight. Examples of the stirring method include, but are not particularly limited to, stirring using a mechanical stirrer and a stirring blade, and stirring using a magnetic stirrer and a rotor. The stirring in the polymerization step is more preferably performed using a stirring blade from the viewpoint that the polycarbonate resin can be produced with a higher molecular weight.

(Reaction temperature)

In the method for producing the polycarbonate resin of the present embodiment, the reaction temperature in the polymerization step is not particularly limited within a range that can produce the polycarbonate resin of the present embodiment, and is preferably 0°C or higher and 150°C or lower, more preferably 0°C or higher and 130°C or lower, further preferably 0°C or higher and 120°C or lower. When the reaction temperature in the polymerization step falls within the

range described above, the weight-average molecular weight of the resulting polycarbonate resin is easier to control within the range of 50,000 or larger and 500,000 or smaller.

(Solvent)

The method for producing the polycarbonate resin of the present embodiment may or may not employ a solvent. Examples of the solvent include, but are not particularly limited to, ether solvents such as diethyl ether, diisopropyl ether, dibutyl ether, diphenyl ether, tetrahydrofuran (THF), 2-methyltetrahydrofuran, 1,4-dioxane, cyclopentyl methyl ether, tert-butyl methyl ether, and propylene glycol monomethyl ether acetate, halogen solvents such as methylene chloride, chloroform, dichloromethane, dichloroethane, and trichloroethane, saturated hydrocarbon solvents such as hexane, heptane, octane, nonane, cyclohexane, and methylcyclohexane, aromatic hydrocarbon solvents such as toluene, xylene, o-xylene, m-xylene, p-xylene, and cresol, and ketone solvents such as acetone, 2-butanone, 2-pentanone, 3-pentanone, cyclopentanone, cyclohexanone, and methyl isobutyl ketone.

In the case of obtaining a polycarbonate resin having a weight-average molecular weight (Mw) of 100,000 or larger and 500,000 smaller and a ratio (Mw/Mn) of the weight-average molecular weight (Mw) to a number-average molecular weight (Mn) of 2.5 or less, or in the case of obtaining a polycarbonate resin having a rate of decrease in weight of 30% by mass or less at 150 to 260°C when alcohol-insoluble matter is heated in a nitrogen atmosphere by thermogravimetry, the conditions of the production method are preferably as follows.

The polymerization initiator of the present embodiment is preferably an alkylmetal or a metal alkoxide, more preferably an alkoxide of an alkali metal containing tert-butoxide, or n-butyllithium. The alkoxide of an alkali metal or the n-butyllithium for use as the polymerization initiator may be in any associated state such as a monomer, a dimer, a trimer, or a tetramer.

(Reaction temperature)

In the method for producing the polycarbonate resin of the present embodiment, the reaction temperature (polymerization temperature) in the polymerization step is preferably higher than -5°C and 40°C or lower. When the reaction temperature in the polymerization step falls within the range described above, it is easier to control the weight-average molecular weight (Mw) of the resulting polycarbonate resin within the range of 100,000 or larger and 500,000 or smaller, and to adjust the ratio (Mw/Mn) of the weight-average molecular weight to the number-average molecular weight to 2.5 or less. From a similar viewpoint, the reaction temperature is preferably 0°C or higher and 40°C or lower, more preferably 0°C or higher and 35°C or lower, further preferably 0°C or higher and 30°C or lower.

(Initial monomer concentration)

In the method for producing the polycarbonate resin of the present embodiment, the initial concentration of the polymerizable monomer (initial monomer concentration) in the polymerization step is preferably 10% by mass or more. When the initial concentration of the polymerizable monomer falls within the range described above, the weight-average molecular weight (Mw) of the resulting polycarbonate resin is easier to control within the range of 100,000 or larger and 500,000 or smaller. From a similar viewpoint, the initial monomer concentration is preferably 15% by mass or more, more preferably 18% by mass or more, further preferably 20% by mass or more. The upper limit of the initial monomer concentration is not particularly limited and is, for example, 100%.

The amount of the polymerization initiator used in the polymerization step of the method for producing the polycarbonate resin of the present embodiment can be appropriately adjusted depending on the targeted molecular weight of the polycarbonate resin. The amount of the polymerization initiator used is preferably 0.0001 mol% or more and 5 mol% or less, more preferably 0.0002 mol% or more and 1 mol% or less, further preferably 0.0002 mol% or more and 0.5 mol% or less, in terms of the amount of a substance based on the ring-opening polymerizable monomer cyclic carbonate (A1) from the viewpoint of controlling the weight-average molecular weight (Mw) of the polycarbonate resin within the range of 50,000 or larger and 500,000 or smaller. These polymerization initiators described above may be used singly, or in combinations of two or more thereof.

The polycarbonate resin, the polycarbonate resin composition, and an optical molded article comprising the same according to the present embodiment have excellent heat resistance and light resistance and have excellent optical characteristics such as low birefringence, and as such, can be suitably used as various optical materials such as optical lens materials, optical devices, materials for optical components, and display materials. The polycarbonate resin, the polycarbonate resin composition, and the optical molded article comprising the same according to the present embodiment have excellent optical characteristics such as low birefringence and as such, can be suitably used, particularly, in optical members required to have a low double refraction, such as camera lenses for smartphones. Such excellent heat resistance and light resistance can prolong the lifetimes of optical members.

[Cyclic carbonate and method for producing same] (not part of the claimed invention)

As described in Background Art mentioned above, cases of cyclic carbonate made of a renewable resource have been reported. However, there is no report on cases of polyfunctional cyclic carbonate that is expected to be more effective as a cross-linking agent, a monomer, or an additive and widens the range of molecular design as a synthetic intermediate.
The present invention has been made in light of these situations. An object of the present invention is to provide a novel polyfunctional cyclic carbonate made of a renewable resource with lower environmental load.
The present inventors have conducted diligent studies to attain the object and consequently successfully synthesized a novel polyfunctional cyclic carbonate comprising an inositol skeleton made of a renewable resource.

(Cyclic carbonate) (not part of the claimed invention)

The cyclic carbonate of the present embodiment is a polyfunctional cyclic carbonate and is a compound comprising at least two carbonate groups in a molecule and comprising an inositol skeleton, the compound being represented by the following formula (11):

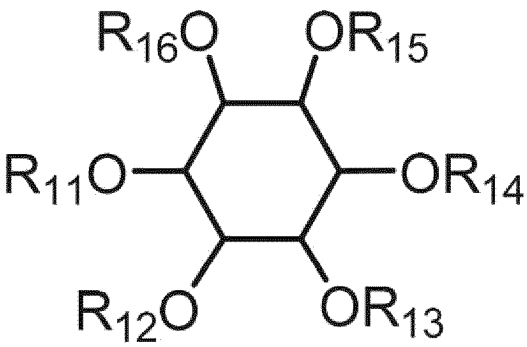

(11)

In the formula (11), $R_{11}$ to $R_{16}$ are each independently a hydrogen atom, an alkyl group having 1 to 20 carbon atoms, an alkenyl group having 2 to 20 carbon atoms, a cycloalkyl group having 3 to 20 carbon atoms, or an aryl group having 6 to 20 carbon atoms, at least two carbonate groups formed by any two of $OR_{11}$ to $OR_{16}$ are contained, and any two or more of $OR_{11}$ to $OR_{16}$ are optionally bonded to each other to form a cyclic structure.
The cyclic carbonate of the present embodiment having such a structure has a high content of a renewable resource and can reduce environmental load. Furthermore, the cyclic carbonate of the present embodiment which is polyfunctional can be used as a synthetic intermediate, an additive for resins such as epoxy, or a monomer for polycarbonate in ring-opening polymerization.
In the present embodiment, the inositol skeleton refers to a skeleton that is derived from 1,2,3,4,5,6-hexahydroxycyclohexane (trivial name: inositol) and represented by the following formula (i):

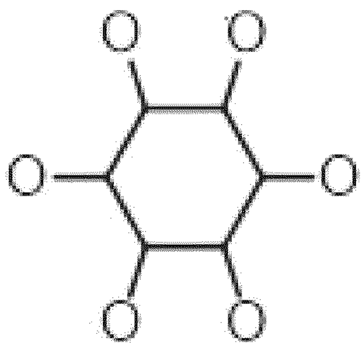

(i)

The inositol used in the present embodiment is not particularly limited as long as the inositol is capable of forming the structure of the formula (11). Examples thereof include allo-inositol, chiro-inositol, cis-inositol, epi-inositol, myo-inositol, muco-inositol, neo-inositol, and scyllo-inositol. myo-Inositol is preferred from the viewpoint of easy availability.

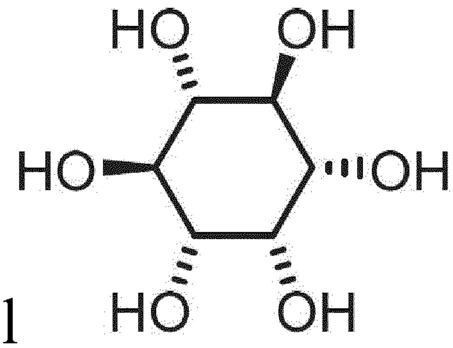

myo-Inositol

The cyclic carbonate of the present embodiment comprising an inositol skeleton may be synthesized using the inositol as a starting material or using an inositol derivative such as methyl ester or phosphoric acid (e.g., phytic acid) ester of inositol as a starting material. Such an inositol derivative is not particularly limited as long as the inositol derivative is capable of forming the structure of the formula (11). Examples thereof include bornesitol, pinitol, ononitol, pinpollitol, and quebrachitol.

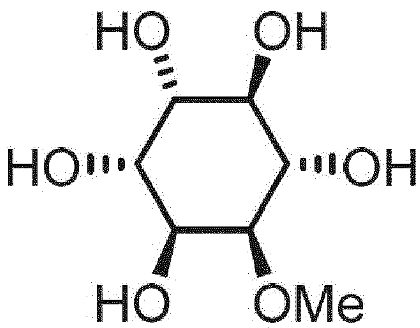

L-Quebrachitol

Next, the substituents $R_{11}$ to $R_{16}$ in the formula (11) in the cyclic carbonate of the present embodiment will be described.

In the formula (11), $R_{11}$ to $R_{16}$ are each independently a hydrogen atom, an alkyl group having 1 to 20 carbon atoms, a cycloalkyl group having 3 to 20 carbon atoms, or an aryl group having 6 to 20 carbon atoms, at least two carbonate groups formed by any two of $OR_{11}$ to $OR_{16}$ are contained, and any two or more of $OR_{11}$ to $OR_{16}$ are optionally bonded to each other to form a cyclic structure.

The alkyl group having 1 to 20 carbon atoms (hereinafter, also referred to as "C1 alkyl to C20 alkyl" in order) of $R_{11}$ to $R_{16}$ refers to a linear or branched hydrocarbon group represented by $C_nH_{2n+1}$ (n = 1 to 20) and is preferably an alkyl group having 1 to 15 carbon atoms, more preferably an alkyl group having 1 to 8 carbon atoms.

Specific examples of the C1 alkyl to C20 alkyl include, but are not limited to: a methyl group as C1 alkyl; an ethyl group as C2 alkyl; a n-propyl group and a 2-propyl group as C3 alkyl; a n-butyl group, an isobutyl group, and a t-butyl group as C4 alkyl; a n-pentyl group, a 1-ethylpropyl group, a 1-methylbutyl group, a 2-methylbutyl group, a 3-methylbutyl group, a 2,2-dimethylpropyl group, and a 1,1-dimethylpropyl group as C5 alkyl; a n-hexyl group, a 1-ethylbutyl group, a 2-ethylbutyl group, a 3-ethylbutyl group, a 1-methylpentyl group, a 2-methylpentyl group, a 3-methylpentyl group, a 4-methylpentyl group, a 1,1-dimethylbutyl group, a 2,2-dimethylbutyl group, a 3,3-dimethylbutyl group, a 1,2-dimethylbutyl group, a 1,3-dimethylbutyl group, and a 2,3-dimethylbutyl group as C6 alkyl; a n-heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1,1-dimethylpentyl group, a 2,2-dimethylpentyl group, a 3,3-dimethylpentyl group, a 4,4-dimethylpentyl group, a 1,2-dimethylpentyl group, a 1,3-dimethylpentyl group, a 1,4-dimethylpentyl group, a 2,3-dimethylpentyl group, a 2,4-dimethylpentyl group, a 3,4-dimethylpentyl group, a 2-methyl-3,3-dimethylbutyl group, a 1-methyl-3,3-dimethylbutyl group, a 1,2,3-trimethylbutyl group, a 1,3-dimethyl-2-pentyl group, and a 2-isopropylbutyl group as C7 alkyl; a n-octyl group, a 2-octyl group, a 3-octyl group, a 4-octyl group, a 2-methylheptyl group, a 3-methylheptyl group, a 4-methylheptyl group, a 5-methylheptyl group, a 6-methylheptyl group, a 2-ethylhexyl group, a 3-ethylhexyl group, a 4-ethylhexyl group, a 5-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1,2-dimethylhexyl group, a 1,3-dimethylhexyl group, a 1,4-dimethylhexyl group, a 1,5-dimethylhexyl group, a 2,3-dimethylhexyl group, a 2,4-dimethylhexyl group, a 2,5-dimethylhexyl group, a 1,1-ethylmethylpentyl group, a 2,2-ethylmethylpentyl group, a 3,3-ethylmethylpentyl group, a

4,4-ethylmethylpentyl group, a 1-ethyl-2-methylpentyl group, a 1-ethyl-3-methylpentyl group, a 1-ethyl-4-methylpentyl group, a 2-ethyl-1-methylpentyl group, a 3-ethyl-1-methylpentyl group, a 4-ethyl-1-methylpentyl group, a 2-ethyl-3-methylpentyl group, a 2-ethyl-4-methylpentyl group, a 3-ethyl-2-methylpentyl group, a 4-ethyl-3-methylpentyl group, a 3-ethyl-4-methylpentyl group, a 4-ethyl-3-methylpentyl group, a 1-(2-methylpropyl)butyl group, a 1-(2-methylpropyl)-2-methylbutyl group, a 1,1-(2-methylpropyl)ethyl group, a 1,1-(2-methylpropyl)ethylpropyl group, a 1,1-diethylpropyl group, a 2,2-diethylpropyl group, a 1,1-ethylmethyl-2,2-dimethylpropyl group, a 2,2-ethylmethyl-1,1-dimethylpropyl group, and a 2-ethyl-1,1-dimethylbutyl group as C8 alkyl; a nonyl group and an isononyl group as C9 alkyl; a decyl group and an isodecyl group as C10 alkyl; an undecyl group as C11 alkyl; a dodecyl group as C12 alkyl; a tridecyl group as C13 alkyl; a tetradecyl group as C14 alkyl; a pentadecyl group as C15 alkyl; a hexadecyl group as C16 alkyl; a heptadecyl group as C17 alkyl; an octadecyl group as C18 alkyl; a nonadecyl group as C19 alkyl; and an icosyl group as C20 alkyl.

The alkyl group as the substituents $R_{11}$ to $R_{16}$ in the formula (11) in the cyclic carbonate of the present embodiment may be a substituted alkyl group.

Specific examples of the substituted alkyl group include, but are not limited to: cycloalkyl-substituted alkyl groups such as a cyclopentylmethyl group, a cyclohexylmethyl group, a 1-cyclopentylethyl group, a 2-cyclopentylethyl group, a 1-cyclohexylethyl group, and a 2-cyclohexylethyl group; aryl-substituted alkyl groups such as a benzyl group, a phenethyl group, a dibenzylmethyl group, a methylphenylmethyl group, and a naphthylbutyl group; and haloalkyl groups such as trifluoromethyl, chloromethyl, and bromomethyl.

Each of the substituents $R_{11}$ to $R_{16}$ in the formula (11) in the cyclic carbonate of the present embodiment may be an alkenyl group having 2 to 20 carbon atoms (hereinafter, also referred to as "C2 alkenyl to C20 alkenyl" in order). The C2 to C20 alkenyl refers to a linear or branched hydrocarbon group in which the alkyl group is partially represented by an unsaturated bond. Specific examples of the C2 to C20 alkenyl include, but are not limited to: a vinyl group as C2 alkenyl; a 1-propenyl group and an allyl group as C3 alkenyl: a 2-butenyl group and a 3-butenyl group as C4 alkenyl; a 4-pentenyl group as C5 alkenyl; a 5-hexenyl group as C6 alkenyl; a 6-heptenyl group as C7 alkenyl; a 7-octenyl group as C8 alkenyl; an 8-nonenyl group as C9 alkenyl; a 9-decenyl group as C10 alkenyl; a 10-undecenyl as C11 alkenyl; a 11-dodecenyl group as C12 alkenyl; a 12-tridecenyl group as C13 alkenyl; a 14-tetradecenyl group as C14 alkenyl; a 14-pentadecenyl group as C15 alkenyl; a 15-hexadecenyl group as C16 alkenyl; a 16-heptadecenyl group as C17 alkenyl; a 17-octadecenyl group as C18 alkenyl; a 18-nonadecenyl group as C19 alkenyl; and a 19-icosenyl group as C20 alkenyl.

Each of the substituents $R_{11}$ to $R_{16}$ in the formula (11) in the cyclic carbonate of the present embodiment may be a cycloalkyl group having 3 to 20 carbon atoms. Specific examples of the cycloalkyl group having 3 to 20 carbon atoms include, but are not limited to: unsubstituted cycloalkyl groups such as a cyclopropyl group, a cyclobutyl group, a cyclopentyl group, a cyclohexyl group, a cycloheptyl group, and a cyclooctyl group; substituted cyclopentyl groups such as a 2-ethylcyclopentyl group, a 3-ethylcyclopentyl group, a 2,3-dimethylcyclopentyl group, and a 2,4-dimethylcyclopentyl group; and substituted cyclohexyl groups such as a 1-methylcyclohexyl group, a 2-methylcyclohexyl group, a 3-methylcyclohexyl group, a 4-methylcyclohexyl group, a 2,3-dimethylcyclohexyl group, a 2,3-dimethylcyclohexyl group, a 2,5-dimethylcyclohexyl group, a 2,6-dimethylcyclohexyl group, a 3,5-dimethylcyclohexyl group, a 2-ethylcyclohexyl group, a 3-ethylcyclohexyl group, and a 4-ethylcyclohexyl group.

Each of the substituents $R_{11}$ to $R_{16}$ in the formula (11) in the cyclic carbonate of the present embodiment may be an aryl group having 6 to 20 carbon atoms. Specific examples of the aryl group having 6 to 20 carbon atoms include, but are not limited to, a phenyl group, a tolyl group, a xylyl group, a trimethylphenyl group, a cumenyl group, a biphenyl group, and a naphthyl group.

Any two or more of the substituents $OR_{11}$ to $OR_{16}$ in the formula (11) in the cyclic carbonate of the present embodiment are optionally bonded to each other to form a ring structure other than a carbonate group. Examples of the form of this bonding include, but are not particularly limited to, acetal, ester, amide, imide, ether (oxide), and acid anhydride. The cyclic carbonate of the present embodiment is a cyclic carbonate having at least two carbonate groups in a molecule in which any two of $OR_{11}$ to $OR_{16}$ in the formula (11) are linked through a carbonate bond, more preferably di-O-substituted dicarbonate-inositol in which $OR_{12}$ and $OR_{13}$ as well as $OR_{15}$ and $OR_{16}$ in the formula (11) are linked through a carbonate bond.

In the cyclic carbonate of the present embodiment, for example, the bonding moieties between the cyclohexane moiety and the carbonate group in the following formula (11)' (bonding moieties indicated by the arrows in the following formula (11)') may be in any of cis and trans forms and are preferably in a trans form.

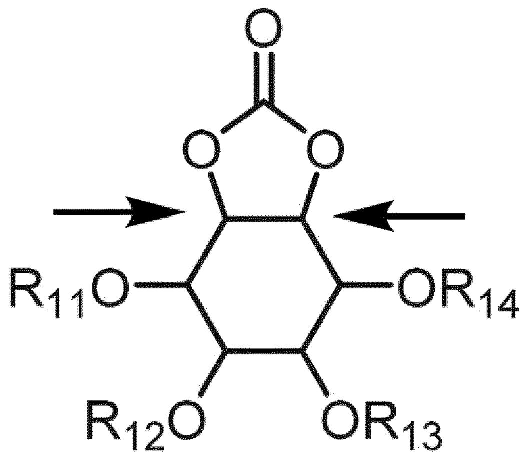

(11) '

In the present embodiment, a carbonate group in which the bonding moieties are in a cis form is referred to as a "cis carbonate group", and a carbonate group in which the bonding moieties are in a trans form is referred to as a "trans carbonate group".

The cyclic carbonate of the present embodiment comprises two carbonate groups in a molecule. At least one of the carbonate groups is preferably in a trans form (trans carbonate group) because of having a more highly reactive carbonate group, and it is more preferred to have at least one cis and one trans carbonate structures because two types of carbonate groups differing in reactivity can be used differently. Examples of the cyclic carbonate having a trans carbonate group include tetra-O-substituted trans-carbonate inositol.

The cyclic carbonate of the present embodiment is preferably a cyclic carbonate having two carbonate groups in a molecule, wherein at least one of the carbonate groups is a trans carbonate group, and the cyclic carbonate is represented by the following formula (12):

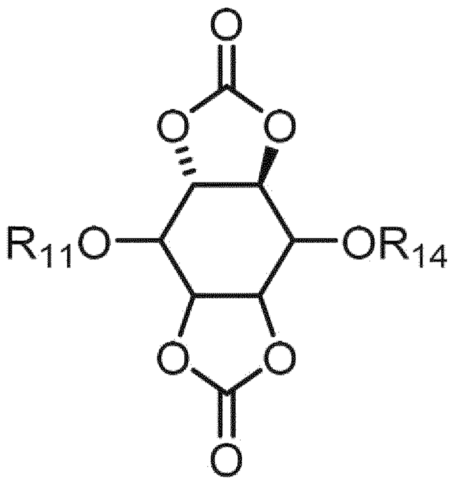

(12)

wherein $R_{11}$ and $R_{14}$ are each independently a hydrogen atom, an alkyl group having 1 to 20 carbon atoms, an alkenyl group having 2 to 20 carbon atoms, a cycloalkyl group having 3 to 20 carbon atoms, or an aryl group having 6 to 20 carbon atoms.

In the formula (12), when one of the two bonding moieties between the cyclohexane moiety and the carbonate group is indicated by a dotted line and the other moiety is indicated by a thick solid line, a trans form is adopted. When both the bonding moieties are indicated by a normal solid line, any of cis and trans forms may be adopted. The carbonate group indicated by the broken line and the thick solid line in the formula (12) shows a (1S,2S)-trans structure. The compound refers to a compound comprising a (1S,2S)-trans form and its enantiomer (1R,2R)-trans form at an arbitrary ratio, unless otherwise specified, and may optionally have a racemic structure comprising equal amounts of the (1S,2S)-trans form and the (1R,2R)-trans form. Hereinafter, this holds true for drawings of compounds containing a carbonate group.

A preferred form of the cyclic carbonate of the present embodiment is a cyclic carbonate represented by the formula (13)' given below having two carbonate groups, a cis carbonate group and a trans carbonate group, more preferably a cyclic carbonate represented by the formula (13) given below. The cyclic carbonate having such a structure

can be expected to employ the respective reactivities differently when used as an additive or a monomer.

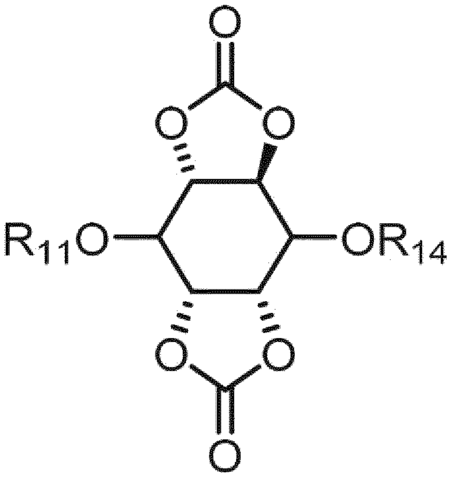

(1 3) '

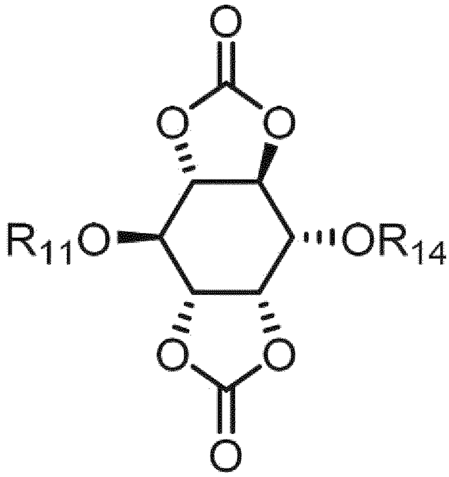

(1 3)

wherein $R_{11}$ and $R_{14}$ are each independently a hydrogen atom, an alkyl group having 1 to 20 carbon atoms, an alkenyl group having 2 to 20 carbon atoms, a cycloalkyl group having 3 to 20 carbon atoms, or an aryl group having 6 to 20 carbon atoms.

In the formulas (13)' and (13), when both the two bonding moieties between the cyclohexane moiety and the carbonate group are indicated by a dotted line, a cis form is adopted. When one of the bonding moieties is indicated by a dotted line and the other moiety is indicated by a thick solid line, a trans form is adopted. In the formula (13), the dotted line and the thick solid line that indicate the bonding moieties between the cyclohexane moiety and $R_{11}O$ or $R_{14}O$ represent the same direction of bonding as in the other dotted lines and thick solid line.

(Method for producing cyclic carbonate) (not part of the claimed invention)

The method for producing the cyclic carbonate of the present embodiment is not particularly limited as long as the cyclic carbonate represented by the formula (11) can be obtained by introducing at least two carbonate groups to a compound comprising an inositol skeleton. Various methods can be used. For example, an approach of reacting diol with phosgene, or an approach of reacting oxide with $CO_2$ in the presence of a catalyst can be used. Other examples thereof include, but are not limited to, an approach of reacting diol with a carbonate compound in the presence of an organic base or in the presence of a metal oxide catalyst as shown in Carbohydrate Research 277 (1995) C5-C7, Japanese Patent Laid-Open No. 2019-127441, and Japanese Patent Laid-Open No. 2019-127442, and an approach of reacting diol with chloroformic acid ester as shown in Polymer journal (2013) 45, 1183-1187. An approach of reacting diol with a carbonate compound or an approach of reacting diol with chloroformic acid ester is preferred because quantitative reaction can be performed and no harmful starting material is used. An approach of reacting diol with a carbonate compound is more preferred because contamination with by-products including halogen is less likely to occur.

A compound comprising an inositol skeleton, such as inositol or an inositol derivative, can be used as a starting material.

For introducing one or more carbonate groups to the compound comprising an inositol skeleton, hydroxy groups other than two hydroxy groups to which a carbonate group is introduced can be appropriately protected and deprotected. Examples of the protective group include, but are not limited to: silyl groups typified by a trimethylsilyl (TMS) group, a triethylsilyl (TES) group, a triisopropylsilyl (TIPS) group, a t-butyldimethylsilyl (TBS) group, and a t-butyldiphenylsilyl (TBDPS) group; acyl groups typified by an acetyl (Ac) group, a benzoyl (Bz) group, and a pivaloyl (Piv) group; and an acetal group, a thioacetal group, a methoxymethyl (MOM) group, an ethoxymethyl (MEM) group, a trityl (Tr) group, a benzyl group, an allyl group, and a sulfonyl group.

These protective groups can be deprotected by an approach appropriate for each protective group, such as hydrolysis under acidic conditions or basic conditions.

Specific examples of the method for introducing a carbonate group include, but are not particularly limited to, a method of reacting chloroformic acid ester with adjacent hydroxy groups of the compound comprising an inositol skeleton described above (e.g., a method of dissolving the compound comprising an inositol skeleton in an organic solvent such as 1,4-dioxane, and reacting the solution with chloroformic acid ester in the presence of a base catalyst), and an approach of reacting the compound with another carbonate compound (e.g., a method of reacting the compound comprising an inositol skeleton with alkyl carbonate in the presence of a catalyst).

In the method for synthesizing the cyclic carbonate using chloroformic acid ester, examples of the chloroformic acid ester include, but are not particularly limited to, methyl chloroformate, ethyl chloroformate, vinyl chloroformate, allyl chloroformate, and phenyl chloroformate. Methyl chloroformate and ethyl chloroformate are preferred, and ethyl chloroformate is more preferred.

The amount of the chloroformic acid ester added is preferably 1 equivalent or more, more preferably 1 to 2 equivalents, particularly preferably 1.2 to 1.7 equivalents, per two hydroxy groups of the inositol compound to which a carbonate group is introduced.

The reaction may be performed without a solvent or may be performed in the presence of an organic solvent and is preferably performed in the presence of an organic solvent. Examples of the organic solvent for use in reaction that is performed in the presence of an organic solvent include, but are not particularly limited to, aliphatic hydrocarbon, halogenated hydrocarbon, aromatic hydrocarbon, halogenated aromatic hydrocarbon, and ether and preferably include: aromatic hydrocarbon such as toluene and xylene; halogenated hydrocarbon such as methylene chloride, and 1,2-dichloroethane; and ether such as diethyl ether, tetrahydrofuran, and 1,4-dioxane. The amount of the solvent is preferably 1 ml or more, more preferably 2 to 50 ml, further preferably 2 to 10 ml, per g of the inositol compound.

In the method for synthesizing the cyclic carbonate using chloroformic acid ester, the base catalyst used may be any of inorganic and organic bases. These may be used singly or may be used as a mixture. Examples of the inorganic base used include, but are not particularly limited to, potassium carbonate, sodium carbonate, calcium hydroxide, and calcium oxide. Examples of the organic base include, but are not particularly limited to, aliphatic tertiary amine, unsubstituted or substituted imidazole, pyridine, and pyrimidine and particularly include trialkylamines such as trimethylamine, triethylamine, tripropylamine, and tributylamine. Trialkylamines such as triethylamine are preferred, and triethylamine is more preferred.

The amount of the base catalyst used is preferably 0.8 to 5 mol, more preferably 1 to 3 mol, particularly preferably 1 to 1.5 mol, per mol of the inositol compound because the formation of by-products is suppressed.

In the method for synthesizing the cyclic carbonate using chloroformic acid ester, the reaction temperature is preferably -30 to 50°C, more preferably 0 to 10°C. The reaction pressure is preferably atmospheric pressure, and the reaction time is preferably 1 to 24 hours, more preferably 6 to 12 hours. The reaction is preferably performed in an inert gas atmosphere such as an argon atmosphere or a nitrogen atmosphere.

The reaction can be performed by a common method such as a batch, semi-batch, or continuous scheme. After the completion of reaction, the reaction product cyclic carbonate comprising an inositol skeleton can be separated and purified by an approach such as concentration, distillation, extraction, crystallization, recrystallization, ion exchange, or a silica gel column.

In the method for synthesizing the cyclic carbonate using alkyl carbonate, examples of the alkyl carbonate used include, but are not particularly limited to: a chain dialkyl carbonate typified by dimethyl carbonate, diethyl carbonate, ethylmethyl carbonate, and diphenyl carbonate; and a cyclic carbonate typified by ethylene carbonate, propylene carbonate, and trimethylene carbonate. Chain dialkyl carbonate is preferred, and diphenyl carbonate is more preferred.

In the method for synthesizing the cyclic carbonate using alkyl carbonate, examples of the catalyst used include, but are not particularly limited to: metal oxide typified by beryllium oxide, magnesium oxide, calcium oxide, strontium oxide, barium oxide, lanthanum oxide, zinc oxide, aluminum oxide, cerium oxide, iron oxide, cobalt oxide, nickel oxide, silicon oxide, copper oxide, and silver oxide; and organic bases typified by triethylamine, tripropylamine, tributylamine, tripentylamine, 4-ethylmorpholine, picoline, pyridine, N,N-dimethyl-4-aminopyridine, diazabicycloundecene, dipropylamine, dibutylamine, propylamine, butylamine, morpholine, pyrimidine, imidazole, and aniline. Metal oxide is preferred,

alkaline earth metal oxide typified by calcium oxide, magnesium oxide, and barium oxide is more preferred, and calcium oxide is further preferred.

In the method for synthesizing the cyclic carbonate using alkyl carbonate, the amount of the catalyst used in the step of producing the cyclic carbonate comprising an inositol skeleton is, for example, preferably 0.05 g to 1.0 g, more preferably 0.05 g to 0.8 g, further preferably 0.1 g to 0.5 g, per g of the starting material inositol.

The amount of the alkyl carbonate used is, for example, preferably 0.5 equivalents to 10 equivalents, more preferably 1.0 equivalent to 4.0 equivalents, further preferably 1.1 equivalents to 2.0 equivalents, per two hydroxy groups to which a carbonate group is introduced, of the starting material inositol. The reaction temperature is, for example, preferably 30°C to 250°C, more preferably 30°C to 180°C, further preferably 90°C to 180°C. The reaction pressure is preferably atmospheric pressure, and the reaction time is preferably 1 to 24 hours, more preferably 1 to 6 hours. The reaction is preferably performed in an inert gas atmosphere such as an argon atmosphere or a nitrogen atmosphere.

The reaction can be performed by a common method such as a batch, semi-batch, or continuous scheme. After the completion of reaction, the reaction product cyclic carbonate comprising an inositol skeleton can be separated and purified by a separation approach such as concentration, distillation, extraction, crystallization, or recrystallization, or a combined separation approach thereof.

In the present embodiment, the cyclic carbonate can be quantified by a technique well known to those skilled in the art. Examples thereof include methods based on $^{1}$H-NMR, $^{13}$C-NMR, high-performance liquid chromatography, or gas chromatography.

## Examples

The present invention will be described further specifically with reference to Examples and Comparative Examples. However, the present invention is not limited by these Examples, etc. by any means.

### [Polycarbonate resin]

In the present specification, the physical properties of a polycarbonate resin were measured as follows.

($^{1}$H-NMR measurement)

The $^{1}$H-NMR spectrum of a polycarbonate resin was obtained by NMR measurement as described below using an NMR apparatus manufactured by JEOL Ltd. (product name: ECZ400S), and a TFH probe. The reference peak of a deuterated solvent was 7.26 ppm in the case of using chloroform-d and 2.50 ppm in the case of using dimethyl sulfoxide-d6. The measurement was performed with the number of scans set to 32.

(Measurement of ratio of mr / (mm + mr + rr))

A solution of 1 g of chloroform-d added to 0.1 g of a polycarbonate resin was used as a measurement sample. $^{13}$C-NMR measurement was performed as described below using an NMR apparatus manufactured by JEOL Ltd. (product name: ECZ400S), and a TFH probe. mm represents an isotactic moiety in a periodic meso structure of the monomer, rr represents a syndiotactic moiety in a periodic structure based on a racemo bond, and mr represents a moiety in which the meso moiety and the racemo moiety are bonded, which were measured by this $^{13}$C-NMR. The reference peak of the deuterated solvent was 77.0 ppm. The measurement was performed with the number of scans set to 4000.

As for peaks observed at 21 to 24 ppm of the obtained $^{13}$C-NMR spectrum, an integration baseline was drawn in which the left end of the mm peak on the lowest magnetic field side was regarded as 23.5 ppm and the right end of the rr peak on the highest magnetic field side was regarded as 21.7 ppm. Subsequently, the peaks were cut at the valleys between mm and mr (around 22.0 ppm) and between mr and rr (around 22.5 ppm). In this respect, when the integrated intensity ratio of the mm peak on the highest magnetic field was defined as 1, the integrated intensity ratios of mr and rr were substituted into mr / (mm + mr + rr) to calculate randomness.

(Measurement of molecular weight - (1))

A weight-average molecular weight (Mw) and a number-average molecular weight (Mn) to be measured by size-exclusion chromatography using polymethyl methacrylate (hereinafter, also referred to as "PMMA") as a standard sample was measured as described below.

A solution of 2.0 g of tetrahydrofuran added to 0.02 g of a polycarbonate resin was used as a measurement sample. The weight-average molecular weight of the polycarbonate resin was measured using an HPLC apparatus (manufactured by Waters Corp., product name "Alliance e2695"). The columns used were TSK guard columns SuperH-H,

TSKgel SuperHM-H, TSKgel SuperHM-H, TSKgel SuperH2000, and TSKgel SuperH1000 manufactured by Tosoh Corp. (all of which are product names of Tosoh Corp.) connected in series. The column temperature was 40°C, and tetrahydrofuran was used as a mobile phase. Analysis was conducted at a rate of 0.35 mL/min. The detector used was an RI detector. A calibration curve was prepared using polymethyl methacrylate standard samples manufactured by Polymer Standards Service GmbH (molecular weight: 2200000, 988000, 608000, 340000, 202000, 88500, 41400, 18700, 9680, 5050, 2380, and 800) as standard samples. The number-average molecular weight and the weight-average molecular weight of the polycarbonate resin were determined on the basis of the calibration curve thus prepared.

(Measurement of molecular weight - (2))

A weight-average molecular weight (Mw) and a number-average molecular weight (Mn) were measured as described below as to a polymer component having a polystyrene (hereinafter, also referred to as "PS")-based molecular weight of 1,000 or larger measured by size-exclusion chromatography.

A solution of 2.0 g of tetrahydrofuran added to 0.02 g of a polycarbonate resin was used as a measurement sample. A high-performance GPC apparatus (manufactured by Tosoh Corp., product name "HLC-8420GPC") was used. The columns used were TSK guard columns SuperH-H, TSKgel SuperHM-H, TSKgel SuperHM-H, TSKgel SuperH2000, and TSKgel SuperH1000 manufactured by Tosoh Corp. (all of which are product names of Tosoh Corp.) connected in series. The column temperature was 40°C, and tetrahydrofuran was used as a mobile phase. Analysis was conducted at a rate of 0.60 mL/min. The detector used was an RI detector. A calibration curve was prepared using polystyrene standard samples manufactured by Polymer Standards Service GmbH (molecular weight: 2520000, 1240000, 552000, 277000, 130000, 66000, 34800, 19700, 8680, 3470, 1306, and 370) as standard samples. The number-average molecular weight and the weight-average molecular weight of the polymer component having a polystyrene-based molecular weight of 1,000 or larger in the polycarbonate resin were determined on the basis of the calibration curve thus prepared.

(Thermogravimetry)

Approximately 10 mg of methanol-insoluble matter of a polycarbonate resin obtained in each of Examples and Comparative Examples mentioned later was used as a measurement sample. The thermogravimetry of the polycarbonate resin was performed by using a simultaneous differential thermal and thermogravimetric measurement apparatus (manufactured by Shimadzu Corp., product name "DTG-60A") and elevating the temperature from 40°C to 400°C at 10°C/min in a nitrogen atmosphere. The rate of decrease in weight (% by mass) at 150 to 260°C was calculated from the obtained results.

(Fractionation and structural analysis of low-molecular-weight component)

A polycarbonate resin was prepared into a 30 mg/mL solution in chloroform and fractionated using a preparative GPC apparatus (manufactured by Japan Analytical Industry Co., Ltd., product name "LC-908"). The column used was JALGEL3H (manufactured by Japan Analytical Industry Co., Ltd.), and chloroform was used as a mobile phase. Analysis was conducted at a rate of 3.33 mL/min. Fractions obtained by fractionation were adjusted within the range of 4 mg/mL to 10 mg/mL, and trans-2-[3-(4-tert-Butylphenyl)-2-methyl-2-propenylidene]malononitrile (DCTB) and sodium trifluoroacetate (NaTFA) were adjusted to 10 mg/mL and 1 mg/mL, respectively, with tetrahydrofuran. Then, these solutions were mixed at 1:1:1 and added dropwise to a plate for measurement. Air-dried samples were analyzed using a MALDI-TOFMS analysis apparatus (manufactured by Bruker, product name "UltrafleXtreme"). Mass values were corrected using a sample of polyethylene glycol (1 mg/mL):DCTB (10 mg/mL):NaTFA (10 mg/mL) = 1:1:1 dissolved in THF. When a structurally predictable component was present, the mass values were corrected again using the component.

(Measurement of glass transition temperature)

A polycarbonate resin dried in vacuum was compression-molded at room temperature at a pressure of 200 $kgf/cm^2$. A 10 mg aliquot was cut out of the compression-molded polycarbonate resin and used as a test piece. Measurement was performed using a differential scanning calorimetric apparatus manufactured by PerkinElmer Co., Ltd. (product name "DSC8500") under conditions involving a nitrogen gas flow rate of 20 mL/min. More specifically, for example, a sample of Example A2 was kept at 40°C for 3 minutes and then warmed from 40°C to 160°C at 20°C/min for primary temperature elevation to completely melt the sample. Then, the sample was cooled from 160°C to 40°C at 50°C/min and kept at 40°C for 5 minutes. Subsequently, the sample was warmed from 40°C to 150°C at 10°C/min for secondary temperature elevation. The point of intersection (midpoint glass transition temperature) between a partial curve of step-like change in a DSC curve drawn by the secondary temperature elevation and a straight line pitched at equal distances in the ordinate direction from extended lines of respective tangents was regarded as a glass transition

temperature (Tg). The glass transition temperatures of samples of the other Examples and Comparative Examples were measured in the same manner as above.

(Preparation of undrawn sample and drawn sample)

First, an undrawn sample of a polycarbonate resin was prepared using a vacuum compression molding machine. Specifically, the sample was prepared as described below. The polycarbonate resin was placed in a 25 to 150 μm thick polyimide frame and sandwiched between two polyimide films, two aluminum sheets and two iron sheets to obtain a laminate. In this respect, the order of lamination for the laminate was the iron sheet, the aluminum sheet, the polyimide film, the polyimide frame, the polyimide film, the aluminum sheet, and the iron sheet. The laminate described above was loaded in a vacuum compression molding machine (manufactured by Shinto Metal Industries, Ltd., model SFV-30), preheated at a predetermined temperature shown in Table 1 under reduced pressure (10 kPa) for 5 minutes, and then compressed at the predetermined temperature for 10 minutes under conditions involving a press pressure of 10 MPa while the reduced pressure conditions were maintained. After cancelation of the reduced pressure and the press pressure, the compressed laminate was transferred to a compression molding machine for cooling (manufactured by Shinto Metal Industries, Ltd., model AYS-10) and solidified by cooling to obtain a press film having a thickness of 20 to 250 μm. The obtained press film was cured for 24 hours in a constant temperature and humidity room of 23°C and 50% humidity to obtain an undrawn sample.

Next, the undrawn sample was cut into a width of 30 mm and a length of 50 mm and placed in a tensile test jig. The tensile test jig was loaded in a tester manufactured by Shimadzu Corp. (product name "Autograph AG-5kNXPlus") connected with a thermostat bath (product name "TCR1W-200T"), and uniaxially freely drawn under the following conditions: the drawing conditions involved a distance of 30 mm between chucks, a drawing temperature of Tg + 20°C, a drawing rate of 500 mm/min, and a draw ratio of 100% and 300%. The sample thus uniaxially freely drawn was immediately taken out thereof and cooled at room temperature and then cured for 24 hours in a constant temperature and humidity room of 23°C and 50% humidity to obtain a drawn film having a thickness of 10 to 500 μm. A few samples of the drawn film obtained by the procedures were prepared. Then, the thickness of each film was measured using a thickness gauge (manufactured by Mitutoyo Corp., "Digimatic Thickness Gauge") .

(Measurement of haze)

A haze was measured as described below using the 300% uniaxially drawn film. For the haze, blank measurement was performed using a turbidimeter manufactured by Nippon Denshoku Industries Co., Ltd. (product name "NDH5000W"), and the haze of the 300% uniaxially drawn film was then measured. The haze of each sample was determined by measuring three different measurement positions of the same test piece and described as a mean from the three measurements.

(Measurement of in-plane phase difference and rate of orientation double refraction)

An in-plane phase difference and a rate of orientation double refraction were measured as described below using the 100% uniaxially drawn film. First, the absolute value of the in-plane phase difference of each drawn film was measured at a wavelength of 587 nm using a phase difference measurement apparatus manufactured by Oji Scientific Instruments Co., Ltd. (product name "KOBRA-WR"). Then, the rate of orientation double refraction was calculated using the obtained absolute value of the in-plane phase difference and the thickness of each drawn film. The absolute value of the in-plane phase difference in terms of a thickness of 100 μm was calculated using the obtained rate of orientation double refraction. The rate of orientation double refraction, the absolute value of the in-plane phase difference, the absolute value of the in-plane phase difference in terms of a thickness of 100 μm, and the thickness of the drawn film satisfy the following expressions (A), (B) and (C):

$$\Delta n = nx - ny \qquad (A)$$

$$Re = \Delta n \times d \qquad (B)$$

$$Re_{100}\ (nm) = \Delta n \times 1.0 \times 10^5 \qquad (C)$$

(Δn: rate of orientation double refraction, nx: refractive index in the direction of drawing, ny: refractive index in a direction perpendicular to the direction of drawing within the plane of the sample, Re: absolute value of the in-plane phase difference, $Re_{100}$: absolute value of the in-plane phase difference in terms of a thickness of 100 μm, d: thickness of the

drawn film)

(Measurement of photoelastic coefficient)

The undrawn sample obtained in the section "Preparation of undrawn sample and drawn sample" was cut into a width of 6 mm and a length of 30 mm and used as a sample. Its photoelastic coefficient was measured as described below. For the detailed measurement method, see Polymer Engineering and Science 1999, 39, 2349-2357. Specifically, the sample described above was arranged in a film tensile apparatus (manufactured by Imoto Machinery Co., Ltd.) placed in a constant temperature and humidity room of 23°C and 50% humidity such that the distance between chucks was 20 mm. Subsequently, a double refraction measurement apparatus (manufactured by Otsuka Electronics Co., Ltd., product name "RETS-100") was arranged such that the light path of the double refraction measurement apparatus was positioned at the central portion of the sample. The distance between chucks was 20 mm, and the chuck movement speed was 0.1 mm/min. The rate of double refraction of the test piece was measured at a wavelength of 550 nm while elongational stress was applied thereto. The photoelastic coefficient ($Pa^{-1}$) was calculated from the relationship between the measured rate of double refraction and the elongational stress by use of the least-square method. For the calculation, data on elongational stress $\sigma$ of 2.5 MPa $\leq \sigma \leq$ 10 MPa was used. The rate of double refraction, the elongational stress, and the photoelastic coefficient satisfy the following expressions (D) and (E):

$$\Delta n = nx - ny \quad (D)$$

$$C = \Delta n / \sigma \quad (E)$$

($\Delta n$: rate of double refraction, nx: refractive index in the direction of drawing, ny: refractive index in a direction perpendicular to the direction of drawing within the plane of the sample, C: photoelastic coefficient, $\sigma$: elongational stress)

(Synthesis of trans-cyclohexene carbonate)

trans-1,2-Cyclohexanediol (200.0 g, 1.722 mmol) and dehydrated 1,4-dioxane (2.0 L) were added to a 5 L four-neck flask under argon stream. Subsequently, ethyl chloroformate (280.2 g, 2.582 mmol) was slowly added dropwise into the reaction solution while the reaction solution was stirred using a mechanical stirrer and the reaction container was cooled in an ice bath. Further, a solution of triethylamine (348.4 g, 3.443 mmol) diluted with dehydrated toluene (2.5 L) was slowly added dropwise into the reaction solution with stirring and cooling maintained. After the dropwise addition, the reaction solution was stirred for 1.5 hours with cooling maintained. Then, the internal temperature of the reaction container was elevated to room temperature, and the reaction solution was further stirred for 12 hours. A secondarily produced white solid was removed by filtration under reduced pressure, and the filtrate was concentrated under reduced pressure. The residue was dissolved by the addition of ethyl acetate (2.0 L) and then washed with an aqueous solution containing 1% by mass of hydrochloric acid (2.0 L). The organic layer was recovered and washed with ion-exchange water (2.0 L) three times. The resultant was further dehydrated by the addition of magnesium sulfate and filtered under reduced pressure. The filtrate was concentrated under reduced pressure, and the obtained white solid was purified by silica gel column chromatography to obtain the trans-cyclohexene carbonate (110 g) of interest.

[Example A1]

The synthesized trans-cyclohexene carbonate (5.02 g, 35.3 mmol) was added to a 50 mL three-neck flask, and the flask was purged with nitrogen. The flask was heated to 60°C to melt the contents. Then, a solution of potassium tert-butoxide in THF (0.18 mL, 1.0 M, 0.18 mmol) was slowly added thereto with stirring using a mechanical stirrer and a stirring blade, and the mixture was stirred at 60°C for 6 hours. The flask was allowed to cool to room temperature. Then, the contents were dissolved by the addition of 30 mL of chloroform, and a trace amount of insoluble matter was removed by filtration through a cotton plug.

Subsequently, the filtrate was added into 300 mL of methanol to deposit a polymer. The deposited polymer was recovered and dissolved again in 60 mL of chloroform. Then, the solution was added dropwise to 1400 mL of methanol for reprecipitation. The polymer was recovered by filtration under reduced pressure and washed with methanol. The obtained polymer was dried in vacuum at 60°C for 2 hours to obtain 2.72 g of a homopolymer (polycarbonate resin). Figure 1 shows results of measuring the obtained homopolymer with a differential scanning calorimetric apparatus. As a result of measuring the $^{1}$H-NMR spectrum of the obtained homopolymer, a spectrum similar to that in Example A3 mentioned later was obtained. Also, the $^{13}$C-NMR spectrum of the obtained homopolymer was measured, and the ratio of mr / (mm + mr + rr) was calculated to be 49%.

[Example A2]

2.76 g of a homopolymer (polycarbonate resin) was obtained by polymerization in the same manner as in Example A1 except that stirring was performed using a magnetic stirrer and a rotor. As a result of measuring the $^1$H-NMR spectrum of the obtained homopolymer, a spectrum similar to that in Example A3 mentioned later was obtained. The polycarbonate resin of Example A2 was fractionated by preparative GPC, and chemical species having a hydroxycyclohexyloxy group, a tert-butoxy group, or a methoxy group as a terminal alkoxy group were observed in the MALDI-TOF-MS spectrum of fractions. Among them, the hydroxycyclohexyloxy group (around 3.7 ppm) and the methoxy group (around 3.6 ppm) were calculated to be 0.10 mol% and 0.58 mol%, respectively, in order per 100 mol% of the polymer main chain from integrated intensity ratios in the $^1$H-NMR spectrum. The MALDI-TOF-MS spectrum is shown in Figure 5.

[Example A3]

3.17 g of a homopolymer (polycarbonate resin) was obtained through polymerization reaction in the same manner as in Example A1 except that: trans-cyclohexene carbonate (5.04 g, 35.4 mmol) was used instead of the trans-cyclohexene carbonate (5.02 g, 35.3 mmol); and m-xylene (5.06 g) was added into the flask after purging of the flask with nitrogen and before heating of the flask to 60°C such that the initial monomer concentration became 50% by mass. Figure 2 shows the $^1$H-NMR spectrum of the obtained homopolymer. Also, the $^{13}$C-NMR spectrum of the obtained homopolymer was measured, and the ratio of mr / (mm + mr + rr) was calculated to be 48%.

[Example A4]

1.04 g of a copolymer (polycarbonate resin) was obtained through polymerization reaction in the same manner as in Example A1 except that trans-cyclohexene carbonate (2.19 g, 15.4 mmol) and 1,4-di-O-octyl-2,3:5,6-dicarbonate-myo-inositol (0.217 g, 0.475 mmol) were used instead of the trans-cyclohexene carbonate (5.02 g, 35.3 mmol) .

The proportion of a component derived from each monomer in the copolymer was calculated by $^1$H-NMR measurement. In the $^1$H-NMR spectrum of a sample dissolved in chloroform-d, a signal assigned to 2H of methylene hydrogen on the cyclohexene carbonate skeleton was observed at 1.9 to 2.3 ppm. Likewise, a signal assigned to 6H of hydrogen of methyl groups on the di-octyl-myo-inositol skeleton was observed at 0.8 to 0.9 ppm. The proportion of the di-octyl-myo-inositol skeleton in the copolymer was calculated to be 4% from the integrated intensity ratios of these signals. Figure 3 shows the $^1$H-NMR spectrum of the obtained copolymer. Also, the $^{13}$C-NMR spectrum of the obtained copolymer was measured, and the ratio of mr / (mm + mr + rr) was calculated to be 49%.

[Comparative Example A1]

6.61 g of a homopolymer (polycarbonate resin) was obtained through polymerization reaction in the same manner as in Example A3 except that: trans-cyclohexene carbonate (10.4 g, 73.0 mmol) was used instead of the trans-cyclohexene carbonate (5.04 g, 35.4 mmol); and m-xylene(10.4 g) having a water content of approximately 100 ppm was added into the flask. As a result of measuring the $^1$H-NMR spectrum of the obtained homopolymer, a spectrum similar to that in Example A3 was obtained.

[Comparative Example A2]

In Comparative Example A2, a bisphenol A-type polycarbonate resin (manufactured by Mitsubishi Engineering-Plastics Corp., product name "Iupilon S-3000") was used.

[Comparative Example A3]

In Comparative Example A3, a poly(cyclohexene carbonate) resin (manufactured by Empower Materials, Inc., product name "QPAC130") was used which was synthesized by the copolymerization of cyclohexene oxide and carbon dioxide. Also, its $^{13}$C-NMR spectrum was measured, and the ratio of mr / (mm + mr + rr) was calculated to be 36%. Figure 4 shows a MALDI-TOF-MS spectrum at m/z 1500 to 1600 among components obtained by fractionation using a preparative GPC apparatus.

Table 1 shows the physical properties of the polycarbonate resins obtained in Examples and Comparative Examples mentioned above.

[Table 1]

| | Example A1 | Example A2 | Example A3 | Example A4 | Comparative Example A1 | Comparative Example A2 | Comparative Example A3 |
|---|---|---|---|---|---|---|---|
| Film preparation temperature (°C) | 210 | 150 | 210 | 200 | 150, 210 | - | - |
| Film preparation results | ○ | ○ | ○ | ○ | × | - | - |
| Mn (PMMA based) | 45000 | 31000 | 49000 | 39000 | 19000 | 30000 | 49000 |
| Mw (PMMA based) | 124000 | 58000 | 98000 | 82000 | 37000 | 68000 | 162000 |
| Mn (PS based) | 56000 | 29000 | 52000 | 36000 | 19000 | 28000 | 45000 |
| Mw (PS based) | 109000 | 44000 | 101000 | 71000 | 32000 | 58000 | 134000 |
| Mw/Mn (PS based) | 2.0 | 1.5 | 1.9 | 2.0 | 1.7 | 2.1 | 3.0 |
| Tg (°C) | 120 | 99 | 104 | 108 | - | 145 | - |
| Photoelastic coefficient ($\times 10^{-12}$/Pa) | 1.7 | 4.4 | 1.7 | 2.6 | - | 70 | - |
| In-plane phase difference (nm) | 40.9 | 29.9 | 49.1 | 56.1 | - | 2010 | - |
| Presence or absence of heterogeneous bonding in main chain | Absent | Absent | Absent | - | - | - | Present |
| Terminal alkoxy group | Methoxy (C1), tert-Butyl (C4) | Methoxy (C1), tert-Butyl (C4) | Methoxy (C1), tert-Butyl (C4) | Methoxy (C1), tert-Butyl (C4) | Methoxy (C1), tert-Butyl (C4) | - | Absent |
| Thickness of 300% uniaxially drawn film (μm) | - | - | 40 | - | - | - | 40 |
| Haze of 300% uniaxially drawn film (%) | 1.78 | 1.76 | 1.50 | 1.19 | - | - | 23.75 |
| mr/(mm+mr+rr),% | 49 | - | 48 | 49 | - | - | 36 |
| Rate of decrease in weight at 150°C to 260°C (nitrogen atmosphere), % by mass | 21.0 | 9.3 | 15.5 | 2.3 | 50.4 | - | - |

The polycarbonate resin of Comparative Example A1 was impossible to mold into a film due to a very fragile molded piece in an attempt to prepare an undrawn sample at a temperature of 150°C or 210°C using a vacuum compression molding machine. Thus, the optical characteristics of the polycarbonate resin of Comparative Example

A1 were impossible to evaluate.

From Table 1, the polycarbonate resins of Examples A1 to A4 had a lower photoelastic coefficient and in-plane phase difference than those of the polycarbonate resin of Comparative Example A2, demonstrating that the polycarbonate resins of Examples A1 to A4 exhibit low birefringence. Furthermore, the polycarbonate resins of Examples A1 to A4 had Tg equivalent to that of the bisphenol A-type polycarbonate resin of Comparative Example A1, demonstrating that these polycarbonate resins possess sufficient heat resistance.

($^1$H-NMR spectrum of Comparative Example A3)

In the $^1$H-NMR spectrum of the polycarbonate resin of Comparative Example A3, a plurality of broad peaks which were not seen in Examples A1 to A3 were observed around 3 to 5 ppm derived from methine hydrogen of the poly(cyclohexene carbonate). These peaks are presumably derived from the heterogeneous bonding of a polyether structure, etc. involving no carbon dioxide, which may be formed by the copolymerization of epoxide and carbon dioxide as in Comparative Example A3, unlike the ring-opening polymerization of the cyclic carbonate monomer shown in Examples A1 to A3.

(Presence of heterogeneous bonding in Comparative Example A3)

In the MALDI-TOF-MS spectrum of fractions obtained by fractionating the polycarbonate resin of Comparative Example A3 by preparative GPC, chemical species containing one or more non-terminal ether bonds involving no carbon dioxide were observed. Such heterogeneous bonding other than a carbonate bond was not observed in the MALDI-TOF-MS spectrum of the poly(cyclohexene carbonate) synthesized by the ring-opening polymerization of a cyclic carbonate as shown in Examples A1 to A3.

(Haze of 300% uniaxially drawn film)

As a result of measuring the haze of the 300% uniaxially drawn film as to the polycarbonate resins of Examples mentioned above and the polycarbonate resin of Comparative Example A3, a 10 or more times higher value was obtained in the polycarbonate resin of Comparative Example A3 compared with the polycarbonate resins of Examples. This is presumably due to difference in partial structure constituting the polymer main chain as seen in the NMR spectrum or the MALDI-TOF-MS spectrum mentioned above.

[Example A5]

The synthesized trans-cyclohexene carbonate (10.1 g, 71.2 mmol) was added to a 50 mL separable flask, and the flask was purged with nitrogen. The flask was dipped in a thermostat bath of 25°C. m-Xylene (40.2 g) was added into the flask, and the mixture was stirred using a mechanical stirrer and a stirring blade so that the monomer was completely dissolved to obtain a monomer solution. Aside from this, a potassium tert-butoxide solution (1.0 M, 0.28 mL, 0.28 mmol) and benzyl alcohol (0.063 g, 0.58 mmol) were weighed into a dried 30 mL Schlenk flask and diluted with 2.6 mL of m-xylene to prepare a polymerization initiator solution. While the monomer solution was stirred, 0.27 mL of the prepared polymerization initiator solution was added at once to the monomer solution. The mixture was stirred at 25°C for 30 minutes for polymerization reaction. The reaction was terminated by the addition of 0.012 g of acetic acid to obtain a polymerization solution containing a polycarbonate resin. A portion of the obtained polymerization solution was sampled and diluted with chloroform-d. The $^1$H-NMR spectrum of the sample was measured, and the rate of monomer conversion was calculated to be 94% from the integrated intensity ratios of a monomer-derived peak at 4.0 ppm and a polymer-derived peak at 4.6 ppm.

Subsequently, a reprecipitation operation was performed as described below for the evaluation of methanol-insoluble matter. 0.52 mL of the polymerization solution was sampled and diluted by the addition of 2.13 g of m-xylene. The diluted solution was added into 26.57 g of methanol to deposit a polymer. The deposited polymer was recovered by filtration under reduced pressure. The obtained polymer was dried in vacuum at 130°C for 2 hours to obtain a homopolymer (polycarbonate resin). The rate of decrease in weight was determined from thermogravimetry results when the obtained homopolymer was heated in a nitrogen atmosphere.

[Example A6]

A polycarbonate resin was obtained by polymerization reaction and a reprecipitation operation in the same manner as in Example A5 except that the polymerization temperature was set to 10°C. The rate of monomer conversion was calculated to be 96% from the $^1$H-NMR spectrum after the termination of reaction. The rate of decrease in weight was

determined from thermogravimetry results when the homopolymer (polycarbonate resin) obtained by the reprecipitation operation was heated in a nitrogen atmosphere.

[Example A7]

A polycarbonate resin was obtained by polymerization reaction and a reprecipitation operation in the same manner as in Example A5 except that the polymerization temperature was set to 5°C. The rate of monomer conversion was calculated to be 97% from the $^1$H-NMR spectrum after the termination of reaction. The rate of decrease in weight was determined from thermogravimetry results when the homopolymer (polycarbonate resin) obtained by the reprecipitation operation was heated in a nitrogen atmosphere.

[Example A8]

A polycarbonate resin was obtained by polymerization reaction and a reprecipitation operation in the same manner as in Example A5 except that the polymerization temperature was set to 0°C. The rate of monomer conversion was calculated to be 97% from the $^1$H-NMR spectrum after the termination of reaction. The rate of decrease in weight was determined from thermogravimetry results when the homopolymer (polycarbonate resin) obtained by the reprecipitation operation was heated in a nitrogen atmosphere.

[Comparative Example A5]

A polycarbonate resin was obtained by polymerization reaction in the same manner as in Example A5 except that the polymerization temperature was set to -5°C. The rate of monomer conversion was calculated to be 5% from the $^1$H-NMR spectrum after the termination of reaction. A polycarbonate resin having weight-average molecular weight Mw (PMMA-based) of 50,000 or larger was not obtained under the conditions of Comparative Example A5.

[Comparative Example A6]

A polycarbonate resin was obtained by polymerization reaction in the same manner as in Example A5 except that the polymerization temperature was set to -10°C. The rate of monomer conversion was calculated to be 8% from the $^1$H-NMR spectrum after the termination of reaction. A polycarbonate resin having weight-average molecular weight Mw (PMMA-based) of 50,000 or larger was not obtained under the conditions of Comparative Example A6.

Table 2 shows the physical properties of the polycarbonate resins obtained in Examples, Reference Examples and Comparative Examples.

[Table 2]

| | Example A5 | Example A6 | Example A7 | Example A8 | Comparative Example A5 | Comparative Example A6 |
|---|---|---|---|---|---|---|
| Polymerization temperature °C | 25 | 10 | 5 | 0 | -5 | -10 |
| Rate of conversion,% | 94 | 96 | 97 | 97 | 5 | 8 |
| Mn (PMMA based) | 104000 | 116000 | 121000 | 94000 | - | - |
| Mw (PMMA based) | 223000 | 224000 | 207000 | 163000 | - | - |
| Mn (PS based) | 88000 | 97000 | 102000 | 80000 | - | - |
| Mw (PS based) | 192000 | 192000 | 179000 | 138000 | - | - |
| Mw/Mn (PS based) | 2.2 | 2.0 | 1.8 | 1.7 | - | - |
| Terminal alkoxy group | Benzyl (C7) | Benzyl (C7) | Benzyl (C7) | Benzyl (C7) | - | - |
| mr/ (mm+mr+rr),% | 48 | 47 | 47 | 47 | - | - |
| Rate of decrease in weight at 150°C to 260°C (nitrogen atmosphere), % by mass | 4.2 | 4.4 | 3.1 | 1.6 | - | - |

(continued)

| | Exampl e A5 | Exampl e A6 | Exampl e A7 | Exampl e A8 | Comparative Example A5 | Comparative Example A6 |
|---|---|---|---|---|---|---|
| Photoelastic coefficient ($\times 10^{-12}$/Pa) | - | 2.0 | - | - | - | - |

From Table 2, the polycarbonate resins of Examples A5 to A8 had a markedly small rate of decrease in weight at 150 to 260°C when alcohol-insoluble matter was heated in a nitrogen atmosphere by thermogravimetry, demonstrating excellent heat resistance.

[Cyclic carbonate] (Reference Example)

Hereinafter, reagents manufactured by FUJIFILM Wako Pure Chemical Corp. were used as reagents described in the present Examples, unless otherwise specified.

Analysis approaches described in the present Examples were as follows.

[Identification of compound and calculation of yield]

The identification of a compound and the calculation of a yield were performed, mainly, using $^1$H-NMR.

In the measurement, the equipment used was ECZ400S from JEOL Ltd., and the probe used was a TFH probe. In the measurement, the reference peak of a deuterated solvent was 7.26 ppm in the case of using chloroform-d (hereinafter, also referred to as "$CDCl_3$") and 2.50 ppm in the case of using dimethyl sulfoxide-d6 (hereinafter, also referred to as "DMSO-d6"). The number of scans was 32.

[Calculation of purity]

A purity was calculated by gas chromatography (GC) analysis. In the measurement, the equipment used was a gas chromatograph from Shimadzu Corp. (GC-2010) with column: DB-WAX, carrier gas: helium, injection temperature: 270°C, and detector (temperature): flame ionization detector (FID) (270°C).

[Gas chromatography mass spectrometry]

Gas chromatography mass spectrometry (GC/MS) was carried out by the following procedures.

The analysis equipment used was TRACE-GC/ISQ (Thermo Fisher Scientific Inc.).

Equity-1 (30 m $\times$ 0.25 mm, film thickness: 0.25 $\mu$m) column was used, and analysis conditions involved ionization: EI (70 eV), mass range: m/z = 10 to 1000, flow rate: 1.0 mL/min (constant flow), and oven temperature: 50°C $\rightarrow$ temperature elevation at 10°C/min $\rightarrow$ 300°C (15 min).

For the pretreatment of a sample, 1.0 mg of an inositol derivative was dissolved by the addition of one drop of dimethyl sulfoxide (hereinafter, also referred to as "DMSO"), then 1.0 mL of BSA (N,O-bistrimethylsilylacetamide) was added thereto, and the mixture was heated at 60°C for 60 minutes.

[Glass transition temperature]

A glass transition temperature (Tg) was measured by the following procedures. A polymer dried in vacuum was compression-molded at room temperature, and approximately 10 mg of a test piece was cut out of the sample. Its glass transition temperature (Tg) was measured using a differential scanning calorimetric apparatus manufactured by PerkinElmer Co., Ltd. (DSC8500) under conditions involving a nitrogen gas flow rate of 20 mL/min.

The sample was kept at 40°C for 3 minutes and then warmed from 40°C to 160°C at 20°C/min for primary temperature elevation to completely melt the sample. Then, the sample was cooled from 160°C to 40°C at 50°C/min and kept at 40°C for 5 minutes. Subsequently, the sample was warmed from 40°C to 150°C at 10°C/min for secondary temperature elevation. The point of intersection (midpoint glass transition temperature) between a partial curve of step-like change in a DSC curve drawn by the secondary temperature elevation and a straight line pitched at equal distances in the ordinate direction from extended lines of respective tangents was regarded as a glass transition temperature (Tg).

[Melting point]

A melting point was measured by the following procedures using a differential scanning calorimeter (DSC). The differential scanning calorimeter (DSC) used was Q200 manufactured by TA Instruments. Measurement conditions involved temperature elevation and drop rates of 10°C/min. The melting point was defined as a melting temperature in a process of temperature elevation from 20°C in the measurement.

[Polymer molecular weight]

A polymer molecular weight was measured by the following procedures by use of gel permeation chromatography (GPC).

A solution of 0.02 g of a polycarbonate resin dissolved in 2.0 g of tetrahydrofuran (also referred to as "THF") was used as a measurement sample. A polymer molecular weight was measured using Alliance e2695 manufactured by Waters Corp. as a high-performance chromatograph system. The columns used were TSK guard columns SuperH-H, TSKgel SuperHM-H, TSKgel SuperHM-H, TSKgel SuperH2000, and TSKgel SuperH1000 manufactured by Tosoh Corp. connected in series. The column temperature was 40°C, and tetrahydrofuran was used as a mobile phase. Analysis was conducted at a rate of 0.35 mL/min. The detector used was a differential refractive index detector (RID). A calibration curve was prepared using polymethyl methacrylate standard samples manufactured by Polymer Standards Service GmbH (molecular weight: 2200000, 988000, 608000, 340000, 202000, 88500, 41400, 18700, 9680, 5050, 2380, and 800) as standard samples. The number-average molecular weight (Mn) and the weight-average molecular weight (Mw) of the polycarbonate resin were determined on the basis of this calibration curve.

[Example B1] Synthesis of 1,4-di-O-benzyl-2,3:5,6-dicarbonate-myo-inositol (BCI)

(Step 1-1) Synthesis of 2,3:5,6-di-O-cyclohexylidene-myo-inositol

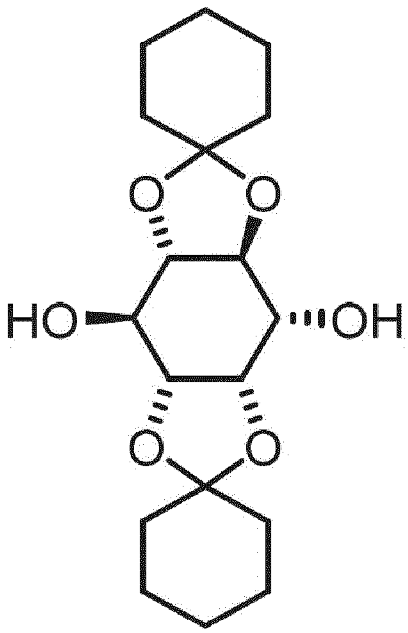

(1 − 1)

In a degassed and argon (Ar)-purged 500 mL four-neck flask, myo-inositol (125 g, 0.69 mol) was dispersed in dehydrated dimethylformamide (hereinafter, also referred to as "DMF"; 860 mL), and 1,1-dimethoxycyclohexane (400 g, 2.77 mol) and p-toluenesulfonic acid monohydrate (13.2 g, 69 mmol, Kishida Chemical Co., Ltd.) were added thereto in order. The obtained reaction solution was heated to 100°C. 45 minutes later, trimethylamine (30 mL) was added to the reaction solution, and the resulting reaction solution was concentrated under reduced pressure. The obtained concentrate was dissolved in ethyl acetate (3.0 L) and washed with an aqueous solution containing 5% by mass of sodium bicarbonate (400 mL). The ethyl acetate phase was dried over an appropriate amount of sodium sulfate, filtered, and then concentrated under reduced pressure to obtain a solid.

The obtained concentrated solid was recrystallized from diisopropyl ether (1.0 L) to obtain 50.5 g (148 mmol) of colorless crystals of 2,3:5,6-di-O-cyclohexylidene-myo-inositol at a yield of 21%. Figure 6 (upper part) shows the $^1$H-NMR chart ($CDCl_3$) of the obtained crystals. From the results of $^1$H-NMR, the obtained crystals were found to be a diol form having a structure of the formula (1-1) (2,3:5,6-di-O-cyclohexylidene-myo-inositol).

(Step 1-2) Synthesis of 1,4-di-O-benzyl-2,3:5,6-di-O-cyclohexylidene-myo-inositol derivative (BCyI)

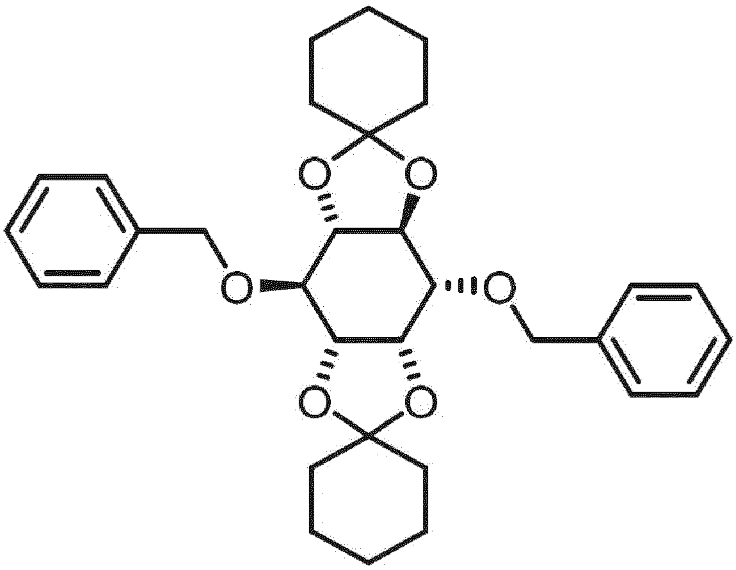

(1 − 2)

A degassed and Ar-purged 1 L four-neck flask was charged with super dehydrated DMF (540 mL), and the diol form (2,3:5,6-di-O-cyclohexylidene-myo-inositol; 50.0 g, 146 mmol) obtained in (Step 1-1) was added thereto and dissolved. Sodium hydride (17.6 g, 0.44 mol) was added to the obtained solution, and the mixture was stirred for 1 hour in an ice bath. Then, benzyl bromide (60.29 g, 352 mmol) dissolved in DMF was reacted therewith to obtain a crude 1,4-di-O-benzyl-2,3:5,6-di-O-cyclohexylidene-myo-inositol derivative. The crude 1,4-di-O-benzyl-2,3:5,6-di-O-cyclohexylidene-myo-inositol derivative was recrystallized from 1 L of ethyl acetate and purified by silica gel chromatography to obtain pure crystals (64.3 g, 123 mmol, yield: 84%) of the 1,4-di-O-benzyl-2,3:5,6-di-O-cyclohexylidene-myo-inositol derivative (hereinafter, also referred to as "BCyI").

Figure 6 (lower part) shows the $^1$H-NMR chart ($CDCl_3$) of the obtained pure crystals. From the results of $^1$H-NMR, the obtained pure crystals were found to be BCyI having a structure of the formula (1-2).

(Step 1-3) Synthesis of 1,4-di-O-benzyl-myo-inositol (BI)

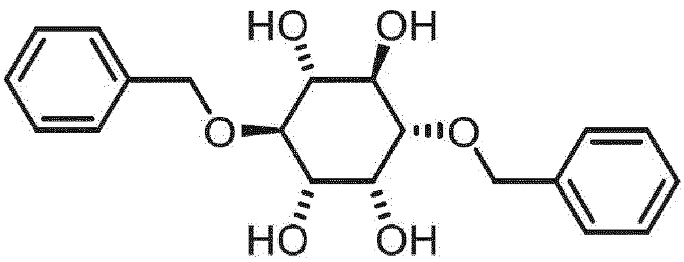

(1 − 3)

In an Ar atmosphere, methanol (1.23 L) was added to a 3 L four-neck flask, further the 1,4-di-O-benzyl-2,3:5,6-di-O-cyclohexylidene-myo-inositol derivative (BCyI; 64.3 g, 123 mmol) obtained in (Step 1-2) was added thereto, and the mixture was stirred to obtain a solution. Concentrated hydrochloric acid (12.3 mL) was gradually added to the obtained solution for hydrolysis. The consumption of the starting material was confirmed by thin-layer chromatography (TLC). The

obtained solution was concentrated under reduced pressure, and the residue was recrystallized from methanol (700 mL) to obtain colorless crystals of 1,4-di-O-benzyl-myo-inositol (hereinafter, also referred to as "BI"; 40.24 g, 111 mmol, yield: 90%). Figure 7 shows the $^1$H-NMR chart (DMSO-d6) of the obtained crystals. From the results of $^1$H-NMR, the obtained crystals were found to be BI having a structure of the formula (1-3).

(Step 1-4) Synthesis of 1,4-di-O-benzyl-2,3:5,6-dicarbonate-myo-inositol (BCI)

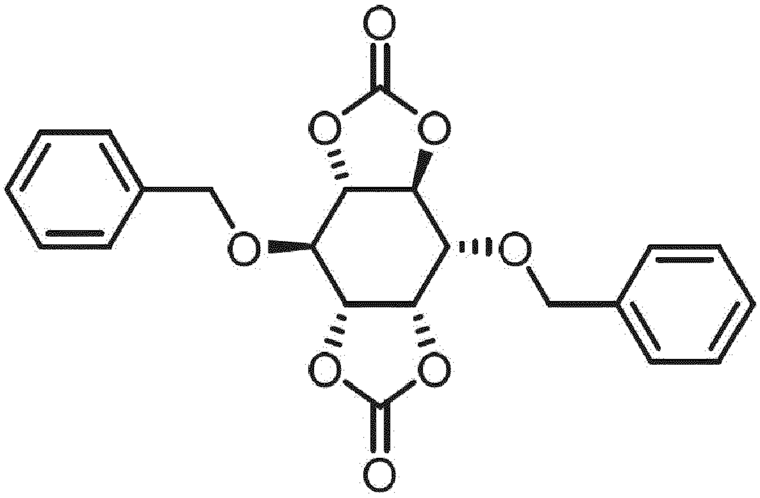

(1 − 4)

In an Ar atmosphere, dehydrated N,N-dimethylacetamide (dehydrated DMA, 350 mL) was added to a 1 L four-neck flask, and 1,4-di-O-benzyl-myo-inositol (BI; 32.65 g, 90.6 mol) obtained in (Step 1-3) and triethylamine (48.56 g, 480 mmol) were added thereto. The obtained solution was cooled to -4°C. Ethyl chloroformate (49.1 g, 45.2 mmol) diluted with N,N-dimethylacetamide (DMA; 100 mL) was added dropwise in small portions to the solution thus cooled, and the mixture was stirred overnight for reaction. A salt formed in the reaction solution was removed by filtration, and the obtained filtrate was washed with THF (300 mL) and then concentrated under reduced pressure. The obtained residue was neutralized by the addition of 1% by mass of hydrochloric acid until becoming acidic. Then, the obtained solution was subjected to extraction with ethyl acetate (1.0 L). The organic phase was subjected to water removal over magnesium sulfate and then concentrated under reduced pressure. The obtained concentrate was purified by silica gel chromatography to obtain 26.66 g of colorless clear crystals of 1,4-di-O-benzyl-2,3:5,6-dicarbonate-myo-inositol (hereinafter, also referred to as "BCI") (yield: 71%). Figure 8 shows the $^1$H-NMR chart ($CDCl_3$) of the obtained crystals. From the results of $^1$H-NMR, the obtained crystals were found to be BCI having a structure of the formula (1-4). The melting point of the obtained BCI was 195°C.

[Example B2] Synthesis of 1,4-di-O-octyl-2,3:5,6-dicarbonate-myo-inositol (OCI)

(Step 2-1) Synthesis of 2,3:5,6-di-O-cyclohexylidene-myo-inositol

A diol form (2,3:5,6-di-O-cyclohexylidene-myo-inositol) was obtained by synthesis in the same manner as in step 1-1 of Example B1.

(Step 2-2) Synthesis of 1,4-di-O-octyl-2,3:5,6-di-O-cyclohexylidene-myo-inositol derivative (OCyI)

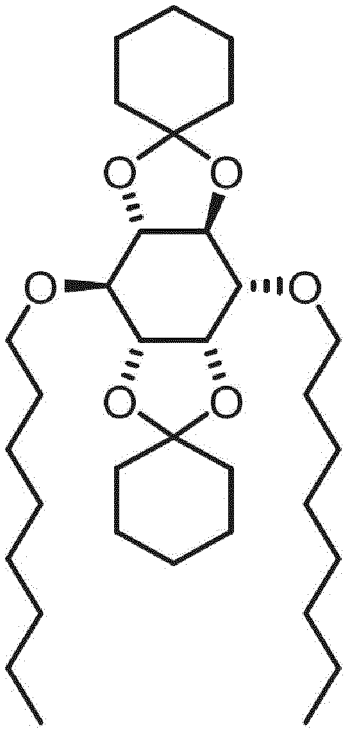

(2-2)

55% sodium hydride (8.66 g, 198 mmol, Kanto Chemical Co., Inc.) was added to a 200 mL two-neck flask and washed with hexane. The flask was degassed and purged with $N_2$, and a solution of the diol form (2,3:5,6-di-O-cyclohexylidene-myo-inositol) (15.0 g, 44.1 mmol) obtained in (Step 2-1) dissolved in dimethylformamide (DMF, 171 mL, Kanto Chemical Co., Inc.) was then added to the flask. The solution in the flask was stirred at room temperature for 1 hour and then reacted with octyl bromide (17.4 g, 15.9 mmol, Tokyo Chemical Industry Co., Ltd.) in an ice bath. The reaction solution was further stirred at room temperature for 18 hours, and 200 mL of water was then added thereto to obtain a precipitate. The precipitated 1,4-di-O-benzyl-2,3:5,6-di-O-cyclohexylidene-myo-inositol derivative was collected by filtration and washed with water. The residue was dried in vacuum to obtain a colorless liquid of the 1,4-di-O-octyl-2,3:5,6-di-O-cyclohexylidene-myo-inositol derivative (hereinafter, also referred to as "OCyl"; 23.7 g, 42.0 mmol, yield: 95%).

(Step 2-3) Synthesis of 1,4-di-O-octyl-myo-inositol (OI)

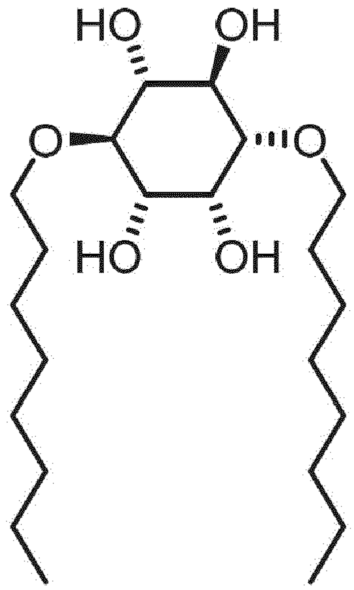

(2－3)

Methanol (18 mL, Daishin Chemical Co., Ltd.) was added to a 500 mL recovery flask, then OCyI (1,4-di-O-octyl-2,3:5,6-di-O-cyclohexylidene-myo-inositol derivative; 1.00 g, 1.77 mmol) obtained in (Step 2-2) was added thereto, and the mixture was stirred to obtain a solution. Concentrated hydrochloric acid (1.8 mL) was gradually added to the obtained solution, and the mixture was stirred at room temperature for 17 hours. The consumption of the starting material was confirmed by thin-layer chromatography (TLC). The obtained reaction solution was concentrated under reduced pressure, and the residue was recrystallized from a mixed solvent of ethyl acetate and hexane to obtain white crystals of 1,4-di-O-octyl-myo-inositol (hereinafter, also referred to as "OI"; 0.50 g, 1.24 mmol, yield: 70%).

(Step 2-4) Synthesis of 1,4-di-O-octyl-2,3:5,6-dicarbonate-myo-inositol (OCI)

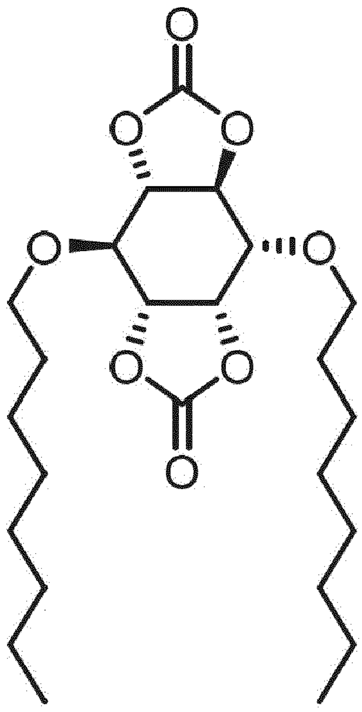

(2－4)

1,4-di-O-Octyl-myo-inositol (1.50 g, 3.71 mmol) obtained in (Step 2-3), triethylamine (10.4 mL, 74.6 mmol, Kanto Chemical Co., Inc.) and dehydrated THF (15.0 mL, Kanto Chemical Co., Inc.) were added to a 200 mL recovery flask, and

the obtained solution was cooled in an ice bath. Ethyl chloroformate (20.4 mL, 214 mmol, Kishida Chemical Co., Ltd.) diluted with THF (21.0 mL) was added dropwise in small portions to the solution thus cooled, and the mixture was stirred at room temperature for 30 minutes for reaction. Then, ethyl chloroformate (10.2 mL, 107 mmol) was added again to the reaction solution, and the mixture was stirred for 1 hour for reaction. A salt formed in the reaction solution was removed by filtration. The obtained filtrate was neutralized with 1% by mass of hydrochloric acid until becoming acidic. Then, THF was distilled off. The obtained residue was dissolved in ethyl acetate (200 mL) and washed with water. The organic phase was dried over anhydrous magnesium sulfate and then concentrated under reduced pressure. The obtained concentrate was recrystallized from hexane to obtain white crystals of 1,4-di-O-octyl-2,3:5,6-dicarbonate-myo-inositol (hereinafter, also referred to as "OCI"; 0.75 g, 1.64 mmol, yield: 44%).

Figure 9 shows the $^1$H-NMR chart ($CDCl_3$) of the obtained crystals. From the results of $^1$H-NMR, the obtained crystals were found to be OCI having a structure of the formula (2-4).

The melting point of the obtained OCI was 120°C.

[Example B3] Synthesis of 1,4-bis-O-(2-ethylhexyl)-2,3:5,6-dicarbonate-myo-inositol (ECI)

(Step 3-1) Synthesis of 2,3:5,6-di-O-cyclohexylidene-myo-inositol

A diol form (2,3:5,6-di-O-cyclohexylidene-myo-inositol) was obtained by synthesis in the same manner as in step 1-1 of Example B1.

(Step 3-2) Synthesis of 1,4-bis-O-(2-ethylhexyl)-2,3:5,6-di-O-cyclohexylidene-myo-inositol derivative (ECyI)

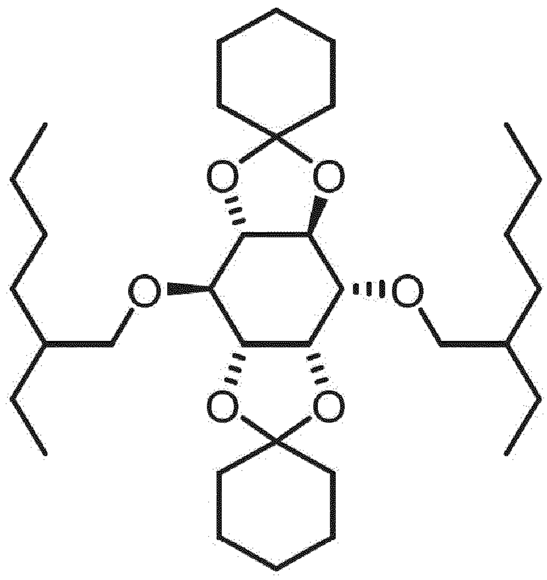

(3 − 2)

55% sodium hydride (5.80 g, 0.120 mol) was added to a 200 mL two-neck flask and washed with hexane. The flask was degassed and purged with $N_2$. Then, a solution of the diol form (2,3:5,6-di-O-cyclohexylidene-myo-inositol) (2.01 g, 5.91 mmol) obtained in (Step 3-1) dissolved in DMF (28 mL) was added thereto, and the mixture was stirred at 50°C for 5 hours. Then, the obtained solution was cooled to room temperature, and 1-bromo-2-ethylhexane (4.20 g, 21.9 mmol, Tokyo Chemical Industry Co., Ltd.) was added thereto, followed by reaction for 38 hours. Then, the obtained reaction solution was washed with 200 mL of water and subjected to extraction with hexane. The hexane phase was concentrated under reduced pressure. The concentrate thus obtained by concentration under reduced pressure was purified by silica gel column chromatography (eluent: ethyl acetate/hexane = 1/2) to separate a 1,4-bis-O-(2-ethylhexyl)-2,3:5,6-di-O-cyclohexylidene-myo-inositol derivative (hereinafter, also referred to as "ECyI"), which was then dried in vacuum to obtain white crystals (0.620 g, 1.01 mmol, yield: 18%).

(Step 3-3) Synthesis of 1,4-bis-O-(2-ethylhexyl)-myo-inositol (EI)

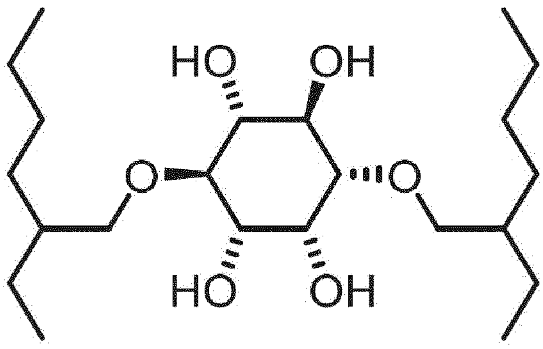

(3 − 3)

Methanol (170 mL) was added to a 200 mL recovery flask, then the 1,4-bis-O-(2-ethylhexyl)-2,3:5,6-di-O-cyclohexylidene-myo-inositol derivative (ECyl; 0.960 g, 1.70 mmol) obtained in (Step 3-2) was added thereto, and the mixture was stirred to obtain a solution. Concentrated hydrochloric acid (7 mL) was gradually added to the obtained solution, and the mixture was stirred at room temperature for 18 hours. The consumption of the starting material was confirmed by thin-layer chromatography (TLC). The obtained reaction solution was concentrated under reduced pressure, and the residue was recrystallized from a mixed solvent of ethyl acetate and hexane to obtain white crystals of 1,4-di-O-(2-ethylhexyl)-myo-inositol (hereinafter, also referred to as "EI"; 0.230 g, 0.569 mmol, yield: 34%).

(Step 3-4) Synthesis of 1,4-bis-O-(2-ethylhexyl)-2,3:5,6-dicarbonate-myo-inositol (ECI)

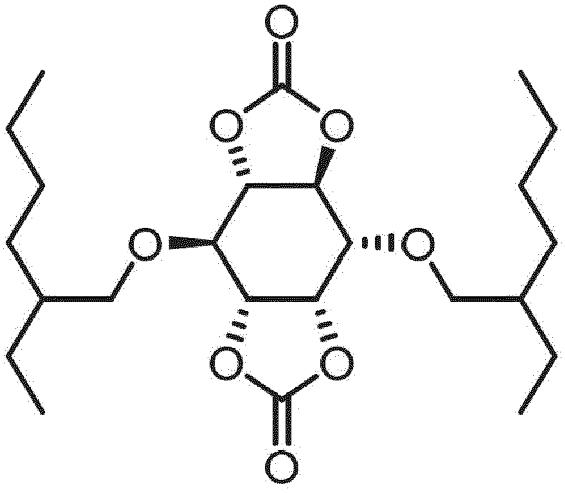

(3 − 4)

1,4-bis-O-(2-Ethylhexyl)-myo-inositol (EI; 1.37 g, 3.39 mmol) obtained in (Step 3-3), triethylamine (8.5 mL, 61.0 mmol) and dehydrated THF (15.0 mL) were added to a 200 mL recovery flask, and the obtained solution was cooled in an ice bath. Ethyl chloroformate (2.20 mL, 23.1 mmol) diluted with THF (21.0 mL) was added dropwise in small portions to the solution thus cooled, and the mixture was stirred at room temperature for 30 minutes for reaction. Then, ethyl chloroformate (2.20 mL, 23.1 mmol) was added again to the reaction solution, and the mixture was stirred for 1 hour for reaction. A salt formed in the reaction solution was removed by filtration. The obtained filtrate was neutralized with 1% by mass of hydrochloric acid until becoming acidic. Then, THF was distilled off. The obtained residue was dissolved in ethyl acetate (200 mL) and washed with water. The organic phase was dried over magnesium sulfate and then concentrated under reduced pressure. The obtained residue was purified by silica gel column chromatography (eluent: ethyl acetate/hexane = 5/1) and then recrystallized from hexane to obtain white crystals of 1,4-bis-O-(2-ethylhexyl)-2,3:5,6-dicarbonate-myo-inositol (hereinafter, also referred to as "ECI"; 0.51 g, 1.10 mmol, 32%).

The melting point of the obtained ECI was 100°C.

[Example B4] Synthesis of carbonate-myo-inositol composition

myo-Inositol (3.60 g, 0.4 M) was added to DMSO (50 mL) and dissolved at 60°C. Then, diphenyl carbonate (8.57

g) and LiOH (0.0024 g, 0.5 mol/%) were added to the solution at room temperature and reacted at room temperature for 70 hours. The amount of phenol formed was calculated as to the mixed reaction solution by $^1$H-NMR to confirm that the reaction proceeded by 90%.

The mixed reaction solution was passed through an ion-exchange resin (inertSep(R) SCX). The solution (82.63 g) thus passed was concentrated in an evaporator to obtain a concentrate (26.99 g). Acetic acid (141.58 g) was added to the obtained concentrate (7.12 g), and the resulting precipitate was filtered by suction and washed to obtain a filtrate (173.88 g). The obtained filtrate was concentrated in an evaporator to obtain a concentrate (8.21 g). A precipitate was formed by the addition of chloroform (226.86 g) to the obtained concentrate (7.47 g). The resulting precipitate was filtered by suction, and the residue was dried to obtain 0.698 g of a carbonate-myo-inositol composition.

The obtained carbonate-myo-inositol composition was trimethylsilylated (TMSylated) and measured by GC/MS to identify two types of compounds given below in the composition. Figure 10 shows the GC/MS measurement results.

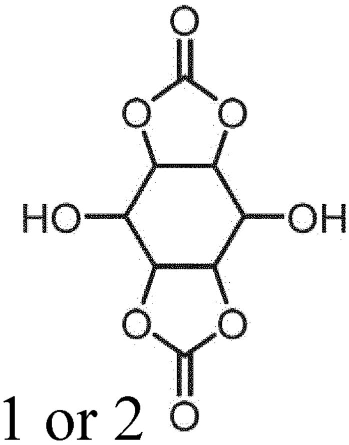

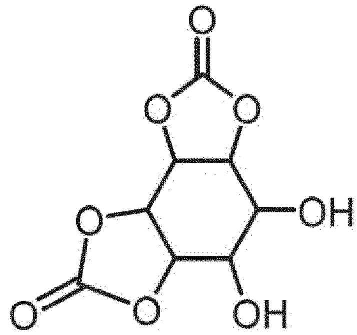

Compound 1 or 2

[Synthesis Example 1] Synthesis of trans-1,2-cyclohexene carbonate (hereinafter, also referred to as "T6C")

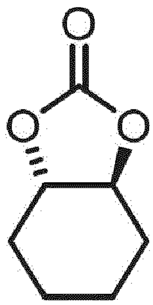

(A-1)

trans-1,2-Cyclohexanediol (200.0 g, 1.7 mol) and dehydrated 1,4-dioxane (2.0 L) were added to a degassed and argon-purged 5 L four-neck flask to obtain a solution. The obtained solution was cooled to 8.0°C. Ethyl chloroformate (280.2 g, 2.6 mol) was slowly added dropwise to the solution thus cooled, further triethylamine (384.4 g, 8.6 mol) diluted with dehydrated toluene (2.5 L) was slowly added dropwise thereto, and the mixture was stirred for 12 hours for reaction. A salt formed in the reaction solution was removed by filtration, and the obtained filtrate was concentrated under reduced pressure. The obtained residue was dissolved by the addition of ethyl acetate (2.0 L), and the obtained solution was washed with 1% by mass of hydrochloric acid (2.0 L). The organic phase was subjected to water removal over magnesium sulfate and then concentrated under reduced pressure. The obtained concentrate was purified by silica gel chromatography to obtain 400.2 g of colorless clear crystals (yield: 71%). Figure 11 shows the $^1$H-NMR chart ($CDCl_3$) of the obtained crystals. From the results of $^1$H-NMR, the obtained crystals were found to be T6C having a structure of the formula (A-1).

[Synthesis Example 2] Synthesis of BCI/T6C copolymer, stirring scheme: magnetic

A 50 mL three-neck flask was charged with BCI (0.560 g, 1.36 mmol) obtained in Example B1 and T6C (1.65 g, 11.6 mmol) obtained in Synthesis Example 1. The flask was degassed and purged with nitrogen, and then heated to an

internal temperature of 60°C so that the contents were melted to obtain a solution. Then, while the obtained solution was stirred using a magnetic stirrer and a rotor, a solution of potassium tert-butoxide in THF (65 μL, 1.0 M, 0.0065 mmol) was slowly added thereto. The mixture was stirred at 60°C for 6 hours for polymerization reaction. The flask was allowed to cool to an internal temperature of room temperature. Then, the reaction solution was dissolved by the addition of 15 mL of chloroform, and the prepared polymer solution was added into 150 mL of methanol to deposit a polymer. The deposited polymer was recovered by filtration under reduced pressure and washed with methanol. The obtained polymer was dried in vacuum at 60°C for 4 hours to obtain 0.571 g of a BCI/T6C copolymer (yield: 26%).

The proportion of each monomer component ($M_1$ and $M_2$ components in Table 3) in the copolymer was calculated by $^1$H-NMR measurement. Figure 12 shows the $^1$H-NMR measurement results.

In the $^1$H-NMR spectrum of a sample dissolved in dimethyl sulfoxide-d6, a signal assigned to 8H of methylene hydrogen on T6C was observed at 0.9 to 2.2 ppm. A signal assigned to 10H of hydrogen of phenyl groups on BCI was observed at 7.1 to 7.4 ppm. A proportion (molar ratio) derived from BCI in the copolymer was calculated to be 26% from the integrated intensity ratios of these signals. The number-average molecular weight (Mn), the weight-average molecular weight (Mw), the molecular weight distribution (PDI) and the glass transition temperature (Tg) of the obtained copolymer were also determined. The results are shown in Table 3.

[Synthesis Example 3] Synthesis of OCI/T6C copolymer, stirring scheme: magnetic

0.469 g of an OCI/T6C copolymer was obtained (yield: 22%) through polymerization reaction in the same manner as in Synthesis Example 2 except that OCI (0.560 g, 1.23 mmol) obtained in Example B2 was added as a comonomer instead of BCI to T6C (1.56 g, 11.0 mol).

The proportion of each monomer component ($M_1$ and $M_2$ components in Table 3) in the copolymer was calculated by $^1$H-NMR measurement. Figure 13 shows the $^1$H-NMR measurement results.

Specifically, in the $^1$H-NMR spectrum of a sample dissolved in chloroform-d, a signal assigned to 2H of methylene hydrogen on T6C was observed at 1.9 to 2.3 ppm. A signal assigned to 6H of hydrogen of methyl groups on OCI was observed at 0.8 to 0.9 ppm. A proportion (molar ratio) derived from OCI in the copolymer was calculated to be 21% from the integrated intensity ratios of these signals. The number-average molecular weight (Mn), the weight-average molecular weight (Mw), the molecular weight distribution (PDI) and the glass transition temperature (Tg) of the obtained copolymer were also determined. The results are shown in Table 3.

[Synthesis Example 4] Synthesis of BCI/T6C copolymer, stirring scheme: mechanical

0.951 g of a BCI/T6C copolymer was obtained (yield: 43%) through polymerization reaction in the same manner as in Synthesis Example 2 except that: the amount of BCI added was set to "0.566 g, 1.37 mmol"; the amount of T6C added was set to "1.67 g, 11.7 mmol"; and stirring was performed using a mechanical stirrer and a stirring blade instead of the magnetic stirrer and the rotor.

The proportion of each monomer component ($M_1$ and $M_2$ components in Table 3) in the copolymer was calculated by $^1$H-NMR measurement. Figure 14 shows the $^1$H-NMR measurement results.

A proportion (molar ratio) derived from BCI in the copolymer was calculated to be 17% from integrated intensity ratios in the $^1$H-NMR spectrum. The number-average molecular weight (Mn), the weight-average molecular weight (Mw), the molecular weight distribution (PDI) and the glass transition temperature (Tg) of the obtained copolymer were also determined. The results are shown in Table 3.

[Synthesis Example 5] Synthesis of BCI/T6C copolymer, stirring scheme: mechanical

1.19 g of a BCI/T6C copolymer was obtained (yield: 45%) through polymerization reaction in the same manner as in Synthesis Example 4 except that: the amount of BCI added was set to "0.218 g, 0.529 mmol"; and the amount of T6C added was set to "2.43 g, 17.1 mmol".

The proportion of each monomer component ($M_1$ and $M_2$ components in Table 3) in the copolymer was calculated by $^1$H-NMR measurement. Figure 15 shows the $^1$H-NMR measurement results.

A proportion (molar ratio) derived from BCI in the copolymer was calculated to be 4% from integrated intensity ratios in the $^1$H-NMR spectrum. The number-average molecular weight (Mn), the weight-average molecular weight (Mw), the molecular weight distribution (PDI) and the glass transition temperature (Tg) of the obtained copolymer were also determined. The results are shown in Table 3.

[Synthesis Example 6] Synthesis of OCI/T6C copolymer, stirring scheme: mechanical

1.04 g of an OCI/T6C copolymer was obtained (yield: 43%) through polymerization reaction in the same manner

as in Synthesis Example 3 except that: the amount of OCI added was set to "0.217 g, 0.475 mmol"; and the amount of T6C added was set to "2.19 g, 15.4 mmol".

The proportion of each monomer component ($M_1$ and $M_2$ components in Table 3) in the copolymer was calculated by $^1$H-NMR measurement. Figure 16 shows the $^1$H-NMR measurement results.

A proportion (molar ratio) derived from OCI in the copolymer was calculated to be 4% from integrated intensity ratios in the $^1$H-NMR spectrum. The number-average molecular weight (Mn), the weight-average molecular weight (Mw), the molecular weight distribution (PDI) and the glass transition temperature (Tg) of the obtained copolymer were also determined. The results are shown in Table 3.

From the results of Synthesis Examples described above, it was confirmed by NMR analysis that: among dicarbonates of myo-inositol, only the trans conformation was polymerized while the cis conformation was maintained; and the polymer had a structure represented by the following formula (B-1):

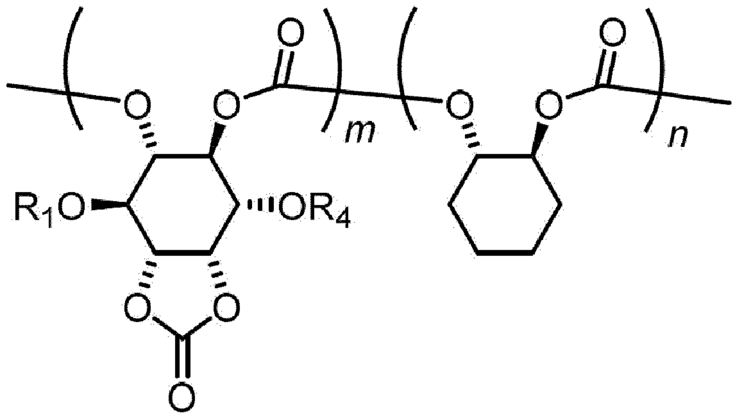

(B−1)

wherein $R_{11}$ and $R_{14}$ each represent benzyl or octyl.

[Table 3]

| Synthesis Example | $M_1$ | $M_2$ | Stirring scheme | $F_1$[a] | $f_1$[b] | Yield, %[c] | Mn × $10^{-3}$ | Mw × $10^{-3}$ | PDI | Tg, °C |
|---|---|---|---|---|---|---|---|---|---|---|
| Synthesis Example 2 | BCI | T6C | Magnetic | 0.10 | 0.26 | 26 | 7.4 | 14 | 1.9 | 117 |
| Synthesis Example 3 | OCI | T6C | Magnetic | 0.10 | 0.21 | 22 | 16 | 24 | 1.5 | 70 |
| Synthesis Example 4 | BCI | T6C | Mechanical | 0.10 | 0.17 | 43 | 8.3 | 15 | 1.8 | 118 |
| Synthesis Example 5 | BCI | T6C | Mechanical | 0.03 | 0.04 | 45 | 18 | 34 | 19 | 108 |
| Synthesis Example 6 | OCI | T6C | Mechanical | 0.03 | 0.04 | 43 | 39 | 82 | 2.1 | 108 |

a) $F_1$: Molar ratio of the $M_1$ component in an added monomer ratio
b) $f_1$: Molar ratio of the $M_1$ component contained in the polymer after purification by methanol reprecipitation (calculated by $^1$H-NMR)
c) Yield: Weight (g) of methanol-insoluble matter obtained by purification by methanol reprecipitation / Weight (g) of the added monomer × 100

The present application is based on Japanese Patent Application No. 2020-005443 filed on January 16, 2020 and Japanese Patent Application No. 2020-015754 filed on January 31, 2020.

Industrial Applicability

The polycarbonate resin, the polycarbonate resin composition, and an optical molded article comprising the same according to the present invention have industrial applicability in the fields of, for example, various optical materials such as optical lens materials, optical devices, materials for optical components, and display materials. The cyclic carbonate of the disclosure which is not claimed, exploits a renewable resource not only at a carbonate site but in a skeleton and can provide a material with low environmental load. The cyclic carbonate of the disclosure which is not claimed, can be used as an electrolyte or an additive for resins, etc. and can be used as a monomer to synthesize polycarbonate.

## Claims

1. A polycarbonate resin comprising a structural unit represented by the following formula (1):

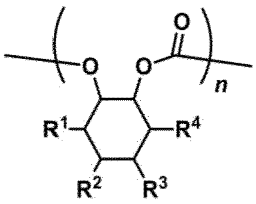

(1)

wherein $R^1$, $R^2$, $R^3$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain, wherein
weight-average molecular weight Mw measured by size-exclusion chromatography using polymethyl methacrylate as a standard sample is 50,000 or larger and 500,000 or smaller.

2. The polycarbonate resin according to claim 1, wherein glass transition temperature Tg measured with a differential scanning calorimeter is 80°C or higher and 180°C or lower.

3. The polycarbonate resin according to claim 1 or 2, wherein the polycarbonate resin comprises the structural unit represented by the formula (1) in the main chain.

4. The polycarbonate resin according to any one of claims 1 to 3, wherein a ratio of mr / (mm + mr + rr) measured by $^{13}C$-NMR is 40% or more, wherein mm represents an isotactic moiety in a periodic meso structure of the monomer, rr represents a syndiotactic moiety in a periodic structure based on a racemo bond, and mr represents a moiety in which the meso moiety and the racemo moiety are bonded.

5. The polycarbonate resin according to any one of claims 1 to 4, wherein the polycarbonate resin comprises a terminal alkoxy group.

6. The polycarbonate resin according to claim 5,
wherein the number of carbon atoms in the alkoxy group is 1 to 10.

7. The polycarbonate resin according to any one of claims 1 to 6, wherein a polymer component having a polystyrene-based molecular weight of 1,000 or larger measured by size-exclusion chromatography has a weight-average molecular weight (Mw) of 100,000 or larger and 500,000 or smaller and a ratio (Mw/Mn) of the weight-average molecular weight (Mw) to a number-average molecular weight (Mn) of 2.5 or less.

8. The polycarbonate resin according to claim 7, wherein a rate of decrease in weight at 150 to 260°C is 30% by mass or less when alcohol-insoluble matter is heated in a nitrogen atmosphere by thermogravimetry, measured as indicated in the specification.

9. The polycarbonate resin according to any one of claims 1 to 8, wherein an absolute value of a photoelastic coefficient is $10 \times 10^{-12}$ $Pa^{-1}$ or smaller, measured as indicated in the specification.

10. The polycarbonate resin according to any one of claims 1 to 9, wherein an absolute value of an in-plane phase difference in a 100% uniaxially drawn film of the polycarbonate resin is 100 nm or smaller in terms of a thickness of 100 μm, measured as indicated in the specification.

11. The polycarbonate resin according to any one of claims 1 to 10, wherein the polycarbonate resin comprises a structural unit represented by the formula (1) wherein each of $R^1$, $R^2$, $R^3$ and $R^4$ is a hydrogen atom.

**12.** The polycarbonate resin according to any one of claims 1 to 11, wherein the polycarbonate resin comprises a structural unit represented by the following formula (1'):

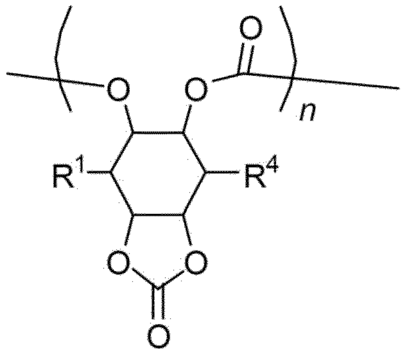

(1')

wherein $R^1$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain.

**13.** A polycarbonate resin composition comprising
the polycarbonate resin according to any one of claims 1 to 12 and an antioxidant.

**14.** An optical component comprising the polycarbonate resin according to any one of claims 1 to 12 or the polycarbonate resin composition according to claim 13.

**15.** Use of the polycarbonate resin according to any one of claims 1 to 12 or the polycarbonate resin composition according to claim 13 as a material for an optical component.

**16.** A method for producing the polycarbonate resin according to any one of claims 1 to 12, comprising
a polymerization step of ring-opening polymerizing a cyclic carbonate represented by the following formula (3) to obtain the polycarbonate resin:

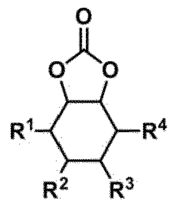

(3)

wherein $R^1$, $R^2$, $R^3$ and $R^4$ are each independently a hydrogen atom, a hydroxy group, a phosphoric acid group, an amino group, a vinyl group, an allyl group, an alkoxy group having 1 to 10 carbon atoms, an ester group having 1 to 11 carbon atoms, an acyl group having 1 to 11 carbon atoms, or an unsubstituted linear, branched, or cyclic alkyl group having 1 to 10 carbon atoms, and are optionally bonded to each other via an alkylene group or a carbonate group to form a cyclic structure, wherein the alkylene group is optionally substituted by a hydroxy group, a phosphoric acid group, an amino group, an alkoxy group, or an ester group, and optionally has a carbonyl group inserted in the main chain.

## Patentansprüche

**1.** Polycarbonatharz, umfassend eine durch die folgende Formel (1) wiedergegebene Struktureinheit:

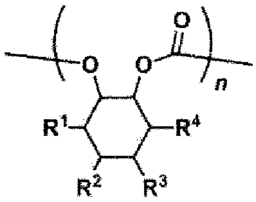

(1)

wobei $R^1$, $R^2$, $R^3$ und $R^4$ jeweils unabhängig für ein Wasserstoffatom, eine Hydroxygruppe, eine Phosphorsäuregruppe, eine Aminogruppe, eine Vinylgruppe, eine Allylgruppe, eine Alkoxygruppe mit 1 bis 10 Kohlenstoffatomen, eine Estergruppe mit 1 bis 11 Kohlenstoffatomen, eine Acylgruppe mit 1 bis 11 Kohlenstoffatomen oder eine unsubstituierte lineare, verzweigte oder cyclische Alkylgruppe mit 1 bis 10 Kohlenstoffatomen stehen und gegebenenfalls über eine Alkylengruppe oder eine Carbonatgruppe unter Bildung einer cyclischen Struktur aneinander gebunden sind, wobei die Alkylengruppe gegebenenfalls durch eine Hydroxygruppe, eine Phosphorsäuregruppe, eine Aminogruppe, eine Alkoxygruppe oder eine Estergruppe substituiert ist und gegebenenfalls eine in die Hauptkette eingefügte Carbonylgruppe aufweist, wobei das gewichtsmittlere Molekulargewicht Mw, gemessen durch Größenausschlusschromatographie unter Verwendung von Polymethylmethacrylat als Standardprobe, 50.000 oder mehr und 500.000 oder weniger beträgt.

2. Polycarbonatharz nach Anspruch 1, wobei die Glasübergangstemperatur Tg, die mit einem Differential-Scanning-Kalorimeter gemessen wird, 80 °C oder höher und 180 °C oder niedriger ist.

3. Polycarbonatharz nach Anspruch 1 oder 2, wobei das Polycarbonatharz die durch die Formel (1) wiedergegebene Struktureinheit in der Hauptkette umfasst.

4. Polycarbonatharz nach einem der Ansprüche 1 bis 3, wobei ein Verhältnis von mr / (mm + mr + rr), gemessen mit $^{13}C$-NMR, 40 % oder mehr beträgt, wobei mm eine isotaktische Gruppierung in einer periodischen *meso*-Struktur des Monomers bedeutet, rr eine syndiotaktische Gruppierung in einer periodischen Struktur auf Basis einer *racemo*-Bindung bedeutet und mr eine Gruppierung bedeutet, in der die *meso*-Gruppierung und die *racemo*-Gruppierung gebunden sind.

5. Polycarbonatharz nach einem der Ansprüche 1 bis 4, wobei das Polycarbonatharz eine endständige Alkoxygruppe umfasst.

6. Polycarbonatharz nach Anspruch 5, wobei die Anzahl der Kohlenstoffatome in der Alkoxygruppe 1 bis 10 beträgt.

7. Polycarbonatharz nach einem der Ansprüche 1 bis 6, wobei eine Polymerkomponente mit einem polystyrolbezogenen Molekulargewicht von 1.000 oder größer, gemessen mit Größenausschlusschromatographie, ein gewichtsmittleres Molekulargewicht (Mw) von 100.000 oder größer und 500.000 oder kleiner und ein Verhältnis (Mw/Mn) des gewichtsmittleren Molekulargewichts (Mw) zu einem zahlenmittleren Molekulargewicht (Mn) von 2,5 oder kleiner aufweist.

8. Polycarbonatharz nach Anspruch 7, wobei eine Gewichtsabnahmerate bei 150 bis 260°C 30 Masse-% oder weniger beträgt, wenn alkoholunlösliche Stoffe in einer Stickstoffatmosphäre thermogravimetrisch erhitzt werden, gemessen wie in der Beschreibung angegeben.

9. Polycarbonatharz nach einem der Ansprüche 1 bis 8, wobei ein Absolutwert eines photoelastischen Koeffizienten 10 x $10^{-12}$ $Pa^{-1}$ oder kleiner ist, gemessen wie in der Beschreibung angegeben.

10. Polycarbonatharz nach einem der Ansprüche 1 bis 9, wobei ein Absolutwert einer In-Plane-Phasendifferenz in der Ebene in einer 100 % uniaxial verstreckten Folie des Polycarbonatharzes 100 nm oder weniger in Bezug auf eine Dicke von 100 $\mu$m beträgt, gemessen wie in der Beschreibung angegeben.

11. Polycarbonatharz nach einem der Ansprüche 1 bis 10, wobei das Polycarbonatharz eine Struktureinheit umfasst, die durch die Formel (1) wiedergegeben wird, wobei $R^1$, $R^2$, $R^3$ und $R^4$ jeweils für ein Wasserstoffatom stehen.

12. Polycarbonatharz nach einem der Ansprüche 1 bis 11, wobei das Polycarbonatharz eine durch die folgende Formel (1') wiedergegebene Struktureinheit umfasst:

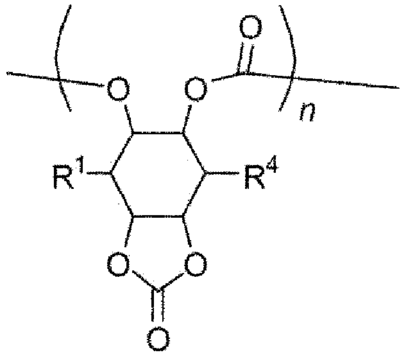

(1 ')

wobei $R^1$ und $R^4$ jeweils unabhängig für ein Wasserstoffatom, eine Hydroxygruppe, eine Phosphorsäuregruppe, eine Aminogruppe, eine Vinylgruppe, eine Allylgruppe, eine Alkoxygruppe mit 1 bis 10 Kohlenstoffatomen, eine Estergruppe mit 1 bis 11 Kohlenstoffatomen, eine Acylgruppe mit 1 bis 11 Kohlenstoffatomen oder eine unsubstituierte lineare, verzweigte oder cyclische Alkylgruppe mit 1 bis 10 Kohlenstoffatomen stehen und gegebenenfalls über eine Alkylengruppe oder eine Carbonatgruppe unter Bildung einer cyclischen Struktur aneinander gebunden sind, wobei die Alkylengruppe gegebenenfalls durch eine Hydroxygruppe, eine Phosphorsäuregruppe, eine Aminogruppe, eine Alkoxygruppe oder eine Estergruppe substituiert ist und gegebenenfalls eine in die Hauptkette eingefügte Carbonylgruppe aufweist.

**13.** Polycarbonatharz-Zusammensetzung, umfassend das Polycarbonatharz nach einem der Ansprüche 1 bis 12 und ein Antioxidans.

**14.** Optische Komponente, umfassend das Polycarbonatharz nach einem der Ansprüche 1 bis 12 oder die Polycarbonatharz-Zusammensetzung nach Anspruch 13.

**15.** Verwendung des Polycarbonatharzes nach einem der Ansprüche 1 bis 12 oder der Polycarbonatharz-Zusammensetzung nach Anspruch 13 als Material für eine optische Komponente.

**16.** Verfahren zur Herstellung des Polycarbonatharzes nach einem der Ansprüche 1 bis 12, umfassend

einen Polymerisationsschritt, bei dem ein durch die folgende Formel (3) wiedergegebenes cyclisches Carbonat unter Ringöffnung polymerisiert wird, so dass das Polycarbonatharz erhalten wird:

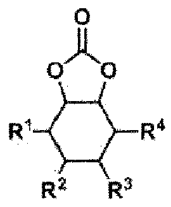

(3)

wobei $R^1$, $R^2$, $R^3$ und $R^4$ jeweils unabhängig für ein Wasserstoffatom, eine Hydroxygruppe, eine Phosphorsäuregruppe, eine Aminogruppe, eine Vinylgruppe, eine Allylgruppe, eine Alkoxygruppe mit 1 bis 10 Kohlenstoffatomen, eine Estergruppe mit 1 bis 11 Kohlenstoffatomen, eine Acylgruppe mit 1 bis 11 Kohlenstoffatomen oder eine unsubstituierte lineare, verzweigte oder cyclische Alkylgruppe mit 1 bis 10 Kohlenstoffatomen stehen und gegebenenfalls über eine Alkylengruppe oder eine Carbonatgruppe unter Bildung einer cyclischen Struktur aneinander gebunden sind, wobei die Alkylengruppe gegebenenfalls durch eine Hydroxygruppe, eine Phosphorsäuregruppe, eine Aminogruppe, eine Alkoxygruppe oder eine Estergruppe substituiert ist und gegebenenfalls eine in die Hauptkette eingefügte Carbonylgruppe aufweist.

## Revendications

**1.** Résine de polycarbonate comprenant un motif structural représenté par la formule (1) suivante :

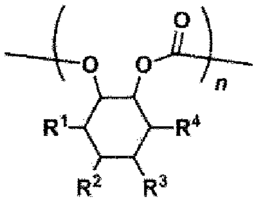

(1)

dans laquelle $R^1$, $R^2$, $R^3$ et $R^4$ sont chacun indépendamment un atome d'hydrogène, un groupe hydroxy, un groupe acide phosphorique, un groupe amino, un groupe vinyle, un groupe allyle, un groupe alcoxy ayant 1 à 10 atomes de carbone, un groupe ester ayant 1 à 11 atomes de carbone, un groupe acyle ayant 1 à 11 atomes de carbone, ou un groupe alkyle linéaire, ramifié ou cyclique non substitué ayant 1 à 10 atomes de carbone, et sont éventuellement liés les uns aux autres par l'intermédiaire d'un groupe alkylène ou d'un groupe carbonate pour former une structure cyclique, dans laquelle le groupe alkylène est éventuellement substitué par un groupe hydroxy, un groupe acide phosphorique, un groupe amino, un groupe alcoxy ou un groupe ester, et a éventuellement un groupe carbonyle inséré dans la chaîne principale, dans laquelle

le poids moléculaire moyen en poids Mw mesuré par chromatographie d'exclusion stérique en utilisant un poly(méthacrylate de méthyle) comme échantillon de référence est de 50 000 ou plus et de 500 000 ou moins.

2. Résine de polycarbonate selon la revendication 1, dans laquelle la température de transition vitreuse Tg mesurée avec un calorimètre différentiel à balayage est de 80 °C ou plus et de 180 °C ou moins.

3. Résine de polycarbonate selon la revendication 1 ou 2, dans laquelle la résine de polycarbonate comprend le motif structural représenté par la formule (1) dans la chaîne principale.

4. Résine de polycarbonate selon l'une quelconque des revendications 1 à 3, dans laquelle un rapport mr / (mm + mr + rr) mesuré par RMN du $^{13}C$ est de 40 % ou plus, dans laquelle mm représente un groupement isotactique dans une structure méso périodique du monomère, rr représente un groupement syndiotactique dans une structure périodique basée sur une liaison racémo, et mr représente un groupement dans lequel le groupement méso et le groupement racémo sont liés.

5. Résine de polycarbonate selon l'une quelconque des revendications 1 à 4, dans laquelle la résine de polycarbonate comprend un groupe alcoxy terminal.

6. Résine de polycarbonate selon la revendication 5, dans laquelle le nombre d'atomes de carbone dans le groupe alcoxy est de 1 à 10.

7. Résine de polycarbonate selon l'une quelconque des revendications 1 à 6, dans laquelle un composant polymère ayant un poids moléculaire à base de polystyrène de 1 000 ou plus mesuré par chromatographie d'exclusion stérique a un poids moléculaire moyen en poids (Mw) de 100 000 ou plus et 500 000 ou moins et un rapport (Mw/Mn) du poids moléculaire moyen en poids (Mw) sur un poids moléculaire moyen en nombre (Mn) de 2,5 ou moins.

8. Résine de polycarbonate selon la revendication 7, dans laquelle un taux de diminution en poids à 150 à 260 °C est de 30 % en masse ou moins lorsqu'une matière insoluble dans l'alcool est chauffée dans une atmosphère d'azote par thermogravimétrie, mesurée comme indiqué dans la spécification.

9. Résine de polycarbonate selon l'une quelconque des revendications 1 à 8, dans laquelle une valeur absolue d'un coefficient photoélastique est de $10 \times 10^{-12}$ $Pa^{-1}$ ou moins, mesurée comme indiqué dans la spécification.

10. Résine de polycarbonate selon l'une quelconque des revendications 1 à 9, dans laquelle une valeur absolue d'une différence de phases dans le plan dans un film étiré uniaxialement à 100 % de la résine de polycarbonate est de 100 nm ou moins en termes d'une épaisseur de 100 $\mu$m, mesurée comme indiqué dans la spécification.

11. Résine de polycarbonate selon l'une quelconque des revendications 1 à 10, dans laquelle la résine de polycarbonate comprend un motif structural représenté par la formule (1) dans laquelle chacun parmi $R^1$, $R^2$, $R^3$ et $R^4$ est un atome d'hydrogène.

12. Résine de polycarbonate selon l'une quelconque des revendications 1 à 11, dans laquelle la résine de polycarbonate

comprend un motif structural représenté par la formule (1') suivante :

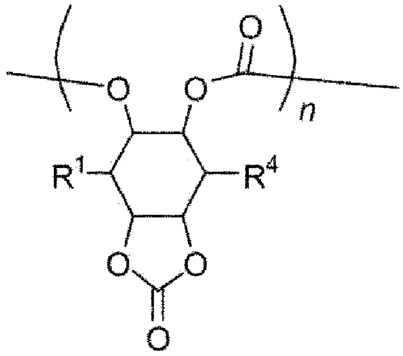

(1')

dans laquelle $R^1$ et $R^4$ sont chacun indépendamment un atome d'hydrogène, un groupe hydroxy, un groupe acide phosphorique, un groupe amino, un groupe vinyle, un groupe allyle, un groupe alcoxy ayant 1 à 10 atomes de carbone, un groupe ester ayant 1 à 11 atomes de carbone, un groupe acyle ayant 1 à 11 atomes de carbone, ou un groupe alkyle linéaire, ramifié ou cyclique non substitué ayant 1 à 10 atomes de carbone, et sont éventuellement liés les uns aux autres par l'intermédiaire d'un groupe alkylène ou d'un groupe carbonate pour former une structure cyclique, dans laquelle le groupe alkylène est éventuellement substitué par un groupe hydroxy, un groupe acide phosphorique, un groupe amino, un groupe alcoxy ou un groupe ester, et a éventuellement un groupe carbonyle inséré dans la chaîne principale.

**13.** Composition de résine de polycarbonate comprenant la résine de polycarbonate selon l'une quelconque des revendications 1 à 12 et un antioxydant.

**14.** Composant optique comprenant la résine de polycarbonate selon l'une quelconque des revendications 1 à 12 ou la composition de résine de polycarbonate selon la revendication 13.

**15.** Utilisation de la résine de polycarbonate selon l'une quelconque des revendications 1 à 12 ou de la composition de résine de polycarbonate selon la revendication 13 comme matériau pour un composant optique.

**16.** Procédé de production de la résine de polycarbonate selon l'une quelconque des revendications 1 à 12, comprenant

une étape de polymérisation consistant à polymériser par ouverture de cycle un carbonate cyclique représenté par la formule (3) suivante pour obtenir la résine de polycarbonate :

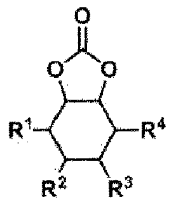

(3)

dans laquelle $R^1$, $R^2$, $R^3$ et $R^4$ sont chacun indépendamment un atome d'hydrogène, un groupe hydroxy, un groupe acide phosphorique, un groupe amino, un groupe vinyle, un groupe allyle, un groupe alcoxy ayant 1 à 10 atomes de carbone, un groupe ester ayant 1 à 11 atomes de carbone, un groupe acyle ayant 1 à 11 atomes de carbone, ou un groupe alkyle linéaire, ramifié ou cyclique non substitué ayant 1 à 10 atomes de carbone, et sont éventuellement liés les uns aux autres par l'intermédiaire d'un groupe alkylène ou d'un groupe carbonate pour former une structure cyclique, dans laquelle le groupe alkylène est éventuellement substitué par un groupe hydroxy, un groupe acide phosphorique, un groupe amino, un groupe alcoxy ou un groupe ester, et a éventuellement un groupe carbonyle inséré dans la chaîne principale.

Figure 1

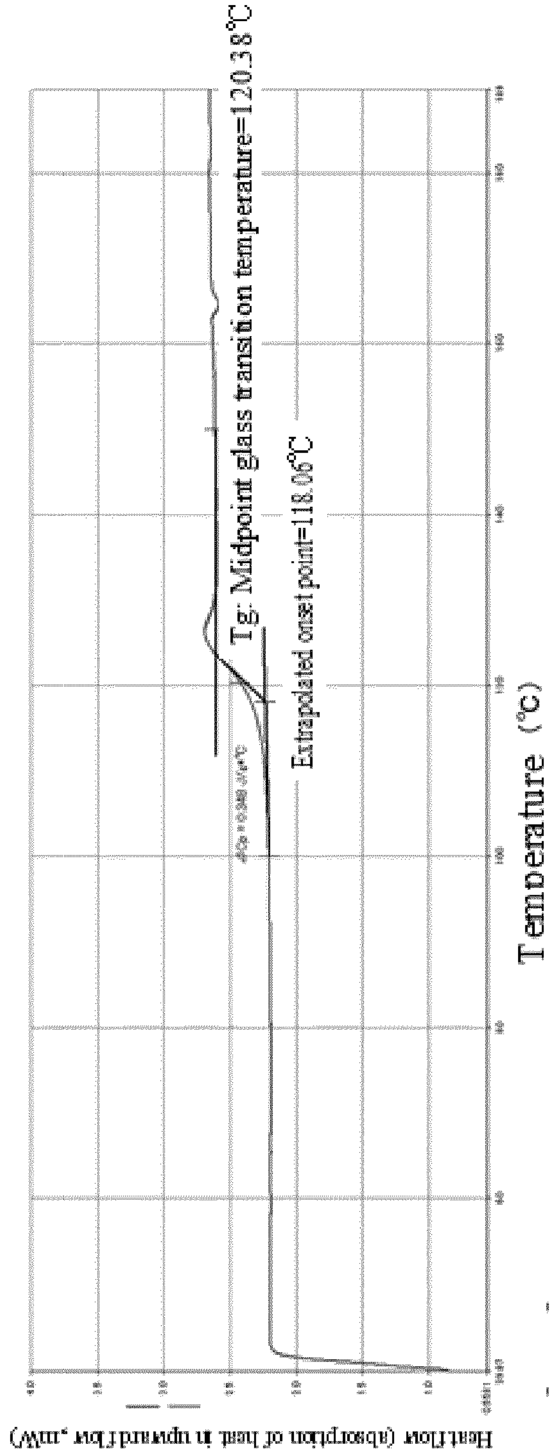
Tg: Midpoint glass transition temperature=120.38°C
Extrapolated onset point=118.06°C
Temperature (°C)
Heat flow (absorption of heat in upward flow, mW)

Figure 2

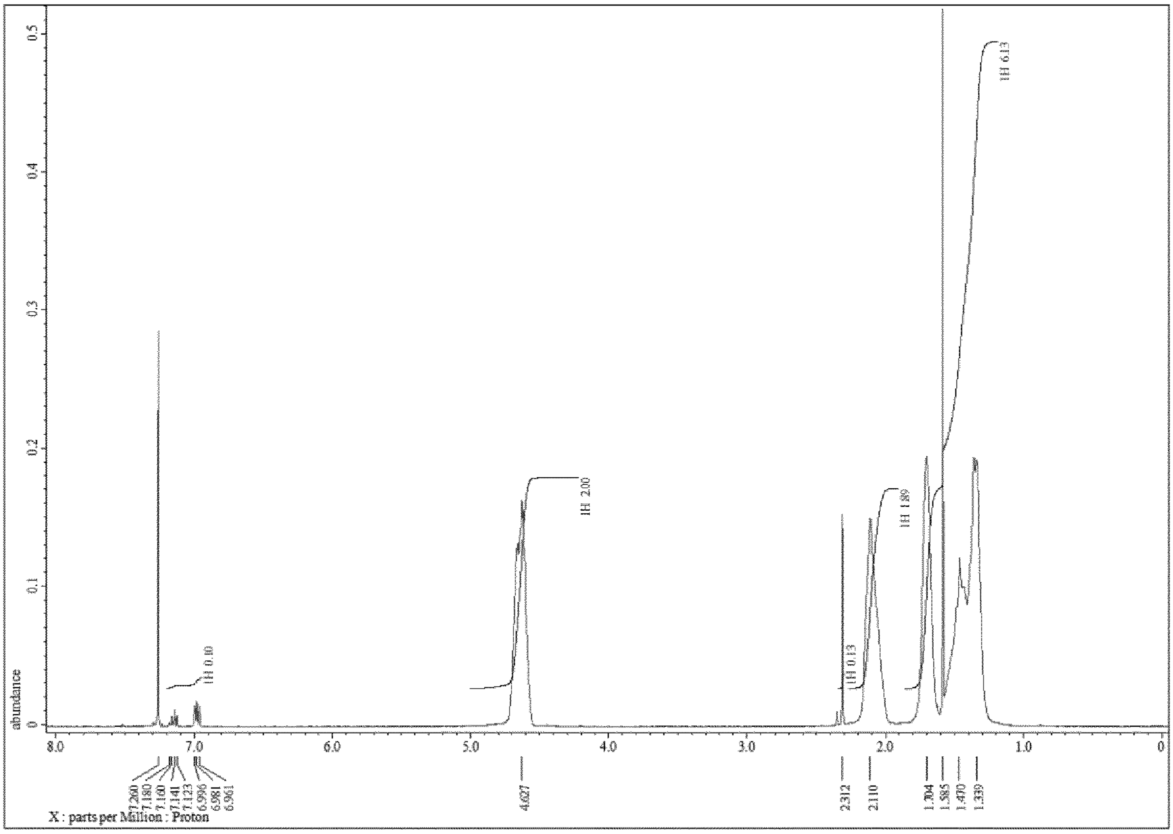
abundance
0.5
0.4
0.3
0.2
0.1
0
1H 6.13
1H 2.00
1H 1.89
1H 0.13
1H 0.10
8.0
7.0
6.0
5.0
4.0
3.0
2.0
1.0
0
7.260
7.180
7.160
7.141
7.123
6.996
6.981
6.961
4.627
2.312
2.110
1.704
1.585
1.470
1.339
X : parts per Million : Proton

Figure 3

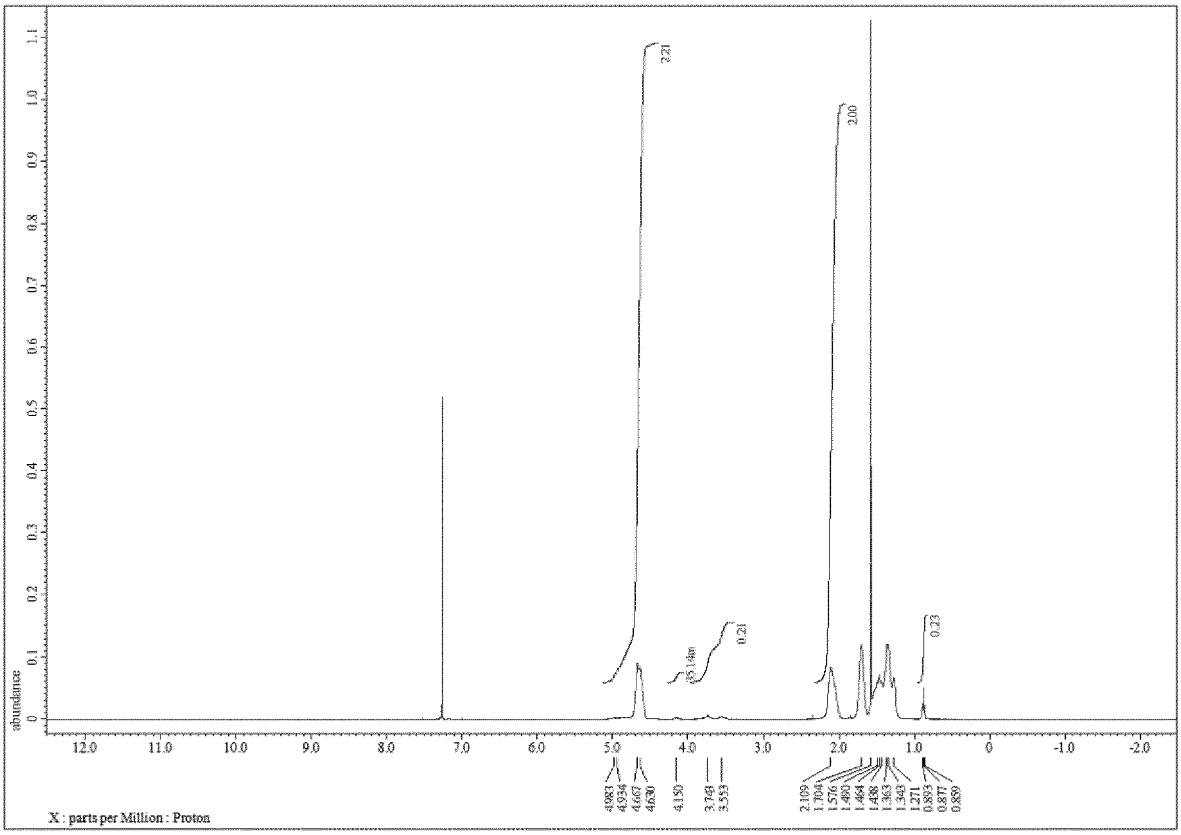
abundance
0
0.1
0.2
0.3
0.4
0.5
0.6
0.7
0.8
0.9
1.0
1.1
2.21
35.14m
0.21
2.00
0.23
12.0
11.0
10.0
9.0
8.0
7.0
6.0
5.0
4.0
3.0
2.0
1.0
0
-1.0
-2.0
4.983
4.934
4.667
4.630
4.150
3.743
3.553
2.109
1.704
1.576
1.490
1.464
1.438
1.363
1.343
1.271
0.893
0.877
0.859
X : parts per Million : Proton

Figure 4

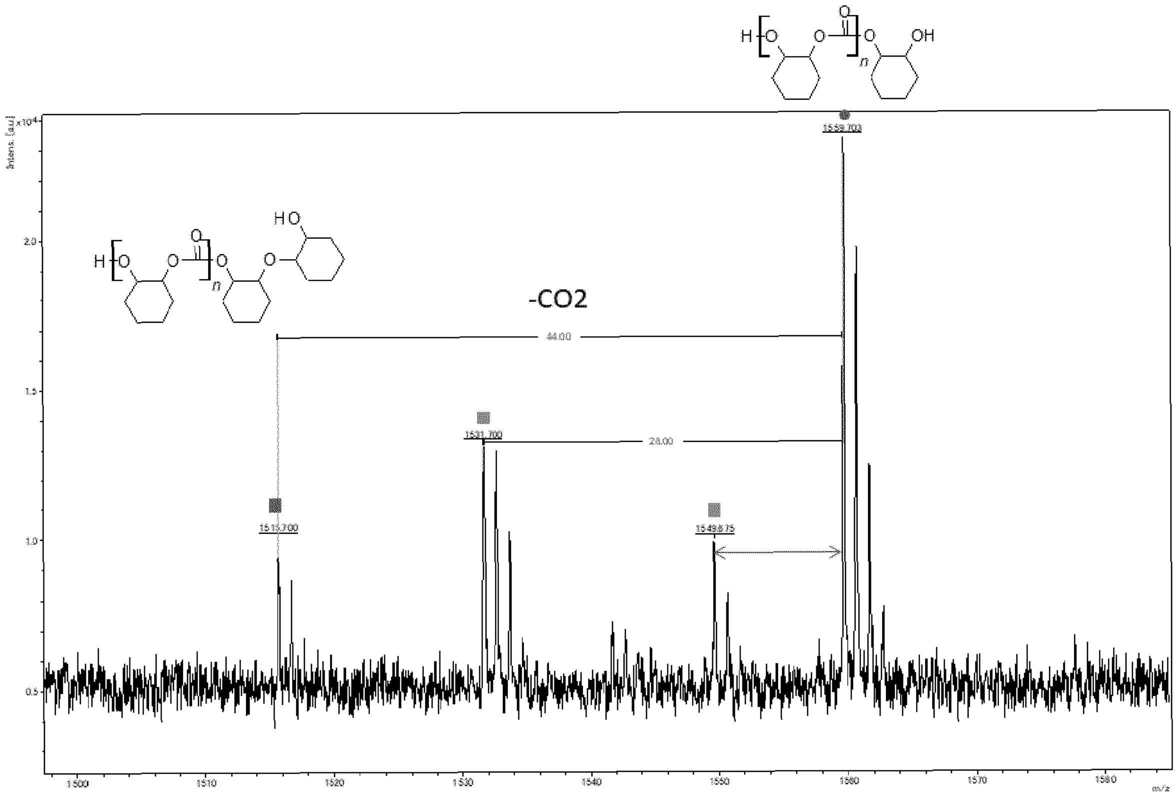

Figure 5

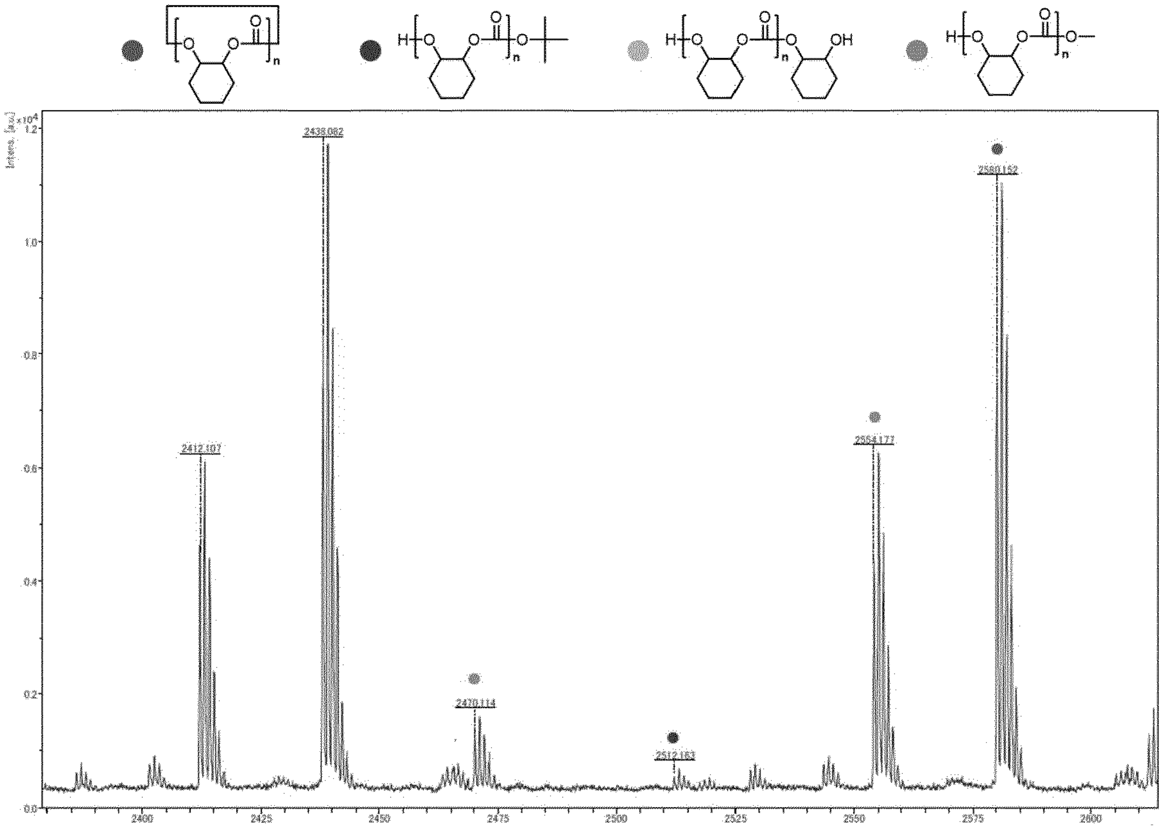
Intens. [a.u.] x10$^4$
1.2
1.0
0.8
0.6
0.4
0.2
0.0
2438.082
2412.107
2470.114
2512.163
2554.177
2580.152
2400
2425
2450
2475
2500
2525
2550
2575
2600

Figure 6

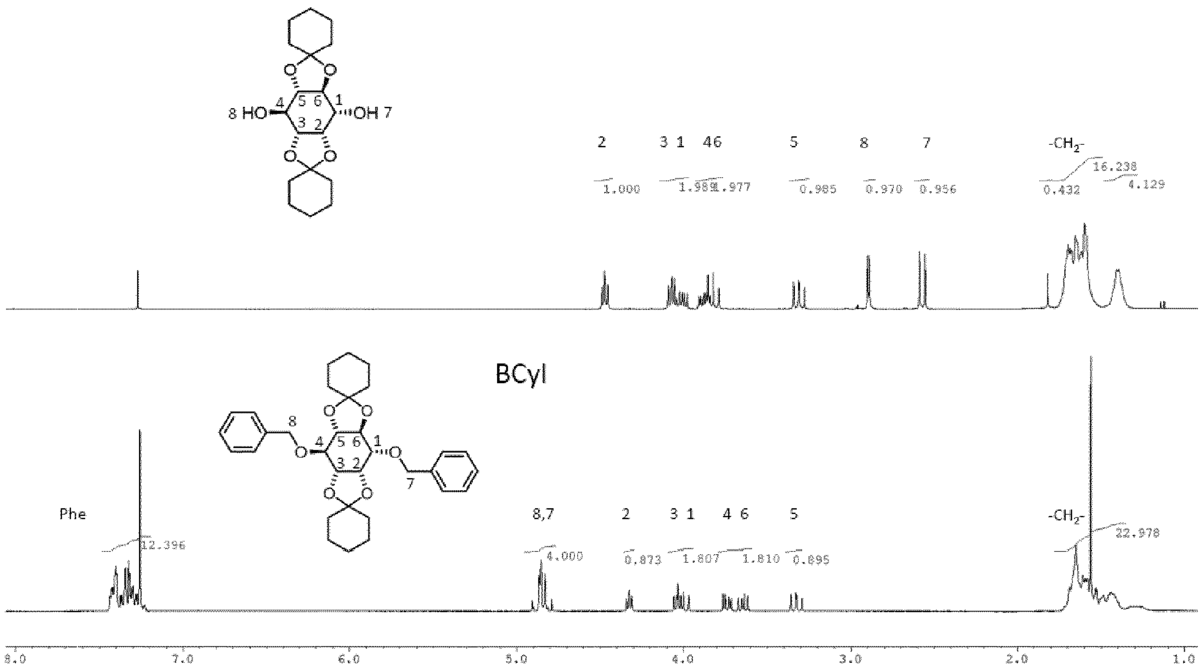
8 HO
OH 7
2
3 1
46
5
8
7
-CH2-
1.000
1.989
1.977
0.985
0.970
0.956
0.432
16.238
4.129
BCyI
Phe
12.396
8,7
4.000
2
0.873
3 1
1.807
4 6
1.810
5
0.895
-CH2-
22.978
8.0
7.0
6.0
5.0
4.0
3.0
2.0
1.0

Figure 7

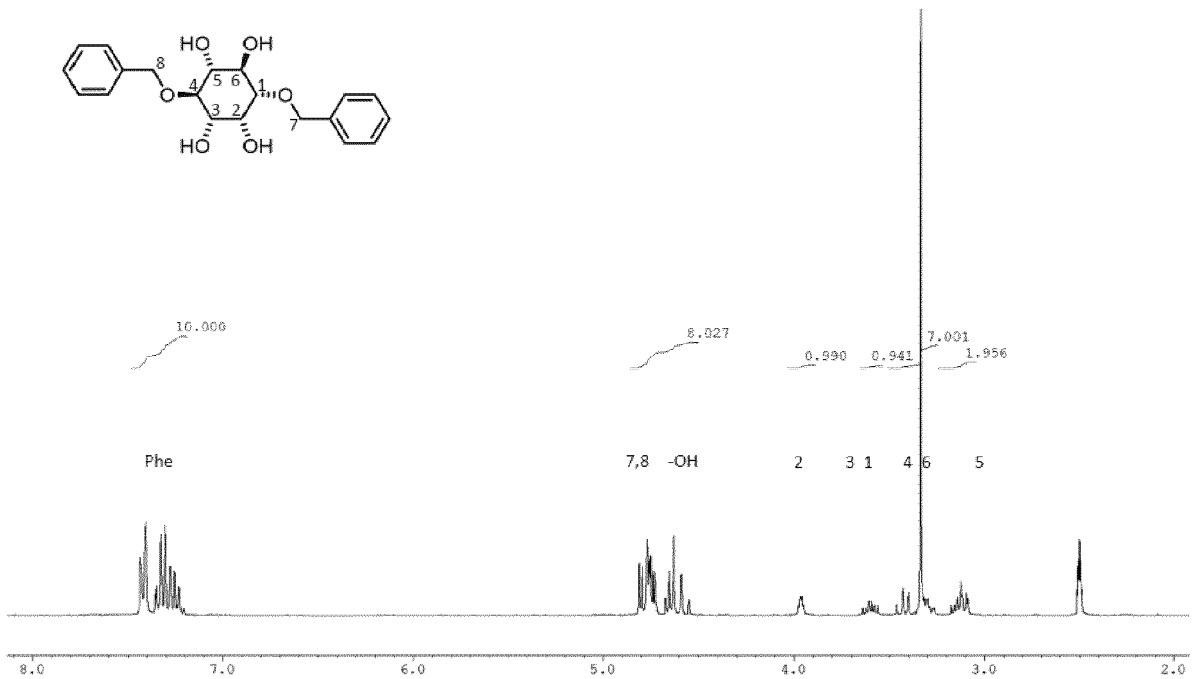
10.000
8.027
0.990
0.941
7.001
1.956
Phe
7,8
-OH
2
3 1
4 6
5
8.0
7.0
6.0
5.0
4.0
3.0
2.0

Figure 8

BCI

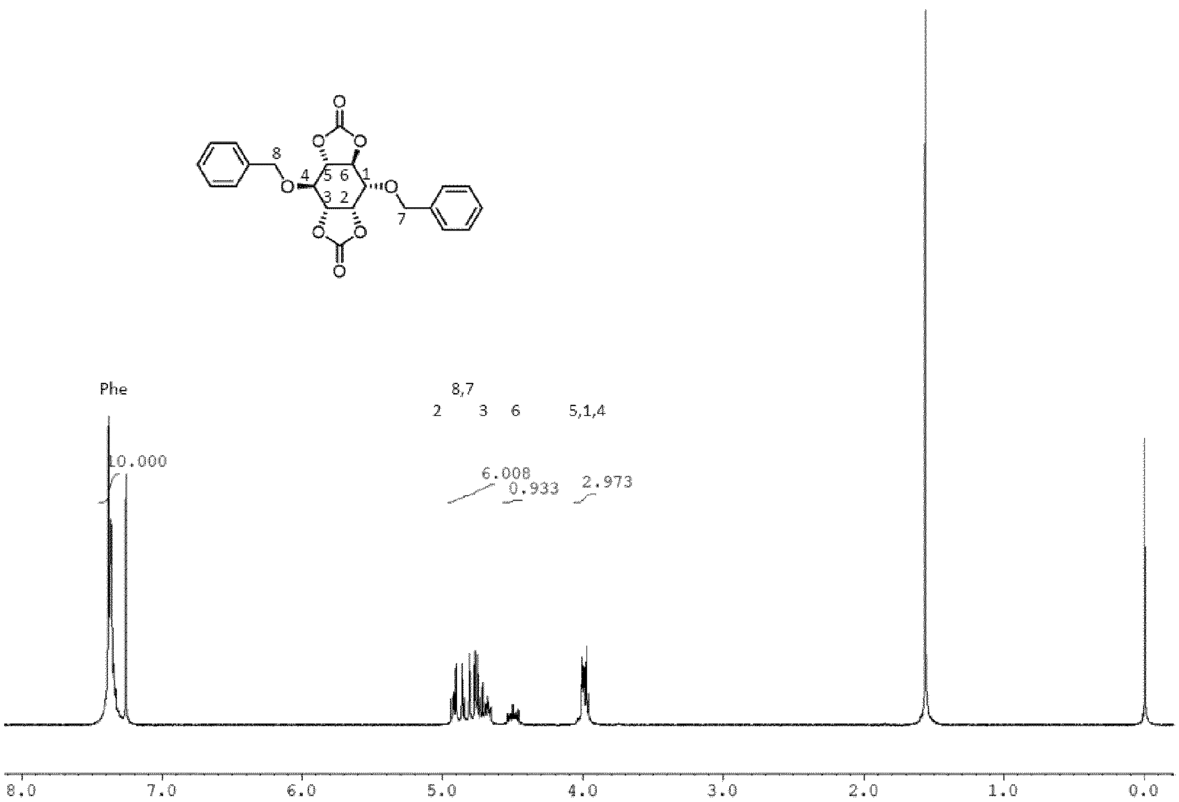

Figure 9

OCI

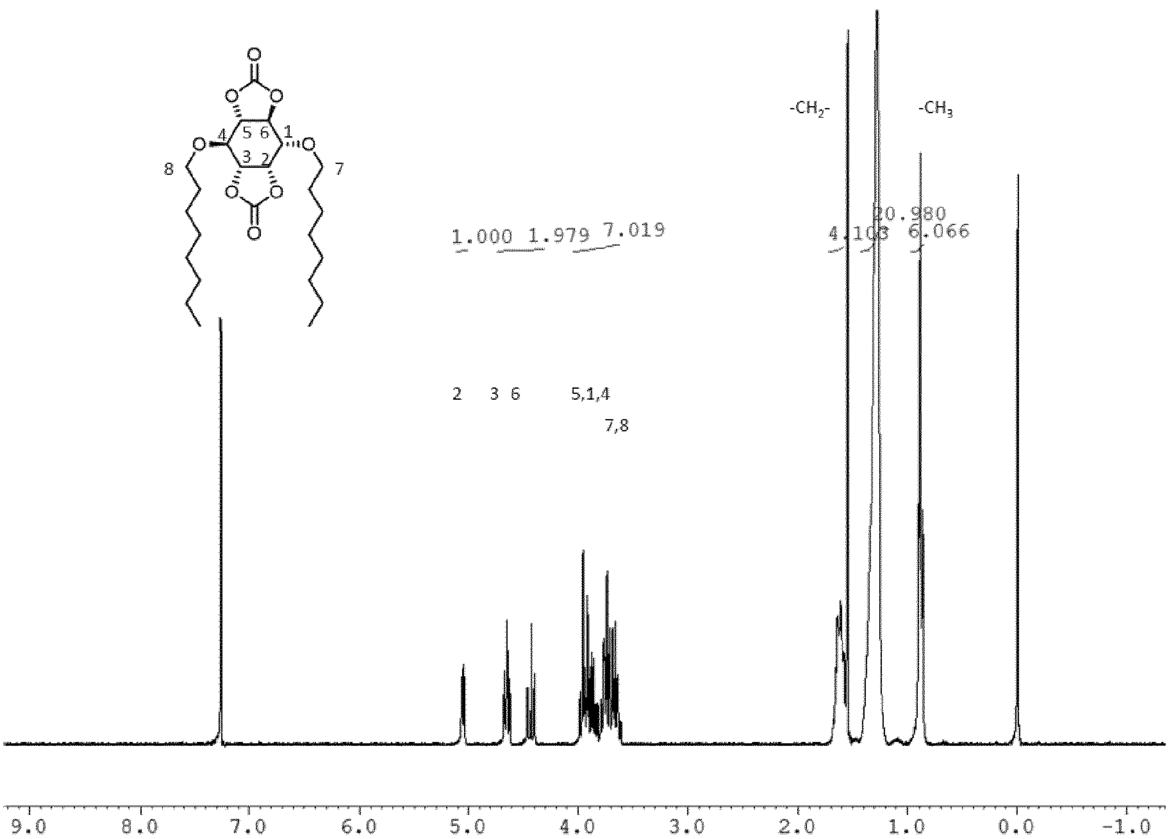
-CH$_2$-
-CH$_3$
1.000 1.979 7.019
20.980
4.103 6.066
2 3 6 5,1,4
7,8
9.0 8.0 7.0 6.0 5.0 4.0 3.0 2.0 1.0 0.0 -1.0

Figure 10

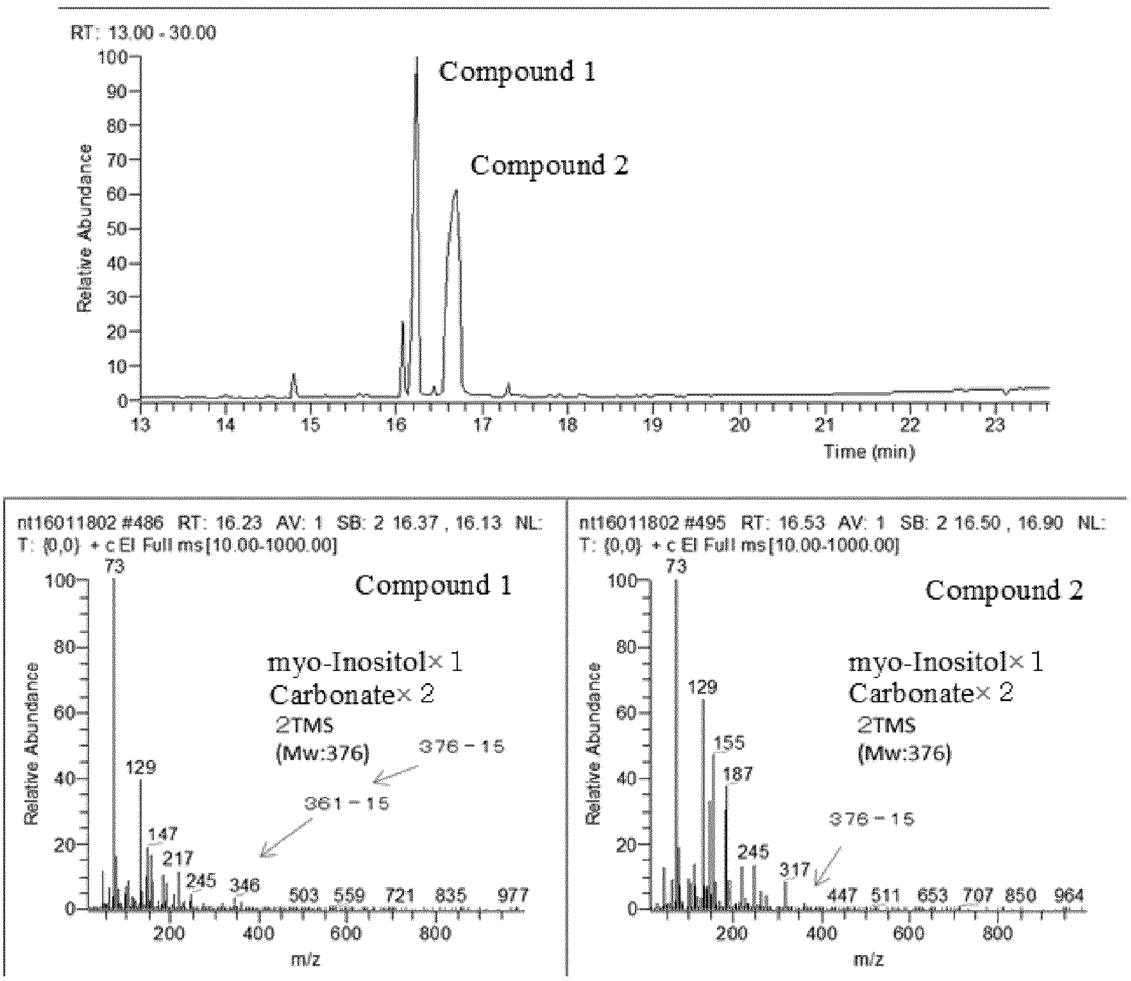
RT: 13.00 - 30.00
Compound 1
Compound 2
Relative Abundance
Time (min)
nt16011802 #486 RT: 16.23 AV: 1 SB: 2 16.37 , 16.13 NL:
T: {0,0} + c EI Full ms [10.00-1000.00]
Compound 1
myo-Inositol× 1
Carbonate× 2
2TMS
(Mw:376)
376 − 15
361 − 15
m/z
nt16011802 #495 RT: 16.53 AV: 1 SB: 2 16.50 , 16.90 NL:
T: {0,0} + c EI Full ms [10.00-1000.00]
Compound 2
myo-Inositol× 1
Carbonate× 2
2TMS
(Mw:376)
376 − 15
m/z

Figure 11

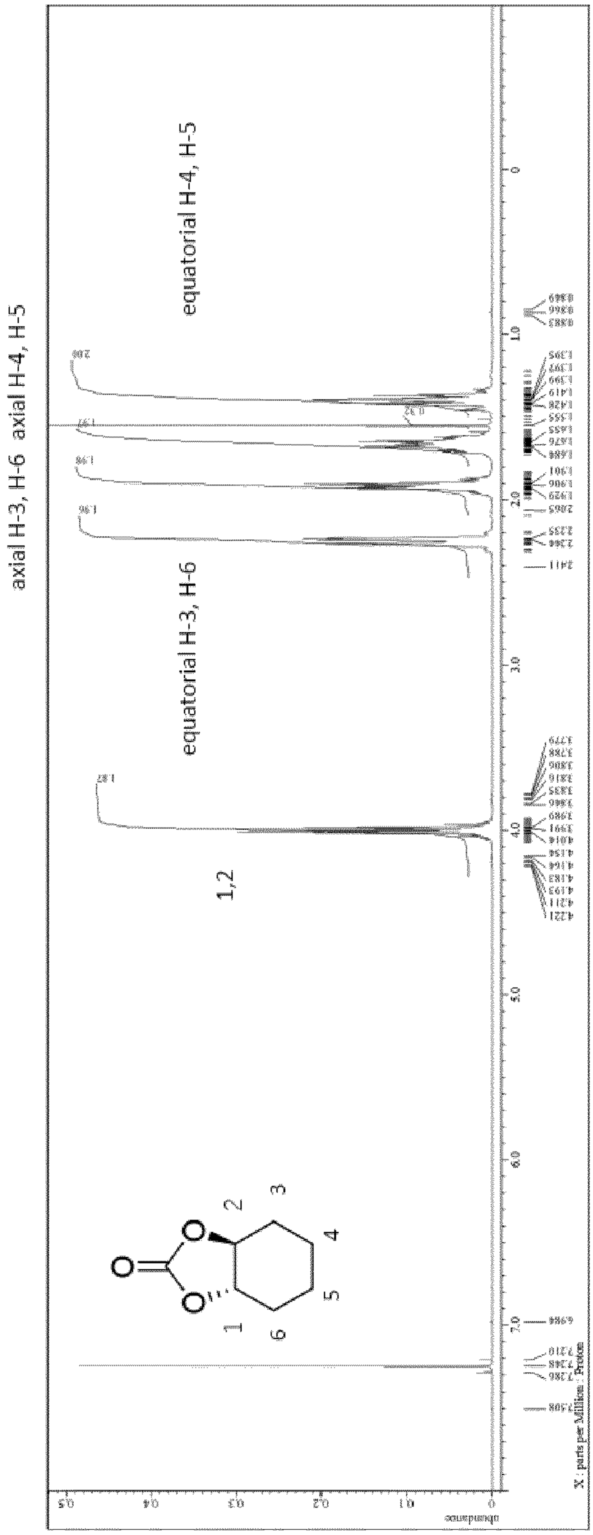
axial H-3, H-6 axial H-4, H-5
equatorial H-4, H-5
equatorial H-3, H-6
1,2
abundance
X : parts per Million : Proton

Figure 12

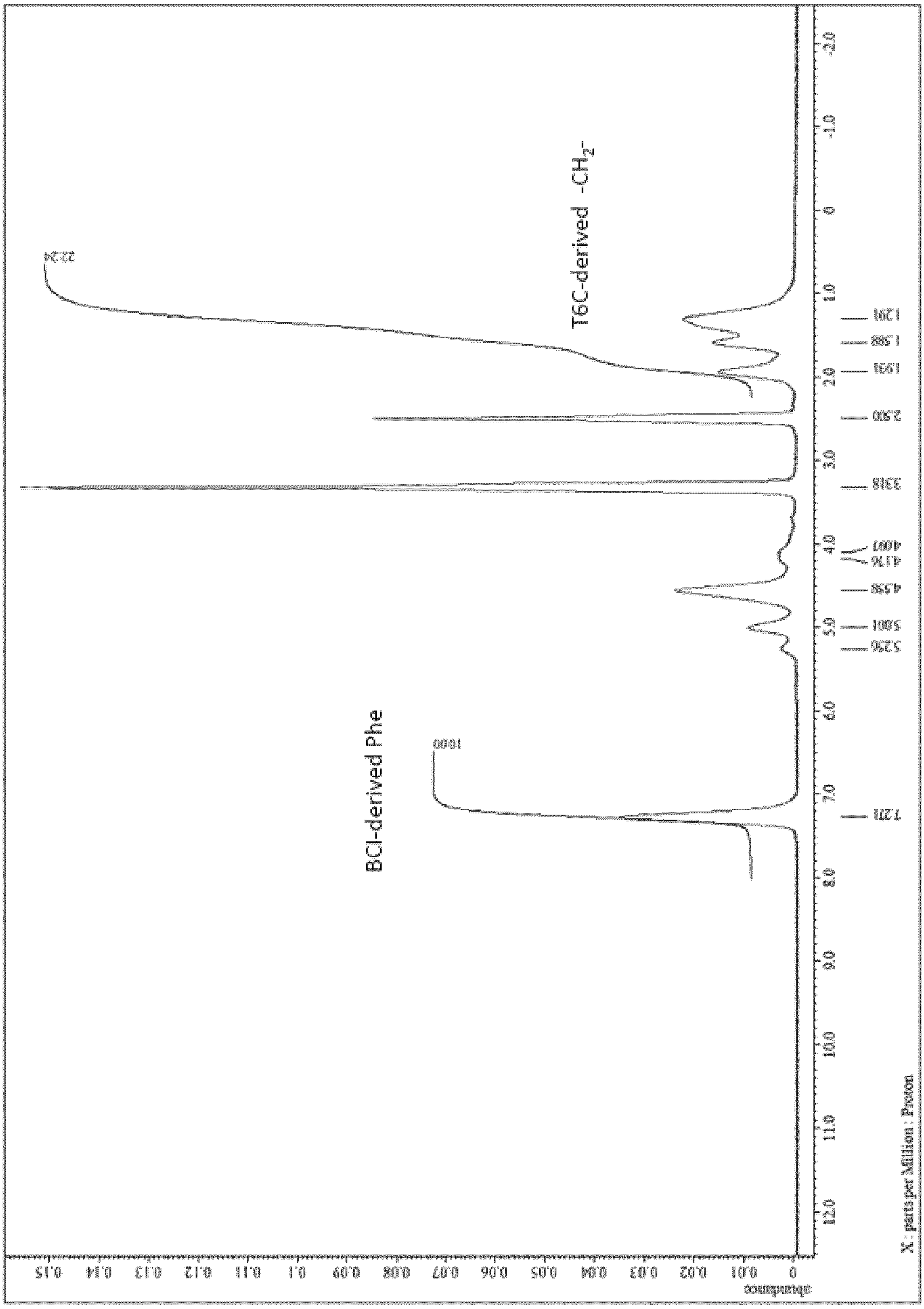
T6C-derived -CH2-
BCI-derived Phe
22.24
10.00
1.291
1.588
1.931
2.500
3.318
4.097
4.176
4.558
5.001
5.256
7.271
abundance
X : parts per Million : Proton

Figure 13

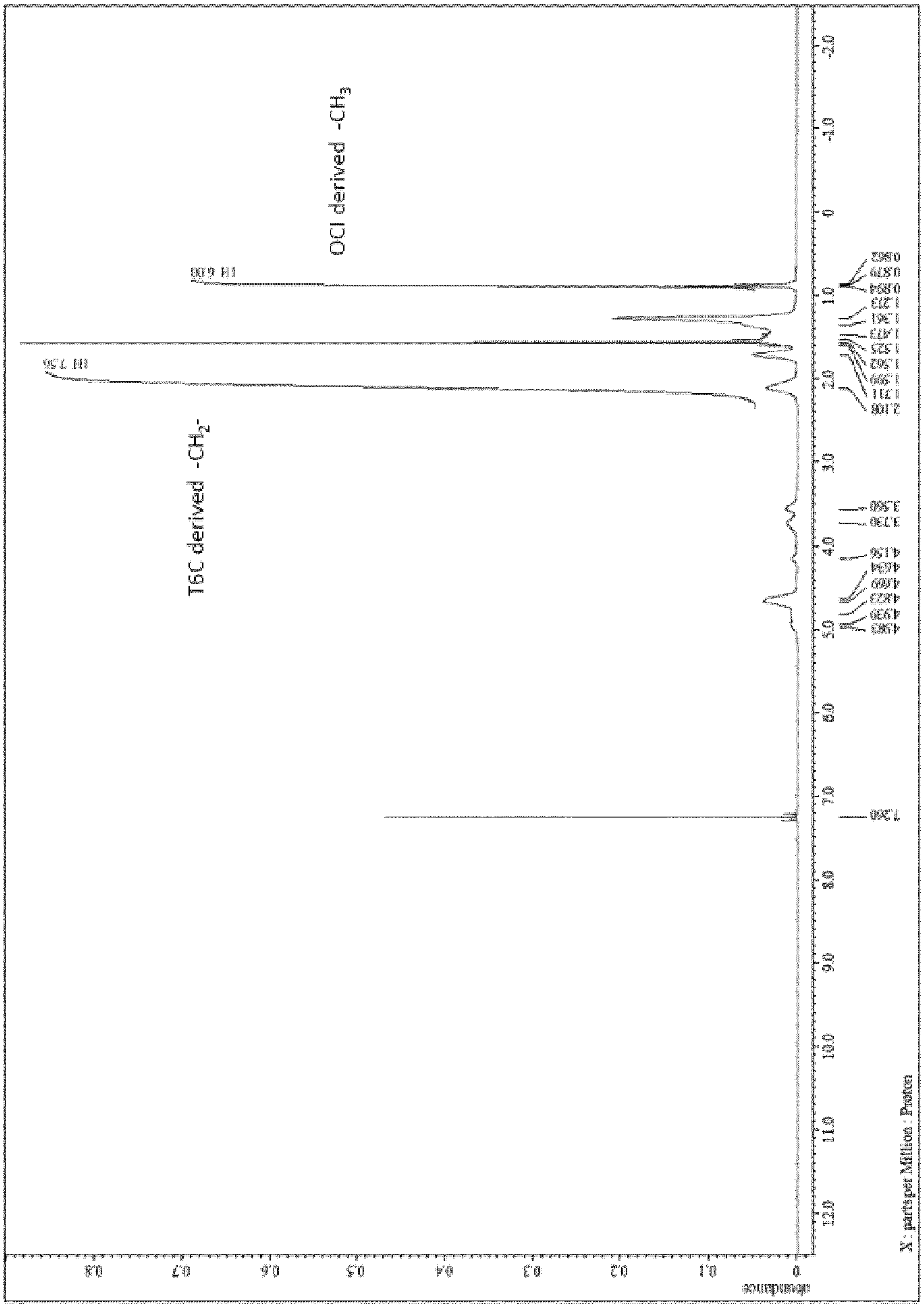
OCI derived -CH3
T6C derived -CH2-
1H 6.00
1H 7.56
X : parts per Million : Proton
abundance

Figure 14

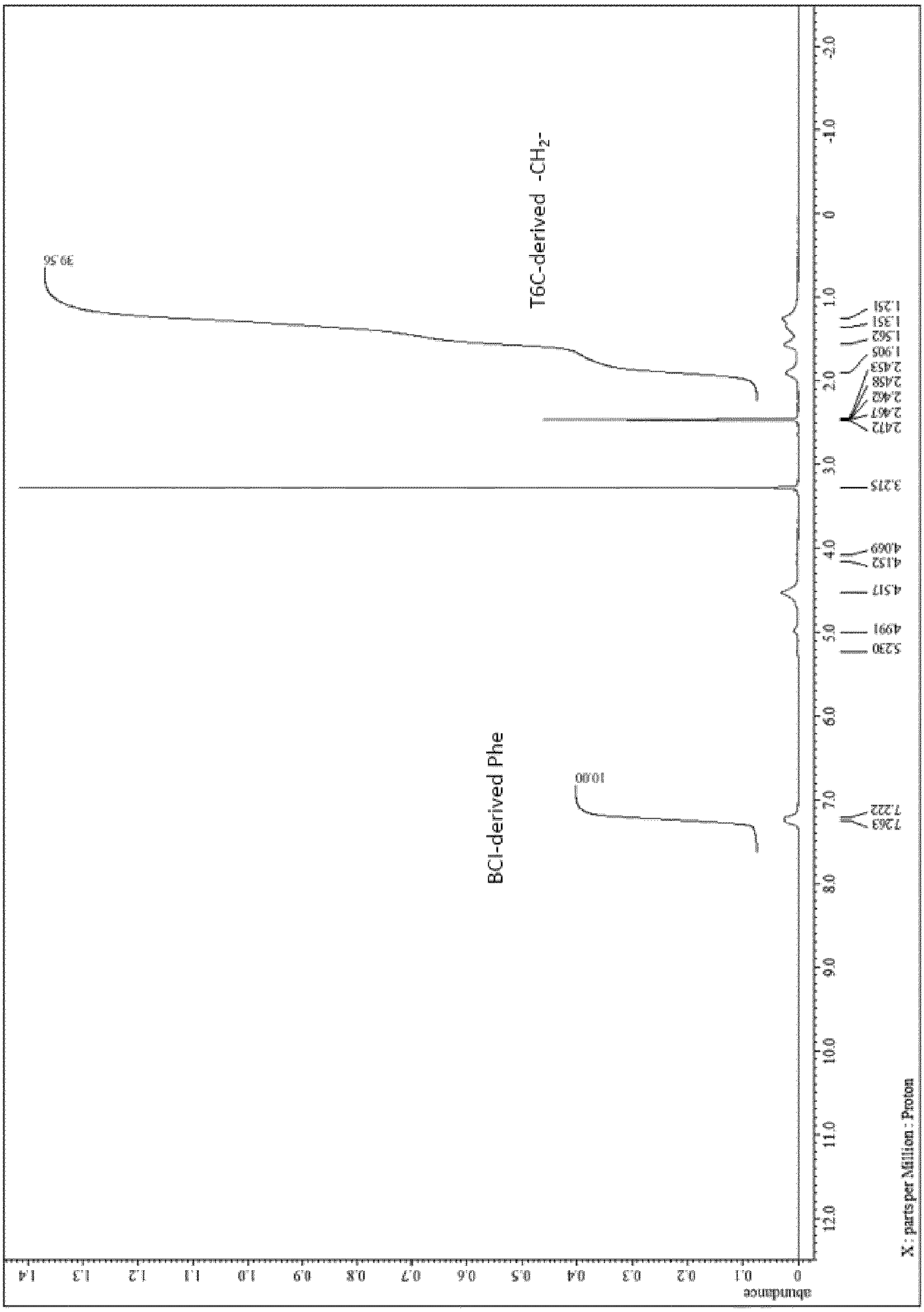
T6C-derived -CH$_2$-
BCI-derived Phe
39.56
10.00
1.251
1.351
1.562
1.905
2.453
2.458
2.462
2.467
2.472
3.215
4.069
4.152
4.517
4.991
5.230
7.222
7.263
X : parts per Million : Proton
abundance

Figure 15

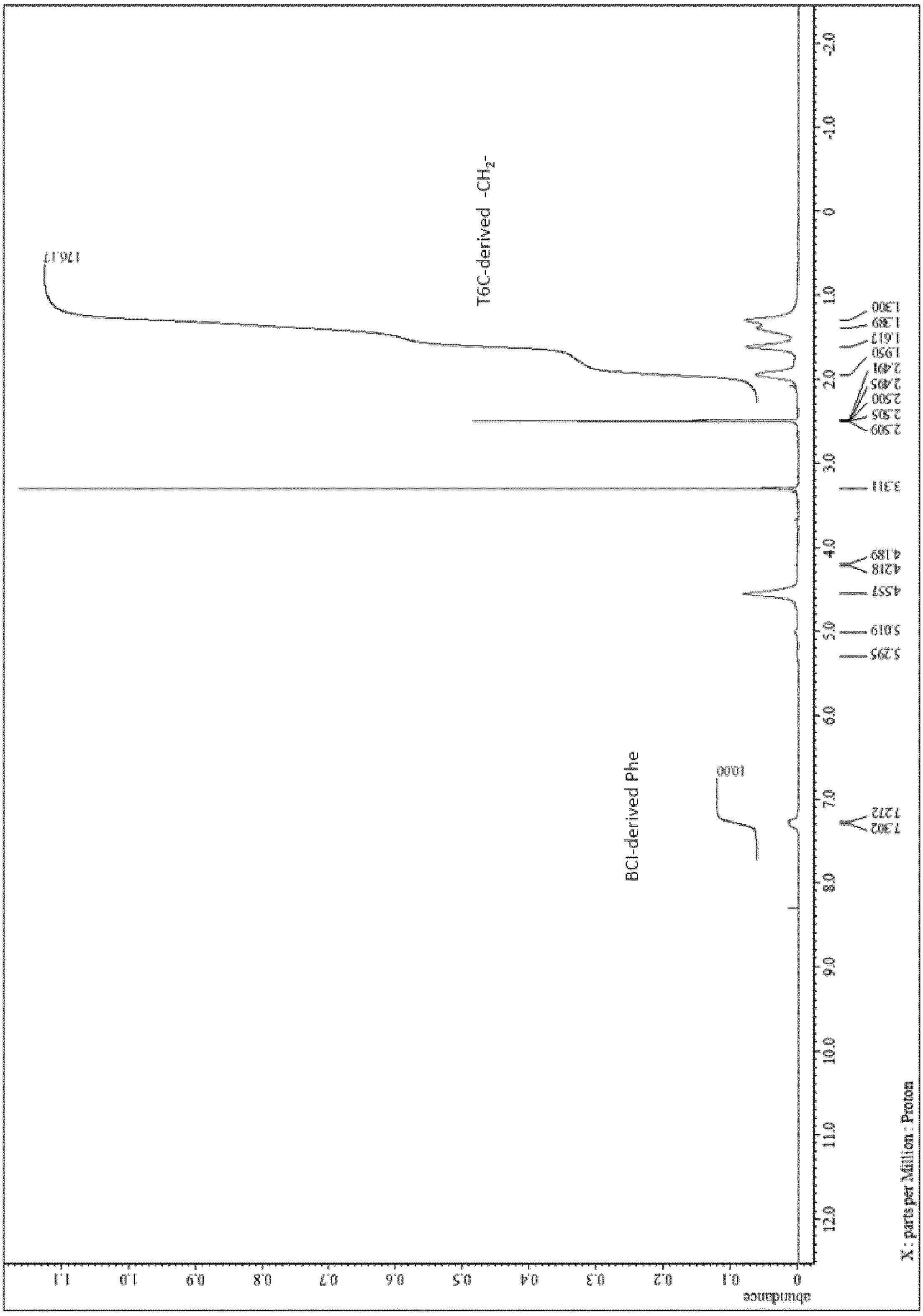
T6C-derived -CH2-
BCI-derived Phe
176.17
10.00
X : parts per Million : Proton
abundance

Figure 16

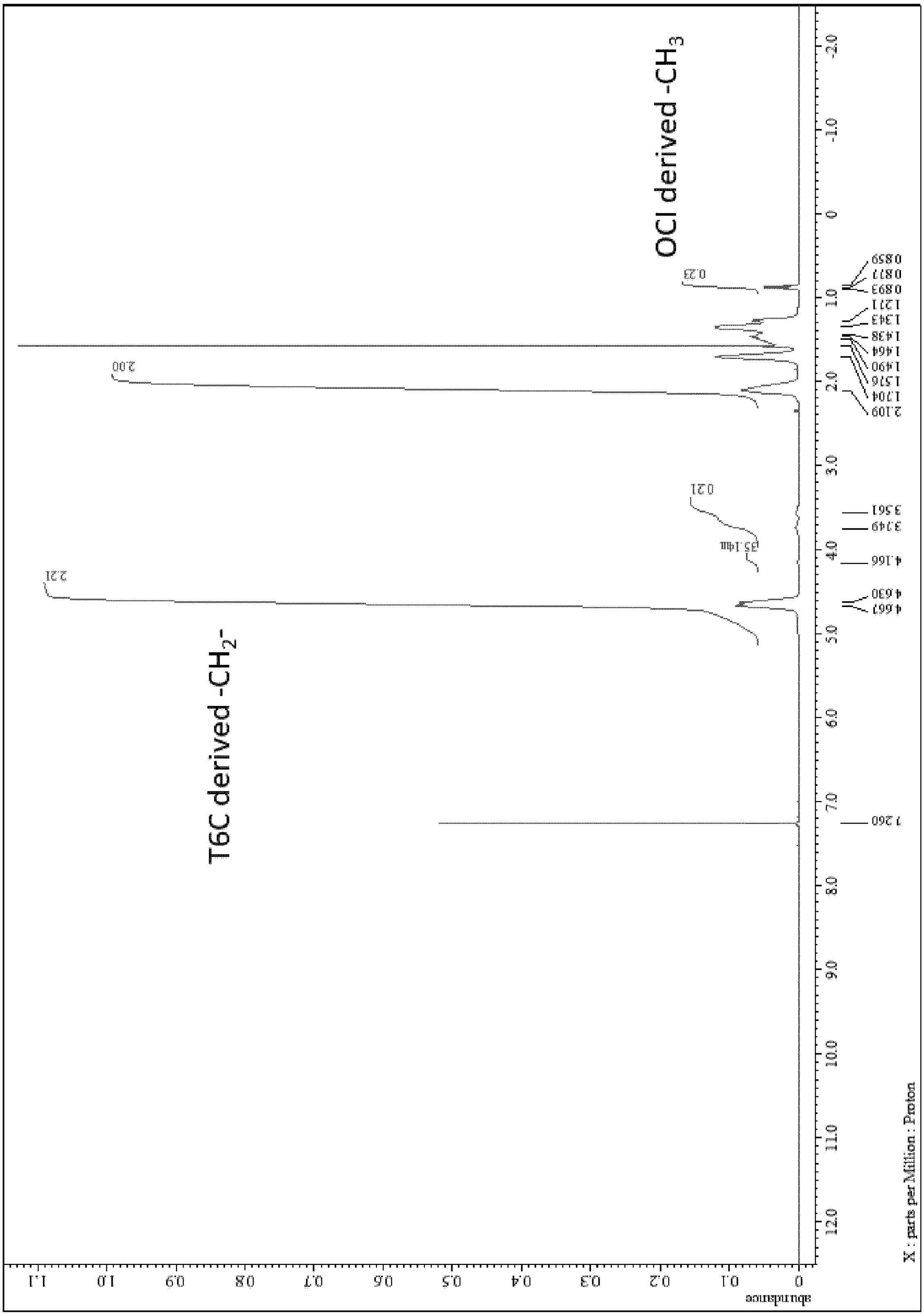

OCI derived $-CH_3$
T6C derived $-CH_2-$
X : parts per Million : Proton
abundance

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6327151 B
- JP 6512219 B
- JP 2019143152 A
- JP 2019131824 A
- JP 2018059107 A
- JP 4774610 B
- JP 6507495 B
- JP 5403537 B
- JP 2019108547 A
- JP 2013528685 W
- JP 2008238417 A
- JP 2019127441 A
- JP 2019127442 A
- CN 107991842 A
- JP 2020005443 A
- JP 2020015754 A


### Non-patent literature cited in the description


- *Macromolecules*, 2014, vol. 47, 4230-4235
- *Polymer Journal*, 2013, vol. 45, 1183-1187
- *Chem. Eur. J.*, 2016, vol. 22, 1722-1727
- *Biomacromolecules*, 2006, vol. 7, 2269-2273
- *ACS Macro Lett.*, 2018, vol. 7, 336-340
- *ACS Catal.*, 2015, vol. 5, 1353-1370
- *Bull. Korean Chem. Soc.*, 2006, vol. 27 (3), 359-360
- *Carbohydrate Research*, 1995, vol. 277, C5-C7
- *Polymer journal*, 2013, vol. 45, 1183-1187
- *Polymer Engineering and Science*, 1999, vol. 39, 2349-2357